# EUROPEAN PATENT APPLICATION

(11) **EP 4 729 536 A1**
(43) Date of publication of application: **22.04.2026**
(21) Application number: 24822789.4
(22) Date of filing: 14.06.2024
(51) Int. Cl.: C07K 16/10, C12N 15/13, A61K 39/42

(54) **HEPATITIS B VIRUS SURFACE ANTIBODY AND USE THEREOF**

(30) Priority: 16.06.2023 CN 202310720736
(71) Applicant: Fudan University, Shanghai 200433 (CN)
(72) Inventor: YUAN, Zhenghong, Shanghai 200433 (CN); WANG, Qiao, Shanghai 200433 (CN); LI, Yaming, Shanghai 200433 (CN)
(74) Representative: Epping - Hermann - Fischer
(86) International application number: PCT/CN2024/099201
(87) International publication number: WO 2024/255841

(57) **Abstract**

The present invention relates to an antibody against a hepatitis B surface antigen (HBsAg) and an antigen-binding fragment thereof, and methods for preparing and using the antibodies and the antigen-binding fragment thereof. The present invention further relates to bispecific antibodies that specifically bind to different epitopes on HBsAg. The antibodies are derived from persons inoculated with hepatitis B vaccines and persons having recovered from hepatitis B infections, show superior effects in recognizing different hepatitis B surface antigens, and have greater advantages in neutralizing hepatitis B virus mutants and reducing hepatitis B mutant antigens.

## Description

### TECHNICAL FIELD

The present invention relates to an antibody against a hepatitis B surface antigen (HBsAg) and an antigen-binding fragment thereof, and methods for preparing and using the antibodies and the antigen-binding fragment thereof.

### BACKGROUND

Hepatitis B virus (HBV) infection, both acute and chronic, is a serious global public health problem that severely threatens human health. While HBV vaccination has greatly controlled the spread of the virus, China still has the largest number of HBV patients in the world. Currently, drugs used clinically for treating chronic HBV, including interferons and nucleoside/nucleotide analogues, can effectively inhibit HBV replication, but they cannot completely eliminate the virus and thus cure chronic HBV infection, and drug resistance may also develop.

HBV is an enveloped DNA virus belonging to the Hepadnaviridae family. Currently, there are nine confirmed HBV genotypes A-I and one undetermined genotype J, which are further subdivided into many subgenotypes with specific geographic distributions. The natural history of HBV infection varies depending on the HBV genotype, including the mode of transmission, the time of HBeAg seroconversion, the speed and severity of liver disease progression (e.g., progression to hepatocellular carcinoma), and the likelihood of HBsAg clearance. HBsAg particles exist in three sizes: large, medium, and small. Small HBsAg, encoded by 226 amino acids, is a major component that triggers the production of protective antibodies. The HBsAg protein possesses four transmembrane regions, including an extracellular domain approximately 70 amino acids long (amino acids 99-169). This extracellular domain is exposed on the surface of the viral particle and is crucial for viral infection of host cells, as well as serving as an important site for recognition by the body's immune system (e.g., antibodies). Within this extracellular region, there is an immunodominant region called the "a" antigenic determinant, located on amino acids 124-147 between transmembrane regions 2 and 3. This is an important epitope recognized by hepatitis B surface antibody anti-HBs. Due to natural selection and the temporal and spatial pressures of survival of the fittest during chronic HBV infection, a series of mutant strains will emerge, and the aforementioned "a" antigenic determinant is a region with a high mutation frequency of HBsAg.

Hepatitis B surface antigen (HBsAg) can originate from HBV cccDNA and/or integrated HBV DNA fragments. It not only participates in the formation of complete viral particles as an envelope protein of HBV, but also combines with host lipids to form non-infectious subviral particles, namely HBsAg particles, which exist freely in the blood of infected individuals, leading to surface antigenemia. Increasing research shows that the large amount of HBsAg present in patients with chronic hepatitis B may be the cause of abnormal or depleted immune responses, and may affect the pathobiology of liver diseases and the development of cancer. Therefore, reducing or clearing HBsAg can not only prevent hepatitis B virus infection, but also help achieve "functional cure," thereby reducing the incidence of complications (cirrhosis and hepatocellular carcinoma). Utilizing monoclonal antibodies targeting HBsAg protein (anti-HBs) to reduce or clear HBsAg protein in the human body is a clinical strategy worthy of exploration and research for "functional cure of hepatitis B."

Currently, the most economical and effective method for preventing and controlling hepatitis B is vaccination. The hepatitis B surface antibody (anti-HBs) produced after vaccination is a protective antibody against hepatitis B. Since China implemented its policy of vaccinating newborns against hepatitis B in 1992, the positive rate of hepatitis B virus antigen in the population has decreased year by year. As of 2020, the positive rate of hepatitis B surface antigen in the population in China was about 6%. However, due to the large population base, there are still 86 million people infected with HBV in China, with approximately 1 million new cases each year.

Antibodies are secreted by plasma cells differentiated from B lymphocytes. Each B lymphocyte can only produce a proprietary antibody targeting one specific antigenic determinant. Antibodies obtained from a single cell or cell line are called monoclonal antibodies. With the development of biotechnology, monoclonal antibody screening technology has also continuously improved. Numerous studies have used various techniques to obtain anti-HBs monoclonal antibodies from peripheral blood B cells of vaccine-immunized individuals, such as phage display Fab library construction, EBV-mediated B cell immortalization, and B cell activation culture. These techniques have yielded monoclonal antibodies that can bind to HBsAg and possess a certain degree of neutralizing activity, with the antigen recognition epitopes all located in the "a" determinant of S-HBsAg. However, the monoclonal antibodies obtained from these studies share a common drawback: the loss of the naturalness of humanized antibodies. Existing research data indicates that volunteers with high serum neutralizing efficacy against hepatitis B virus can produce anti-HBs monoclonal antibodies with high in vitro neutralizing potency and effective inhibition of in vivo hepatitis B virus amplification (1, 2). The recombinant expression yielded multiple naturally humanized anti-HBs antibodies, most of which exhibited neutralizing potency in in vitro experiments. Notably, in in vivo infection experiments with human liver chimeric mice, significant viral escape occurred after several weeks of treatment with a single antibody. Treatment with complementary monoclonal antibodies recognizing different epitopes more effectively suppressed viremia and prevented viral escape in mice. Therefore, the anti-HBs isolated from hepatitis B vaccine recipients demonstrated the ability to block HBV infection and inhibit viral replication in both in vitro and in vivo experimental models.

Although hepatitis B virus (HBV) is a DNA virus, its replication involves an intermediate process using RNA reverse transcriptase. Furthermore, DNA polymerase lacks self-correction capabilities, making it more prone to genomic nucleotide mutations compared to other DNA viruses, thus generating HBV mutants. Most of these newly mutated sequences cannot effectively compete with wild-type viruses or are not viable, but under selective pressure, these nascent sequences provide a starting point for mutant emergence. When discussing viral mutants, we often mention RNA viruses such as SARS-CoV-2 or influenza viruses, which can produce massive and rapid mutations. In contrast, HBV's circular DNA genome has four partially overlapping open reading frames (ORFs) that encode seven proteins. Because gene overlap limits viral variability, mutants or variant forms have been identified for four of these genes. HBV mutations have a certain tendency, with the "a" determinant on HBsAg being a region with a high mutation probability. Human immune responses to HBsAg are mainly directed at the disulfide bond conformational epitopes of the "a" determinant. When mutations occur, they cause changes in the amino acid sites of the corresponding protein and changes in the protein's spatial structure and characteristics (conformational epitopes), which can lead to the inability of antibodies to neutralize viral infection and may also affect diagnostic detection. Existing literatures have reported Q129R(3), M133T(4), D144A, D144E, G145R(5), and G145K(6), etc. Among them, the G145R mutant is the most important HBsAg mutant mentioned in the literatures. This mutant was first observed in children born to HBV-positive mothers who, despite vaccination, still experienced breakthrough infection. Although the children had protective anti-HBs antibodies against wild-type virus, the DNA and HBsAg of this G145R mutant persisted (5, 7). This suggests that the antibodies produced by the hepatitis B vaccine have a protective effect against wild-type HBV, but their protective effect against HBV mutant strains is limited.

The existing human hepatitis B surface antibody product is mainly hepatitis B immunoglobulin (HBIg), which is a concentrated passive immunizing agent to prevent the invasion and replication of hepatitis B virus. Its production method is to screen the plasma of healthy blood donors to find those with high titers of anti-HBs, and then use a bio-concentration process to produce high-titer hepatitis B immunoglobulin. However, blood-derived HBIg also presents several challenges. For instance, HBIg requires high-titer anti-HBs plasma as its raw material, limiting production volume and scale of application. Furthermore, individual variations in plasma supply between batches lead to differences in quality and efficacy, particularly in efficacy, where concentrated HBIg may contain only a portion of antibodies capable of neutralizing HBV. Despite strict national regulations on blood products, the testing of "healthy blood donors" remains limited, resulting in poor biosafety for HBIg and limiting its clinical dosage to 200 IU. Finally, patients receiving HBIg injections may experience allergic reactions, such as IgG4 allergy. In patients with immunosuppression, such as those undergoing liver transplantation, adverse symptoms like nausea, urticaria, and joint pain may occur. While the clinical use of HBIg to block HBV transmission demonstrates the feasibility of anti-HBs in this area, its existing problems highlight the urgent need for the development of engineered, mass-producible, highly efficient, and safe anti-HBs agents, ideally capable of blocking mutant strains.

In recent years, many laboratories both domestically and internationally have used biotechnology to isolate peripheral blood cells from hepatitis B vaccine recipients and then clone human antibodies from B cells. However, this method suffers from problems such as a single source and inability to cover different genotypes or clinical mutants. Therefore, there has been a persistent need in this field for hepatitis B surface antibodies with improved performance.

### SUMMARY OF THE INVENTION

In the present invention, the inventors screened individuals recovered from hepatitis B infection and hepatitis B vaccine recipients with high serum neutralizing activity, and obtained a series of monoclonal antibodies against HBsAg by isolating individual B cells from their bodies. In vivo and in vitro experiments both demonstrated that these monoclonal antibodies possess excellent antigen-binding ability, in vitro neutralizing potency, and broad-spectrum recognition of various mutant strains. Furthermore, the inventors found that in comparison with antibodies from hepatitis B vaccine recipients, the antibodies derived from individuals recovered from hepatitis B infection exhibit a more diverse range of mutant strains that can be recognized and neutralized. In vivo experiments in mice further confirmed the excellent in vivo antiviral efficacy of this series of antibodies, thus completing this invention.

The present invention provides an isolated antibody or antigen-binding fragment thereof, which specifically binds to hepatitis B surface antigen (HBsAg), wherein the isolated antibody or antigen-binding fragment thereof comprises a heavy chain variable region VH and a light chain variable region VL, said heavy chain variable region VH comprising complementarity-determining regions CDRH1, CDRH2, and CDRH3, said light chain variable region VL comprising complementarity-determining regions CDRL1, CDRL2, and CDRL3, wherein CDRH1, CDRH2, CDRH3, CDRL1, CDRL2, and CDRL3 are selected from the following amino acid sequences:
a) CDRH1 of SEQ ID NO:2, CDRH2 of SEQ ID NO:3, CDRH3 of SEQ ID NO:4, CDRL1 of SEQ ID NO:6, CDRL2 of SEQ ID NO:7 and CDRL3 of SEQ ID NO:8;
b) CDRH1 of SEQ ID NO:10, CDRH2 of SEQ ID NO:11, CDRH3 of SEQ ID NO:12, CDRL1 of SEQ ID NO:14, CDRL2 of SEQ ID NO:15 and CDRL3 of SEQ ID NO:16;
c) CDRH1 of SEQ ID NO:18, CDRH2 of SEQ ID NO:19, CDRH3 of SEQ ID NO:20, CDRL1 of SEQ ID NO:22, CDRL2 of SEQ ID NO:23 and CDRL3 of SEQ ID NO:24;
d) CDRH1 of SEQ ID NO:26, CDRH2 of SEQ ID NO:27, CDRH3 of SEQ ID NO:28, CDRL1 of SEQ ID NO:30, CDRL2 of SEQ ID NO:31 and CDRL3 of SEQ ID NO:32;
e) CDRH1 of SEQ ID NO: 34, CDRH2 of SEQ ID NO: 35, CDRH3 of SEQ ID NO: 36, CDRL1 of SEQ ID NO: 38, CDRL2 of SEQ ID NO: 39 and CDRL3 of SEQ ID NO: 40;
f) CDRH1 of SEQ ID NO:42, CDRH2 of SEQ ID NO:43, CDRH3 of SEQ ID NO:44, CDRL1 of SEQ ID NO:46, CDRL2 of SEQ ID NO:47 and CDRL3 of SEQ ID NO:48;
g) CDRH1 of SEQ ID NO:50, CDRH2 of SEQ ID NO:51, CDRH3 of SEQ ID NO:52, CDRL1 of SEQ ID NO:54, CDRL2 of SEQ ID NO:55 and CDRL3 of SEQ ID NO:56;
h) CDRH1 of SEQ ID NO:58, CDRH2 of SEQ ID NO:59, CDRH3 of SEQ ID NO:60, CDRL1 of SEQ ID NO:62, CDRL2 of SEQ ID NO:63 and CDRL3 of SEQ ID NO:64;
i) CDRH1 of SEQ ID NO:66, CDRH2 of SEQ ID NO:67, CDRH3 of SEQ ID NO:68, CDRL1 of SEQ ID NO:70, CDRL2 of SEQ ID NO:71 and CDRL3 of SEQ ID NO:72;
j) CDRH1 of SEQ ID NO:74, CDRH2 of SEQ ID NO:75, CDRH3 of SEQ ID NO:76, CDRL1 of SEQ ID NO:78, CDRL2 of SEQ ID NO:79 and CDRL3 of SEQ ID NO:80;
k) CDRH1 of SEQ ID NO:82, CDRH2 of SEQ ID NO:83, CDRH3 of SEQ ID NO:84, CDRL1 of SEQ ID NO:86, CDRL2 of SEQ ID NO:87 and CDRL3 of SEQ ID NO:88;
1) CDRH1 of SEQ ID NO:90, CDRH2 of SEQ ID NO:91, CDRH3 of SEQ ID NO:92, CDRL1 of SEQ ID NO:94, CDRL2 of SEQ ID NO:95 and CDRL3 of SEQ ID NO:96;
m) CDRH1 of SEQ ID NO:98, CDRH2 of SEQ ID NO:99, CDRH3 of SEQ ID NO:100, CDRL1 of SEQ ID NO:102, CDRL2 of SEQ ID NO:103 and CDRL3 of SEQ ID NO:104;
n) CDRH1 of SEQ ID NO:106, CDRH2 of SEQ ID NO:107, CDRH3 of SEQ ID NO:108, CDRL1 of SEQ ID NO:110, CDRL2 of SEQ ID NO:111 and CDRL3 of SEQ ID NO:112;
o) CDRH1 of SEQ ID NO:114, CDRH2 of SEQ ID NO:115, CDRH3 of SEQ ID NO:116, CDRL1 of SEQ ID NO:118, CDRL2 of SEQ ID NO:119 and CDRL3 of SEQ ID NO:120;
p) CDRH1 of SEQ ID NO:122, CDRH2 of SEQ ID NO:123, CDRH3 of SEQ ID NO:124, CDRL1 of SEQ ID NO:126, CDRL2 of SEQ ID NO:127 and CDRL3 of SEQ ID NO:128;
q) CDRH1 of SEQ ID NO:130, CDRH2 of SEQ ID NO:131, CDRH3 of SEQ ID NO:132, CDRL1 of SEQ ID NO:134, CDRL2 of SEQ ID NO:135 and CDRL3 of SEQ ID NO:136;
r) CDRH1 of SEQ ID NO:138, CDRH2 of SEQ ID NO:139, CDRH3 of SEQ ID NO:140, CDRL1 of SEQ ID NO:142, CDRL2 of SEQ ID NO:143 and CDRL3 of SEQ ID NO:144;
s) CDRH1 of SEQ ID NO:146, CDRH2 of SEQ ID NO:147, CDRH3 of SEQ ID NO:148, CDRL1 of SEQ ID NO:150, CDRL2 of SEQ ID NO:151 and CDRL3 of SEQ ID NO:152;
t) CDRH1 of SEQ ID NO:154, CDRH2 of SEQ ID NO:155, CDRH3 of SEQ ID NO:156, CDRL1 of SEQ ID NO:158, CDRL2 of SEQ ID NO:159 and CDRL3 of SEQ ID NO:160;
u) CDRH1 of SEQ ID NO:162, CDRH2 of SEQ ID NO:163, CDRH3 of SEQ ID NO:164, CDRL1 of SEQ ID NO:166, CDRL2 of SEQ ID NO:167 and CDRL3 of SEQ ID NO:168;
v) CDRH1 of SEQ ID NO: 170, CDRH2 of SEQ ID NO: 171, CDRH3 of SEQ ID NO: 172, CDRL1 of SEQ ID NO: 174, CDRL2 of SEQ ID NO: 175 and CDRL3 of SEQ ID NO: 176;
w) CDRH1 of SEQ ID NO:178, CDRH2 of SEQ ID NO:179, CDRH3 of SEQ ID NO:180, CDRL1 of SEQ ID NO:182, CDRL2 of SEQ ID NO:183 and CDRL3 of SEQ ID NO:184;
x) CDRH1 of SEQ ID NO:186, CDRH2 of SEQ ID NO:187, CDRH3 of SEQ ID NO:188, CDRL1 of SEQ ID NO:190, CDRL2 of SEQ ID NO:191 and CDRL3 of SEQ ID NO:192;
y) CDRH1 of SEQ ID NO:194, CDRH2 of SEQ ID NO:195, CDRH3 of SEQ ID NO:196, CDRL1 of SEQ ID NO:198, CDRL2 of SEQ ID NO:199 and CDRL3 of SEQ ID NO:200;
z) CDRH1 of SEQ ID NO:202, CDRH2 of SEQ ID NO:203, CDRH3 of SEQ ID NO:204, CDRL1 of SEQ ID NO:206, CDRL2 of SEQ ID NO:207 and CDRL3 of SEQ ID NO:208;
aa) CDRH1 of SEQ ID NO:210, CDRH2 of SEQ ID NO:211, CDRH3 of SEQ ID NO:212, CDRL1 of SEQ ID NO:214, CDRL2 of SEQ ID NO:215 and CDRL3 of SEQ ID NO:216;
bb) CDRH1 of SEQ ID NO:218, CDRH2 of SEQ ID NO:219, CDRH3 of SEQ ID NO:220, CDRL1 of SEQ ID NO:222, CDRL2 of SEQ ID NO:223 and CDRL3 of SEQ ID NO:224;
cc) CDRH1 of SEQ ID NO:226, CDRH2 of SEQ ID NO:227, CDRH3 of SEQ ID NO:228, CDRL1 of SEQ ID NO:230, CDRL2 of SEQ ID NO:231 and CDRL3 of SEQ ID NO:232;
dd) CDRH1 of SEQ ID NO:234, CDRH2 of SEQ ID NO:235, CDRH3 of SEQ ID NO:236, CDRL1 of SEQ ID NO:238, CDRL2 of SEQ ID NO:239 and CDRL3 of SEQ ID NO:240;
ee) CDRH1 of SEQ ID NO:242, CDRH2 of SEQ ID NO:243, CDRH3 of SEQ ID NO:244, CDRL1 of SEQ ID NO:246, CDRL2 of SEQ ID NO:247 and CDRL3 of SEQ ID NO:248;
ff) CDRH1 of SEQ ID NO:250, CDRH2 of SEQ ID NO:251, CDRH3 of SEQ ID NO:252, CDRL1 of SEQ ID NO:254, CDRL2 of SEQ ID NO:255 and CDRL3 of SEQ ID NO:256;
gg) CDRH1 of SEQ ID NO:258, CDRH2 of SEQ ID NO:259, CDRH3 of SEQ ID NO:260, CDRL1 of SEQ ID NO:262, CDRL2 of SEQ ID NO:263 and CDRL3 of SEQ ID NO:264;
hh) CDRH1 of SEQ ID NO:266, CDRH2 of SEQ ID NO:267, CDRH3 of SEQ ID NO:268, CDRL1 of SEQ ID NO:270, CDRL2 of SEQ ID NO:271 and CDRL3 of SEQ ID NO:272;
ii) CDRH1 of SEQ ID NO:274, CDRH2 of SEQ ID NO:275, CDRH3 of SEQ ID NO:276, CDRL1 of SEQ ID NO:278, CDRL2 of SEQ ID NO:279 and CDRL3 of SEQ ID NO:280;
jj) CDRH1 of SEQ ID NO:282, CDRH2 of SEQ ID NO:283, CDRH3 of SEQ ID NO:284, CDRL1 of SEQ ID NO:286, CDRL2 of SEQ ID NO:287 and CDRL3 of SEQ ID NO:288;
kk) CDRH1 of SEQ ID NO:290, CDRH2 of SEQ ID NO:291, CDRH3 of SEQ ID NO:292, CDRL1 of SEQ ID NO:294, CDRL2 of SEQ ID NO:295 and CDRL3 of SEQ ID NO:296;
11) CDRH1 of SEQ ID NO:298, CDRH2 of SEQ ID NO:299, CDRH3 of SEQ ID NO:300, CDRL1 of SEQ ID NO:302, CDRL2 of SEQ ID NO:303 and CDRL3 of SEQ ID NO:304;
mm) CDRH1 of SEQ ID NO:306, CDRH2 of SEQ ID NO:307, CDRH3 of SEQ ID NO:308, CDRL1 of SEQ ID NO:310, CDRL2 of SEQ ID NO:311 and CDRL3 of SEQ ID NO:312;
nn) CDRH1 of SEQ ID NO:314, CDRH2 of SEQ ID NO:315, CDRH3 of SEQ ID NO:316, CDRL1 of SEQ ID NO:318, CDRL2 of SEQ ID NO:319 and CDRL3 of SEQ ID NO:320;
oo) CDRH1 of SEQ ID NO:322, CDRH2 of SEQ ID NO:323, CDRH3 of SEQ ID NO:324, CDRL1 of SEQ ID NO:326, CDRL2 of SEQ ID NO:327 and CDRL3 of SEQ ID NO:328;
pp) CDRH1 of SEQ ID NO:330, CDRH2 of SEQ ID NO:331, CDRH3 of SEQ ID NO:332, CDRL1 of SEQ ID NO:334, CDRL2 of SEQ ID NO:335 and CDRL3 of SEQ ID NO:336;
qq) CDRH1 of SEQ ID NO:338, CDRH2 of SEQ ID NO:339, CDRH3 of SEQ ID NO:340, CDRL1 of SEQ ID NO:342, CDRL2 of SEQ ID NO:343 and CDRL3 of SEQ ID NO:344;
rr) CDRH1 of SEQ ID NO:346, CDRH2 of SEQ ID NO:347, CDRH3 of SEQ ID NO:348, CDRL1 of SEQ ID NO:350, CDRL2 of SEQ ID NO:351 and CDRL3 of SEQ ID NO:352;
ss) CDRH1 of SEQ ID NO:354, CDRH2 of SEQ ID NO:355, CDRH3 of SEQ ID NO:356, CDRL1 of SEQ ID NO:358, CDRL2 of SEQ ID NO:359 and CDRL3 of SEQ ID NO:360;
tt) CDRH1 of SEQ ID NO:362, CDRH2 of SEQ ID NO:363, CDRH3 of SEQ ID NO:364, CDRL1 of SEQ ID NO:366, CDRL2 of SEQ ID NO:367 and CDRL3 of SEQ ID NO:368;
uu) CDRH1 of SEQ ID NO:370, CDRH2 of SEQ ID NO:371, CDRH3 of SEQ ID NO:372, CDRL1 of SEQ ID NO:374, CDRL2 of SEQ ID NO:375 and CDRL3 of SEQ ID NO:376;
vv) CDRH1 of SEQ ID NO:378, CDRH2 of SEQ ID NO:379, CDRH3 of SEQ ID NO:380, CDRL1 of SEQ ID NO:382, CDRL2 of SEQ ID NO:383 and CDRL3 of SEQ ID NO:384;
ww) CDRH1 of SEQ ID NO:386, CDRH2 of SEQ ID NO:387, CDRH3 of SEQ ID NO:388, CDRL1 of SEQ ID NO:390, CDRL2 of SEQ ID NO:391 and CDRL3 of SEQ ID NO:392;
xx) CDRH1 of SEQ ID NO:394, CDRH2 of SEQ ID NO:395, CDRH3 of SEQ ID NO:396, CDRL1 of SEQ ID NO:398, CDRL2 of SEQ ID NO:399 and CDRL3 of SEQ ID NO:400;
yy) CDRH1 of SEQ ID NO:402, CDRH2 of SEQ ID NO:403, CDRH3 of SEQ ID NO:404, CDRL1 of SEQ ID NO:406, CDRL2 of SEQ ID NO:407 and CDRL3 of SEQ ID NO:408;
zz) CDRH1 of SEQ ID NO:410, CDRH2 of SEQ ID NO:411, CDRH3 of SEQ ID NO:412, CDRL1 of SEQ ID NO:414, CDRL2 of SEQ ID NO:415 and CDRL3 of SEQ ID NO:416;
aaa) CDRH1 of SEQ ID NO:418, CDRH2 of SEQ ID NO:419, CDRH3 of SEQ ID NO:420, CDRL1 of SEQ ID NO:422, CDRL2 of SEQ ID NO:423 and CDRL3 of SEQ ID NO:424;
bbb) CDRH1 of SEQ ID NO:426, CDRH2 of SEQ ID NO:427, CDRH3 of SEQ ID NO:428, CDRL1 of SEQ ID NO:430, CDRL2 of SEQ ID NO:431 and CDRL3 of SEQ ID NO:432;
ccc) CDRH1 of SEQ ID NO:434, CDRH2 of SEQ ID NO:435, CDRH3 of SEQ ID NO:436, CDRL1 of SEQ ID NO:438, CDRL2 of SEQ ID NO:439 and CDRL3 of SEQ ID NO:440;
ddd) CDRH1 of SEQ ID NO:442, CDRH2 of SEQ ID NO:443, CDRH3 of SEQ ID NO:444, CDRL1 of SEQ ID NO:446, CDRL2 of SEQ ID NO:447 and CDRL3 of SEQ ID NO:448;
eee) CDRH1 of SEQ ID NO:450, CDRH2 of SEQ ID NO:451, CDRH3 of SEQ ID NO:452, CDRL1 of SEQ ID NO:454, CDRL2 of SEQ ID NO:455 and CDRL3 of SEQ ID NO:456;
fff) CDRH1 of SEQ ID NO:458, CDRH2 of SEQ ID NO:459, CDRH3 of SEQ ID NO:460, CDRL1 of SEQ ID NO:462, CDRL2 of SEQ ID NO:463 and CDRL3 of SEQ ID NO:464;
ggg) CDRH1 of SEQ ID NO:466, CDRH2 of SEQ ID NO:467, CDRH3 of SEQ ID NO:468, CDRL1 of SEQ ID NO:470, CDRL2 of SEQ ID NO:471 and CDRL3 of SEQ ID NO:472;
hhh) CDRH1 of SEQ ID NO:474, CDRH2 of SEQ ID NO:475, CDRH3 of SEQ ID NO:476, CDRL1 of SEQ ID NO:478, CDRL2 of SEQ ID NO:479 and CDRL3 of SEQ ID NO:480;
iii) CDRH1 of SEQ ID NO:482, CDRH2 of SEQ ID NO:483, CDRH3 of SEQ ID NO:484, CDRL1 of SEQ ID NO:486, CDRL2 of SEQ ID NO:487 and CDRL3 of SEQ ID NO:488; and
jjj) CDRH1 of SEQ ID NO:490, CDRH2 of SEQ ID NO:491, CDRH3 of SEQ ID NO:492, CDRL1 of SEQ ID NO:494, CDRL2 of SEQ ID NO:495 and CDRL3 of SEQ ID NO:496.

In one embodiment, the heavy chain variable region VH and the light chain variable region VL are selected from the following amino acid sequences:
a) VH of SEQ ID NO:1 and VL of SEQ ID NO:5;
b) VH of SEQ ID NO:9 and VL of SEQ ID NO:13;
c) VH of SEQ ID NO:17 and VL of SEQ ID NO:21;
d) VH of SEQ ID NO:25 and VL of SEQ ID NO:29;
e) VH of SEQ ID NO:33 and VL of SEQ ID NO:37;
f) VH of SEQ ID NO:41 and VL of SEQ ID NO:45;
g) VH of SEQ ID NO:49 and VL of SEQ ID NO:53;
h) VH of SEQ ID NO:57 and VL of SEQ ID NO:61;
i) VH of SEQ ID NO:65 and VL of SEQ ID NO:69;
j) VH of SEQ ID NO:73 and VL of SEQ ID NO:77;
k) VH of SEQ ID NO:81 and VL of SEQ ID NO:85;
1) VH of SEQ ID NO:89 and VL of SEQ ID NO:93;
m) VH of SEQ ID NO:97 and VL of SEQ ID NO: 101;
n) VH of SEQ ID NO:105 and VL of SEQ ID NO:109;
o) VH of SEQ ID NO:113 and VL of SEQ ID NO:117;
p) VH of SEQ ID NO:121 and VL of SEQ ID NO:125;
q) VH of SEQ ID NO:129 and VL of SEQ ID NO:133;
r) VH of SEQ ID NO:137 and VL of SEQ ID NO:141;
s) VH of SEQ ID NO:145 and VL of SEQ ID NO:149;
t) VH of SEQ ID NO:153 and VL of SEQ ID NO:157;
u) VH of SEQ ID NO:161 and VL of SEQ ID NO:165;
v) VH of SEQ ID NO:169 and VL of SEQ ID NO:173;
w) VH of SEQ ID NO:177 and VL of SEQ ID NO:181;
x) VH of SEQ ID NO:185 and VL of SEQ ID NO:189;
y) VH of SEQ ID NO:193 and VL of SEQ ID NO:197;
z) VH of SEQ ID NO:201 and VL of SEQ ID NO:205;
aa) VH of SEQ ID NO:209 and VL of SEQ ID NO:213;
bb) VH of SEQ ID NO:217 and VL of SEQ ID NO:221;
cc) VH of SEQ ID NO:225 and VL of SEQ ID NO:229;
dd) VH of SEQ ID NO:233 and VL of SEQ ID NO:237;
ee) VH of SEQ ID NO:241 and VL of SEQ ID NO:245;
ff) VH of SEQ ID NO:249 and VL of SEQ ID NO:253;
gg) VH of SEQ ID NO:257 and VL of SEQ ID NO:261;
hh) VH of SEQ ID NO:265 and VL of SEQ ID NO:269;
ii) VH of SEQ ID NO:273 and VL of SEQ ID NO:277;
jj) VH of SEQ ID NO:281 and VL of SEQ ID NO:285;
kk) VH of SEQ ID NO:289 and VL of SEQ ID NO:293;
11) VH of SEQ ID NO:297 and VL of SEQ ID NO:301;
mm) VH of SEQ ID NO:305 and VL of SEQ ID NO:309;
nn) VH of SEQ ID NO:313 and VL of SEQ ID NO:317;
oo) VH of SEQ ID NO:321 and VL of SEQ ID NO:325;
pp) VH of SEQ ID NO:329 and VL of SEQ ID NO:333;
qq) VH of SEQ ID NO:337 and VL of SEQ ID NO:341;
rr) VH of SEQ ID NO:345 and VL of SEQ ID NO:349;
ss) VH of SEQ ID NO:353 and VL of SEQ ID NO:357;
tt) VH of SEQ ID NO:361 and VL of SEQ ID NO:365;
uu) VH of SEQ ID NO:369 and VL of SEQ ID NO:373;
vv) VH of SEQ ID NO:377 and VL of SEQ ID NO:381;
ww) VH of SEQ ID NO:385 and VL of SEQ ID NO:389;
xx) VH of SEQ ID NO:393 and VL of SEQ ID NO:397;
yy) VH of SEQ ID NO:401 and VL of SEQ ID NO:405;
zz) VH of SEQ ID NO:409 and VL of SEQ ID NO:413;
aaa) VH of SEQ ID NO:417 and VL of SEQ ID NO:421;
bbb) VH of SEQ ID NO:425 and VL of SEQ ID NO:429;
ccc) VH of SEQ ID NO:433 and VL of SEQ ID NO:437;
ddd) VH of SEQ ID NO:441 and VL of SEQ ID NO:445;
eee) VH of SEQ ID NO:449 and VL of SEQ ID NO:453;
fff) VH of SEQ ID NO:457 and VL of SEQ ID NO:461;
ggg) VH of SEQ ID NO:465 and VL of SEQ ID NO:469;
hhh) VH of SEQ ID NO:473 and VL of SEQ ID NO:477;
iii) VH of SEQ ID NO:481 and VL of SEQ ID NO:485; and
jjj) VH of SEQ ID NO:489 and VL of SEQ ID NO:493.

In one embodiment, the isolated antibody or antigen-binding fragment thereof specifically binds to an epitope in the S region of the HBsAg extracellular domain.

In one embodiment, the isolated antibody or antigen-binding fragment thereof specifically binds to HBV and its mutant strains. In one embodiment, the HBV mutant strain is a naturally occurring HBV mutant strain. In one embodiment, the HBV mutant strain is selected from G145R, K122R-G145R, and F134L.

In one embodiment, the isolated antibody or antigen-binding fragment thereof described herein neutralizes HBV and its mutant strains in vitro at a 50% inhibitory concentration (IC₅₀) of less than 0.5 µg/ml, as determined by the method based on this disclosure. In one embodiment, the HBV mutant strain is a naturally occurring HBV mutant strain. In one embodiment, the HBV mutant strain is selected from G145R, K122R-G145R, and F134L.

In one embodiment, the isolated antibody or antigen-binding fragment thereof has neutralizing activity against different HBV genotypes. In one embodiment, the genotypes are selected from genotypes A, B, C, and D.

In one embodiment, the isolated antibody or antigen-binding fragment thereof described herein is a monoclonal antibody, a polyclonal antibody, a recombinant antibody, a human antibody, a humanized antibody, a chimeric antibody, a multispecific antibody or an antibody fragment thereof.

In one embodiment, the antigen-binding fragment is a Fab fragment, a Fab' fragment, an F(ab')₂ fragment, an Fv fragment, a Diabody, or a single-chain antibody molecule.

In one embodiment, the isolated antibody or antigen-binding fragment thereof described herein is IgG1, IgG2, IgG3 or IgG4 isotype.

In one embodiment, the isolated antibody or antigen-binding fragment thereof described herein is bispecific or multispecific.

The present invention also provides a bispecific antibody comprising a first domain that specifically binds to a first epitope on hepatitis B surface antigen (HBsAg) and a second domain that specifically binds to a second epitope on HBsAg, wherein the first epitope and the second epitope are each independently selected from:
a) an epitopes comprising amino acid residues 110I, 122K, 125T, 134F, 141K, and 165W;
b) an epitope comprising amino acid residues 122K, 123T, 145G, 161Y, and 165W; and
c) an epitope comprising amino acid residues 109L-111P, 120P-130G, 133M-136S, 144D, 146N, 153P, 161Y, and 164E-167S,

wherein the numbering of amino acid residues is based on the amino acid positions shown in SEQ ID NO:497,
wherein the first epitope is different from the second epitope.

In one embodiment, the first domain comprises a heavy chain variable region VH and a light chain variable region VL, said heavy chain variable region VH comprising complementarity-determining regions CDRH1, CDRH2, and CDRH3, said light chain variable region VL comprising complementarity-determining regions CDRL1, CDRL2, and CDRL3, wherein CDRH1, CDRH2, CDRH3, CDRL1, CDRL2, and CDRL3 are selected from the following amino acid sequences:
a) CDRH1 of SEQ ID NO:178, CDRH2 of SEQ ID NO:179, CDRH3 of SEQ ID NO:180, CDRL1 of SEQ ID NO:182, CDRL2 of SEQ ID NO:183 and CDRL3 of SEQ ID NO:184;
b) CDRH1 of SEQ ID NO:210, CDRH2 of SEQ ID NO:211, CDRH3 of SEQ ID NO:212, CDRL1 of SEQ ID NO:214, CDRL2 of SEQ ID NO:215 and CDRL3 of SEQ ID NO:216;
c) CDRH1 of SEQ ID NO:266, CDRH2 of SEQ ID NO:267, CDRH3 of SEQ ID NO:268, CDRL1 of SEQ ID NO:270, CDRL2 of SEQ ID NO:271 and CDRL3 of SEQ ID NO:272;
d) CDRH1 of SEQ ID NO:434, CDRH2 of SEQ ID NO:435, CDRH3 of SEQ ID NO:436, CDRL1 of SEQ ID NO:438, CDRL2 of SEQ ID NO:439 and CDRL3 of SEQ ID NO:440; and
e) CDRH1 of SEQ ID NO:458, CDRH2 of SEQ ID NO:459, CDRH3 of SEQ ID NO:460, CDRL1 of SEQ ID NO:462, CDRL2 of SEQ ID NO:463 and CDRL3 of SEQ ID NO:464;
the second domain comprises a heavy chain variable region VH and a light chain variable region VL, said heavy chain variable region VH comprising complementarity-determining regions CDRH1, CDRH2, and CDRH3, said light chain variable region VL comprising complementarity-determining regions CDRL1, CDRL2, and CDRL3, wherein CDRH1, CDRH2, CDRH3, CDRL1, CDRL2, and CDRL3 are selected from the following amino acid sequences:
a) CDRH1 of SEQ ID NO:154, CDRH2 of SEQ ID NO:155, CDRH3 of SEQ ID NO:156, CDRL1 of SEQ ID NO:158, CDRL2 of SEQ ID NO:159 and CDRL3 of SEQ ID NO:160;
b) CDRH1 of SEQ ID NO:218, CDRH2 of SEQ ID NO:219, CDRH3 of SEQ ID NO:220, CDRL1 of SEQ ID NO:222, CDRL2 of SEQ ID NO:223 and CDRL3 of SEQ ID NO:224;
c) CDRH1 of SEQ ID NO:234, CDRH2 of SEQ ID NO:235, CDRH3 of SEQ ID NO:236, CDRL1 of SEQ ID NO:238, CDRL2 of SEQ ID NO:239 and CDRL3 of SEQ ID NO:240;
d) CDRH1 of SEQ ID NO:282, CDRH2 of SEQ ID NO:283, CDRH3 of SEQ ID NO:284, CDRL1 of SEQ ID NO:286, CDRL2 of SEQ ID NO:287 and CDRL3 of SEQ ID NO:288; and
e) CDRH1 of SEQ ID NO:314, CDRH2 of SEQ ID NO:315, CDRH3 of SEQ ID NO:316, CDRL1 of SEQ ID NO:318, CDRL2 of SEQ ID NO:319 and CDRL3 of SEQ ID NO:320.

In one embodiment, the first domain comprises a heavy chain variable region VH and a light chain variable region VL selected from the following amino acid sequences:
a) VH of SEQ ID NO:177 and VL of SEQ ID NO:181;
b) VH of SEQ ID NO:209 and VL of SEQ ID NO:213;
c) VH of SEQ ID NO:265 and VL of SEQ ID NO:269;
d) VH of SEQ ID NO:433 and VL of SEQ ID NO:437; and
e) VH of SEQ ID NO:457 and VL of SEQ ID NO:461;
   or amino acid sequences that have at least 80%, 85%, 90%, 95%, 96%, 97%, 98%, 99%, or 100% sequence identity with any of the above sets of sequences,
   the second domain comprises a heavy chain variable region VH and a light chain variable region VL selected from the following amino acid sequences:
      a) VH of SEQ ID NO:153 and VL of SEQ ID NO:157;
      b) VH of SEQ ID NO:217 and VL of SEQ ID NO:221;
      c) VH of SEQ ID NO:233 and VL of SEQ ID NO:237;
      d) VH of SEQ ID NO:281 and VL of SEQ ID NO:285; and
      e) VH of SEQ ID NO:313 and VL of SEQ ID NO:317;
   or amino acid sequences that have at least 80%, 85%, 90%, 95%, 96%, 97%, 98%, 99%, or 100% sequence identity with any of the above sets of sequences.

In one embodiment, the first domain comprises a heavy chain variable region VH and a light chain variable region VL, said heavy chain variable region VH comprising complementarity-determining regions CDRH1, CDRH2, and CDRH3, said light chain variable region VL comprising complementarity-determining regions CDRL1, CDRL2, and CDRL3, wherein CDRH1, CDRH2, CDRH3, CDRL1, CDRL2, and CDRL3 are selected from the following amino acid sequences:
a) CDRH1 of SEQ ID NO:178, CDRH2 of SEQ ID NO:179, CDRH3 of SEQ ID NO:180, CDRL1 of SEQ ID NO:182, CDRL2 of SEQ ID NO:183 and CDRL3 of SEQ ID NO:184;
b) CDRH1 of SEQ ID NO:210, CDRH2 of SEQ ID NO:211, CDRH3 of SEQ ID NO:212, CDRL1 of SEQ ID NO:214, CDRL2 of SEQ ID NO:215 and CDRL3 of SEQ ID NO:216;
c) CDRH1 of SEQ ID NO:266, CDRH2 of SEQ ID NO:267, CDRH3 of SEQ ID NO:268, CDRL1 of SEQ ID NO:270, CDRL2 of SEQ ID NO:271 and CDRL3 of SEQ ID NO:272;
d) CDRH1 of SEQ ID NO:434, CDRH2 of SEQ ID NO:435, CDRH3 of SEQ ID NO:436, CDRL1 of SEQ ID NO:438, CDRL2 of SEQ ID NO:439 and CDRL3 of SEQ ID NO:440; and
e) CDRH1 of SEQ ID NO:458, CDRH2 of SEQ ID NO:459, CDRH3 of SEQ ID NO:460, CDRL1 of SEQ ID NO:462, CDRL2 of SEQ ID NO:463 and CDRL3 of SEQ ID NO:464;
the second domain comprises a heavy chain variable region VH and a light chain variable region VL, said heavy chain variable region VH comprising complementarity-determining regions CDRH1, CDRH2, and CDRH3, said light chain variable region VL comprising complementarity-determining regions CDRL1, CDRL2, and CDRL3, wherein CDRH1, CDRH2, CDRH3, CDRL1, CDRL2, and CDRL3 are selected from the following amino acid sequences:
a) CDRH1 of SEQ ID NO:2, CDRH2 of SEQ ID NO:3, CDRH3 of SEQ ID NO:4, CDRL1 of SEQ ID NO:6, CDRL2 of SEQ ID NO:7 and CDRL3 of SEQ ID NO:8;
b) CDRH1 of SEQ ID NO:50, CDRH2 of SEQ ID NO:51, CDRH3 of SEQ ID NO:52, CDRL1 of SEQ ID NO:54, CDRL2 of SEQ ID NO:55 and CDRL3 of SEQ ID NO:56;
c) CDRH1 of SEQ ID NO:82, CDRH2 of SEQ ID NO:83, CDRH3 of SEQ ID NO:84, CDRL1 of SEQ ID NO:86, CDRL2 of SEQ ID NO:87 and CDRL3 of SEQ ID NO:88;
d) CDRH1 of SEQ ID NO:90, CDRH2 of SEQ ID NO:91, CDRH3 of SEQ ID NO:92, CDRL1 of SEQ ID NO:94, CDRL2 of SEQ ID NO:95 and CDRL3 of SEQ ID NO:96;
e) CDRH1 of SEQ ID NO:106, CDRH2 of SEQ ID NO:107, CDRH3 of SEQ ID NO:108, CDRL1 of SEQ ID NO:110, CDRL2 of SEQ ID NO:111 and CDRL3 of SEQ ID NO:112;
f) CDRH1 of SEQ ID NO:114, CDRH2 of SEQ ID NO:115, CDRH3 of SEQ ID NO:116, CDRL1 of SEQ ID NO:118, CDRL2 of SEQ ID NO:119 and CDRL3 of SEQ ID NO:120;
g) CDRH1 of SEQ ID NO:322, CDRH2 of SEQ ID NO:323, CDRH3 of SEQ ID NO:324, CDRL1 of SEQ ID NO:326, CDRL2 of SEQ ID NO:327 and CDRL3 of SEQ ID NO:328;
j) CDRH1 of SEQ ID NO:346, CDRH2 of SEQ ID NO:347, CDRH3 of SEQ ID NO:348, CDRL1 of SEQ ID NO:350, CDRL2 of SEQ ID NO:351 and CDRL3 of SEQ ID NO:352;
i) CDRH1 of SEQ ID NO:354, CDRH2 of SEQ ID NO:355, CDRH3 of SEQ ID NO:356, CDRL1 of SEQ ID NO:358, CDRL2 of SEQ ID NO:359 and CDRL3 of SEQ ID NO:360;
j) CDRH1 of SEQ ID NO:378, CDRH2 of SEQ ID NO:379, CDRH3 of SEQ ID NO:380, CDRL1 of SEQ ID NO:382, CDRL2 of SEQ ID NO:383 and CDRL3 of SEQ ID NO:384;
k) CDRH1 of SEQ ID NO:386, CDRH2 of SEQ ID NO:387, CDRH3 of SEQ ID NO:388, CDRL1 of SEQ ID NO:390, CDRL2 of SEQ ID NO:391 and CDRL3 of SEQ ID NO:392; and
l) CDRH1 of SEQ ID NO:418, CDRH2 of SEQ ID NO:419, CDRH3 of SEQ ID NO:420, CDRL1 of SEQ ID NO:422, CDRL2 of SEQ ID NO:423 and CDRL3 of SEQ ID NO:424.

In one embodiment, the first domain comprises a heavy chain variable region VH and a light chain variable region VL selected from the following amino acid sequences:
a) VH of SEQ ID NO:177 and VL of SEQ ID NO:181;
b) VH of SEQ ID NO:209 and VL of SEQ ID NO:213;
c) VH of SEQ ID NO:265 and VL of SEQ ID NO:269;
d) VH of SEQ ID NO:433 and VL of SEQ ID NO:437; and
e) VH of SEQ ID NO:457 and VL of SEQ ID NO:461;
   or amino acid sequences that have at least 80%, 85%, 90%, 95%, 96%, 97%, 98%, 99%, or 100% sequence identity with any of the above sets of sequences,
   the second domain comprises a heavy chain variable region VH and a light chain variable region VL selected from the following amino acid sequences:
      a) VH of SEQ ID NO:1 and VL of SEQ ID NO:5;
      b) VH of SEQ ID NO:49 and VL of SEQ ID NO:53;
      c) VH of SEQ ID NO:81 and VL of SEQ ID NO:85;
      d) VH of SEQ ID NO:89 and VL of SEQ ID NO:93;
      e) VH of SEQ ID NO:105 and VL of SEQ ID NO:109;
      f) VH of SEQ ID NO:113 and VL of SEQ ID NO:117;
      g) VH of SEQ ID NO:321 and VL of SEQ ID NO:325;
      h) VH of SEQ ID NO:345 and VL of SEQ ID NO:349;
      i) VH of SEQ ID NO:353 and VL of SEQ ID NO:357;
      j) VH of SEQ ID NO:377 and VL of SEQ ID NO:381;
      k) VH of SEQ ID NO:385 and VL of SEQ ID NO:389; and
      l) VH of SEQ ID NO:417 and VL of SEQ ID NO:421;
or amino acid sequences that have at least 80%, 85%, 90%, 95%, 96%, 97%, 98%, 99%, or 100% sequence identity with any of the above sets of sequences.

In one embodiment, the first domain comprises a heavy chain variable region VH and a light chain variable region VL, said heavy chain variable region VH comprising complementarity-determining regions CDRH1, CDRH2, and CDRH3, said light chain variable region VL comprising complementarity-determining regions CDRL1, CDRL2, and CDRL3, wherein CDRH1, CDRH2, CDRH3, CDRL1, CDRL2, and CDRL3 are selected from the following amino acid sequences:
a) CDRH1 of SEQ ID NO:154, CDRH2 of SEQ ID NO:155, CDRH3 of SEQ ID NO:156, CDRL1 of SEQ ID NO:158, CDRL2 of SEQ ID NO:159 and CDRL3 of SEQ ID NO:160;
b) CDRH1 of SEQ ID NO:218, CDRH2 of SEQ ID NO:219, CDRH3 of SEQ ID NO:220, CDRL1 of SEQ ID NO:222, CDRL2 of SEQ ID NO:223 and CDRL3 of SEQ ID NO:224;
c) CDRH1 of SEQ ID NO:234, CDRH2 of SEQ ID NO:235, CDRH3 of SEQ ID NO:236, CDRL1 of SEQ ID NO:238, CDRL2 of SEQ ID NO:239 and CDRL3 of SEQ ID NO:240;
d) CDRH1 of SEQ ID NO:282, CDRH2 of SEQ ID NO:283, CDRH3 of SEQ ID NO:284, CDRL1 of SEQ ID NO:286, CDRL2 of SEQ ID NO:287 and CDRL3 of SEQ ID NO:288; and
e) CDRH1 of SEQ ID NO:314, CDRH2 of SEQ ID NO:315, CDRH3 of SEQ ID NO:316, CDRL1 of SEQ ID NO:318, CDRL2 of SEQ ID NO:319 and CDRL3 of SEQ ID NO:320;
the second domain comprises a heavy chain variable region VH and a light chain variable region VL, said heavy chain variable region VH comprising complementarity-determining regions CDRH1, CDRH2, and CDRH3, said light chain variable region VL comprising complementarity-determining regions CDRL1, CDRL2, and CDRL3, wherein CDRH1, CDRH2, CDRH3, CDRL1, CDRL2, and CDRL3 are selected from the following amino acid sequences:
a) CDRH1 of SEQ ID NO:2, CDRH2 of SEQ ID NO:3, CDRH3 of SEQ ID NO:4, CDRL1 of SEQ ID NO:6, CDRL2 of SEQ ID NO:7 and CDRL3 of SEQ ID NO:8;
b) CDRH1 of SEQ ID NO:50, CDRH2 of SEQ ID NO:51, CDRH3 of SEQ ID NO:52, CDRL1 of SEQ ID NO:54, CDRL2 of SEQ ID NO:55 and CDRL3 of SEQ ID NO:56;
c) CDRH1 of SEQ ID NO:82, CDRH2 of SEQ ID NO:83, CDRH3 of SEQ ID NO:84, CDRL1 of SEQ ID NO:86, CDRL2 of SEQ ID NO:87 and CDRL3 of SEQ ID NO:88;
d) CDRH1 of SEQ ID NO:90, CDRH2 of SEQ ID NO:91, CDRH3 of SEQ ID NO:92, CDRL1 of SEQ ID NO:94, CDRL2 of SEQ ID NO:95 and CDRL3 of SEQ ID NO:96;
e) CDRH1 of SEQ ID NO:106, CDRH2 of SEQ ID NO:107, CDRH3 of SEQ ID NO:108, CDRL1 of SEQ ID NO:110, CDRL2 of SEQ ID NO:111 and CDRL3 of SEQ ID NO:112;
f) CDRH1 of SEQ ID NO:114, CDRH2 of SEQ ID NO:115, CDRH3 of SEQ ID NO:116, CDRL1 of SEQ ID NO:118, CDRL2 of SEQ ID NO:119 and CDRL3 of SEQ ID NO:120;
g) CDRH1 of SEQ ID NO:322, CDRH2 of SEQ ID NO:323, CDRH3 of SEQ ID NO:324, CDRL1 of SEQ ID NO:326, CDRL2 of SEQ ID NO:327 and CDRL3 of SEQ ID NO:328;
h) CDRH1 of SEQ ID NO:346, CDRH2 of SEQ ID NO:347, CDRH3 of SEQ ID NO:348, CDRL1 of SEQ ID NO:350, CDRL2 of SEQ ID NO:351 and CDRL3 of SEQ ID NO:352;
i) CDRH1 of SEQ ID NO:354, CDRH2 of SEQ ID NO:355, CDRH3 of SEQ ID NO:356, CDRL1 of SEQ ID NO:358, CDRL2 of SEQ ID NO:359 and CDRL3 of SEQ ID NO:360;
j) CDRH1 of SEQ ID NO:378, CDRH2 of SEQ ID NO:379, CDRH3 of SEQ ID NO:380, CDRL1 of SEQ ID NO:382, CDRL2 of SEQ ID NO:383 and CDRL3 of SEQ ID NO:384;
k) CDRH1 of SEQ ID NO:386, CDRH2 of SEQ ID NO:387, CDRH3 of SEQ ID NO:388, CDRL1 of SEQ ID NO:390, CDRL2 of SEQ ID NO:391 and CDRL3 of SEQ ID NO:392; and
l) CDRH1 of SEQ ID NO:418, CDRH2 of SEQ ID NO:419, CDRH3 of SEQ ID NO:420, CDRL1 of SEQ ID NO:422, CDRL2 of SEQ ID NO:423 and CDRL3 of SEQ ID NO:424.

In one embodiment, the first domain comprises a heavy chain variable region VH and a light chain variable region VL selected from the following amino acid sequences:
a) VH of SEQ ID NO:153 and VL of SEQ ID NO:157;
b) VH of SEQ ID NO:217 and VL of SEQ ID NO:221;
c) VH of SEQ ID NO:233 and VL of SEQ ID NO:237;
d) VH of SEQ ID NO:281 and VL of SEQ ID NO:285; and
e) VH of SEQ ID NO:313 and VL of SEQ ID NO:317;
   or amino acid sequences that have at least 80%, 85%, 90%, 95%, 96%, 97%, 98%, 99%, or 100% sequence identity with any of the above sets of sequences,
   the second domain comprises a heavy chain variable region VH and a light chain variable region VL selected from the following amino acid sequences:
      a) VH of SEQ ID NO:1 and VL of SEQ ID NO:5;
      b) VH of SEQ ID NO:49 and VL of SEQ ID NO:53;
      c) VH of SEQ ID NO:81 and VL of SEQ ID NO:85;
      d) VH of SEQ ID NO:89 and VL of SEQ ID NO:93;
      e) VH of SEQ ID NO:105 and VL of SEQ ID NO:109;
      f) VH of SEQ ID NO:113 and VL of SEQ ID NO:117;
      g) VH of SEQ ID NO:321 and VL of SEQ ID NO:325;
      h) VH of SEQ ID NO:345 and VL of SEQ ID NO:349;
      i) VH of SEQ ID NO:353 and VL of SEQ ID NO:357;
      j) VH of SEQ ID NO:377 and VL of SEQ ID NO:381;
      k) VH of SEQ ID NO:385 and VL of SEQ ID NO:389; and
      l) VH of SEQ ID NO:417 and VL of SEQ ID NO:421;
or amino acid sequences that have at least 80%, 85%, 90%, 95%, 96%, 97%, 98%, 99%, or 100% sequence identity with any of the above sets of sequences.

In one specific embodiment, the first domain comprises a heavy chain variable region VH and a light chain variable region VL, said heavy chain variable region VH comprising CDRH1 of SEQ ID NO:282, CDRH2 of SEQ ID NO:283, CDRH3 of SEQ ID NO:284, CDRL1 of SEQ ID NO:286, CDRL2 of SEQ ID NO:287, and CDRL3 of SEQ ID NO:288; and the second domain comprises a heavy chain variable region VH and a light chain variable region VL, said heavy chain variable region VH comprising CDRH1 of SEQ ID NO:50, CDRH2 of SEQ ID NO:51, CDRH3 of SEQ ID NO:52, CDRL1 of SEQ ID NO:54, CDRL2 of SEQ ID NO:55, and CDRL3 of SEQ ID NO:56.

In another specific embodiment, the first domain comprises VH of SEQ ID NO:281 and VL of SEQ ID NO:285, and the second domain comprises VH of SEQ ID NO:49 and VL of SEQ ID NO:53.

In one embodiment, the first domain and/or the second domain are IgG1, IgG2, IgG3 or IgG4 isotype.

In one embodiment, the first domain is a full-length IgG antibody and the second domain is an scfv; or the first domain is an scfv and the second domain is a full-length IgG antibody.

In one embodiment, the N-terminus of the scfv is connected to the C-terminus of at least one heavy chain of the full-length IgG antibody; or the N-terminus of the scfv is connected to the C-terminus of at least one light chain of the full-length IgG antibody.

In one embodiment, the first domain is connected to the second domain by a flexible peptide linker, preferably the flexible peptide linker has a length of no more than about 30 amino acids, and more preferably the flexible peptide linker is (G4S)₃. In one embodiment, the scfv is connected to a full-length IgG antibody via a flexible peptide linker.

In one embodiment, the bispecific antibody is CrossMAb-Fab.

The present invention also provides an isolated polynucleotide encoding the isolated antibody or antigen-binding fragment thereof described herein.

The present invention also provides an expression vector comprising the polynucleotides described herein.

The present invention also provides a host cell comprising the expression vector described herein.

The present invention also provides a method for generating the isolated antibody or antigen-binding fragment thereof described herein, wherein the method comprises culturing the host cell described herein under conditions allowing expression of the antibody or antigen-binding fragment thereof, and recovering the antibody or antigen-binding fragment thereof produced by the host cell.

The present invention also provides a pharmaceutical composition comprising the isolated antibody or antigen-binding fragment thereof or bispecific antibody described herein, and a pharmaceutically acceptable carrier.

The present invention also provides the use of the isolated antibody or antigen-binding fragment thereof or bispecific antibody in the preparation of a medicament for treating or preventing HBV infection in subjects in need thereof.

The present invention also provides the use of the isolated antibody or antigen-binding fragment thereof or bispecific antibody in the preparation of a medicament for the treatment or prevention of HBV and its mutant strain infection in subjects in need thereof.

In some aspects, the present invention provides a method for treating or preventing HBV infection in a subject in need thereof, the method comprising administering to the subject a therapeutically effective amount of at least one isolated antibody or antigen-binding fragment thereof or bispecific antibody disclosed herein.

In some aspects, the present invention provides a method for treating or preventing HBV and its mutant strain infection in a subject in need thereof, the method comprising administering to the subject a therapeutically effective amount of at least one isolated antibody or antigen-binding fragment thereof or bispecific antibody disclosed herein.

In some respects, the present invention provides an isolated antibody or antigen-binding fragment thereof or bispecific antibody disclosed herein, for use in the treatment or prevention of HBV infection in a subject in need thereof.

In some respects, the present invention provides an isolated antibody or antigen-binding fragment thereof or bispecific antibody disclosed herein, for use in the treatment or prevention of HBV and its mutant strain infection in a subject in need thereof.

In one embodiment of the above-mentioned aspects, the HBV includes genotypes A, B, C, and D.

In some aspects, the present invention provides a hepatitis B virus surface antibody or an antigen-binding fragment thereof specifically binding to an epitope in the S region of the extracellular domain of HBsAg. In one embodiment, the epitope is located at amino acid residues 99-169 of the S region of HBsAg. In one embodiment, the epitope is located at amino acid residues 124-147 of the S region of HBsAg. In one embodiment, the HBsAg has the amino acid sequence shown in SEQ ID NO:497. In one embodiment, the numbering of the amino acid residues is based on the amino acid positions shown in SEQ ID NO:497. In some embodiments, the antibody or antigen-binding fragment thereof binds to an epitope in the amino acid sequence having at least 90% identity with SEQ ID NO:497 or its above-described subsequences.

### BRIEF DESCRIPTION OF THE DRAWINGS

The invention will be better understood in conjunction with the accompanying drawings.
Figure 1 shows a schematic diagram of the extracellular domain of HBsAg, illustrating the configuration of amino acid residues 99-169.
Figure 2 shows the antibody responses in recovered hepatitis B patients and those vaccinated against hepatitis B. The neutralizing effect of antibodies against HBV was assessed in an HBV cell infection model at different serum dilutions.
Figure 3 shows the gating strategy during cell sorting. Double-positive (bait protein-APC+ and bait protein-PE+) cells were used for antibody cloning after single-cell sorting.
Figure 4 shows the results of flow cytometry sorting of B cells with different donor specificities, illustrating the frequency of B lymphocytes recognizing HBsAg from hepatitis B vaccine-immunized control donors (low anti-HBs levels) and selected hepatitis B recovered patients and hepatitis B vaccine-immunized individuals. Representative flow cytometry results show biotinylated HBsAg labeled with phycocyanin (APC) and phycoerythrin (PE), respectively, used in a dual-fluorescence-dye sorting strategy.
Figure 5 shows the area under the curve (AUC) value of antibody-bound HBsAg protein ELISA.
Figure 6 shows the neutralizing activity of antibodies assessed in an HBV cell infection model. In a cell model of HBV infection in vitro based on HepG2-NTCP cells, the levels of HBV antigens HBsAg and HBeAg in the cell supernatant represent the neutralizing efficacy of human monoclonal antibodies.

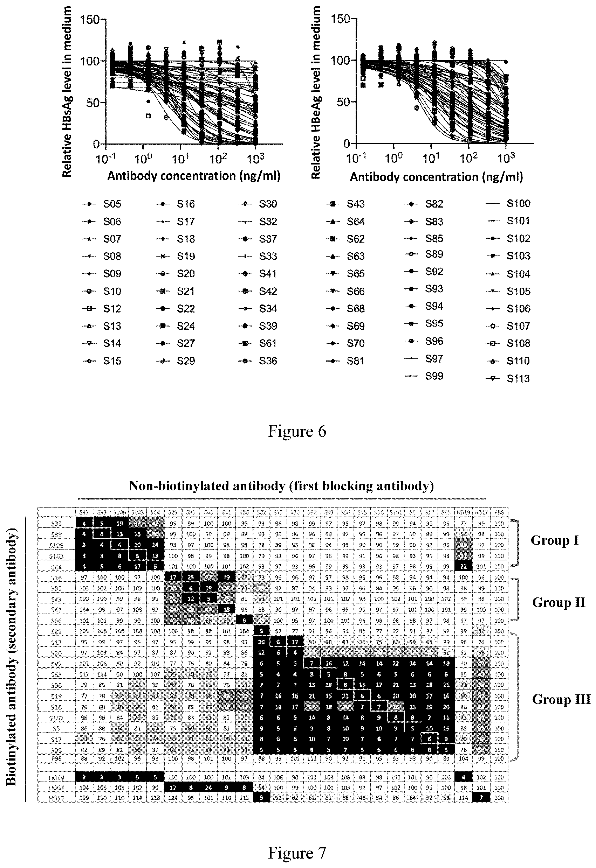

Figure 7 shows the competitive ELISA and the grouping of three major hepatitis B surface antibodies. A competitive ELISA was used to validate three non-competitive epitopes on HBsAg. The primary antibody was an unlabeled HBsAg-binding antibody used to block epitopes on coating HBsAg. The secondary antibody was a biotinylated HBsAg antibody used for streptavidin-HRP-dependent detection. H019 was the group I control antibody, H017 was the group III control antibody, and H007 was the group II control antibody. PBS buffer was used as a normalized reference instead of the primary blocking antibody.
Figure 8 shows the results of the antibody amino acid scanning. Consistent with the results of the competitive ELISA, the antibodies in the four groups showed their binding levels to HBsAg at specific mutated amino acid sites. The binding level of each antibody to wild-type HBsAg was used as a normalized reference for its binding to other HBsAg mutants, with PBS serving as a negative control.
Figure 9 shows the neutralizing activity of antibodies against different genotypes (genotypes A, B, and C) of HBV in an in vitro model. The HBV antigen-HBsAg level in the cell supernatant represents the neutralizing potency of the human monoclonal antibody. Antibodies capable of neutralizing HBV are shown.
Figure 10 shows the results of antibody binding to different HBV mutants. The binding efficacy of different HBsAg mutants produced by transfected cells to the antibodies was detected by ELISA. The binding level of each antibody to wild-type HBsAg served as a normalized reference for its binding to other HBsAg mutants, and PBS was used as a negative control.
Figure 11 shows the neutralizing activity against HBV mutant strains (HBV-G145R, HBV-K122R G145R, and HBV-F134L).
Figure 12 shows an exemplary design scheme for bispecific antibodies.
Figure 13 shows the results of physicochemical property detection (A) and SDS PAGE (B) of an exemplary bispecific antibody.
Figure 14 shows the OD values of an exemplary bispecific antibody-bound HBsAg protein ELISA.
Figure 15 shows the evaluation of the neutralizing activity of an exemplary bispecific antibody in an HBV cell infection model. In a cell model of HBV infection in vitro based on HepG2-NTCP cells, the levels of HBV antigens HBsAg and HBeAg in the cell supernatant represent the neutralizing potency of the antibody.

### DETAILED DESCRIPTION OF THE INVENTION

The present disclosure provides antibodies or antigen-binding fragments thereof that specifically bind to HBsAg. In some embodiments, the antibody or antigen-binding fragment thereof binds to an epitope in the extracellular domain of HBsAg. In some embodiments, the antibody or antigen-binding fragment thereof exhibits excellent antigen-binding ability, in vitro neutralizing potency, and broad-spectrum recognition of various mutant strains. In some embodiments, the antibody is a human monoclonal antibody.

For convenience, the following sections outline the meanings of the various terms used herein, followed by a discussion of various aspects of antibodies or antigen-binding fragments thereof, and then demonstrate various embodiments of antibodies or antigen-binding fragments thereof through specific examples.

### I. Definition

It should be understood that the foregoing general description and the following detailed description are illustrative and explanatory only, and are not intended to limit the claimed invention. In the present application, unless otherwise expressly stated, the use of the singular includes the plural. In the present application, unless otherwise stated, the use of "or" means "and/or". Furthermore, the use of the term "including" and other forms such as "comprising" is not limiting and covers the term "consisting of".

The section headings used in the disclosure are for the purpose of organization only and should not be construed as limiting the subject matter. All references, or portions thereof, cited in the present application, including but not limited to patents, patent applications, articles, books, and papers, are incorporated herein by reference in their entirety for any purpose.

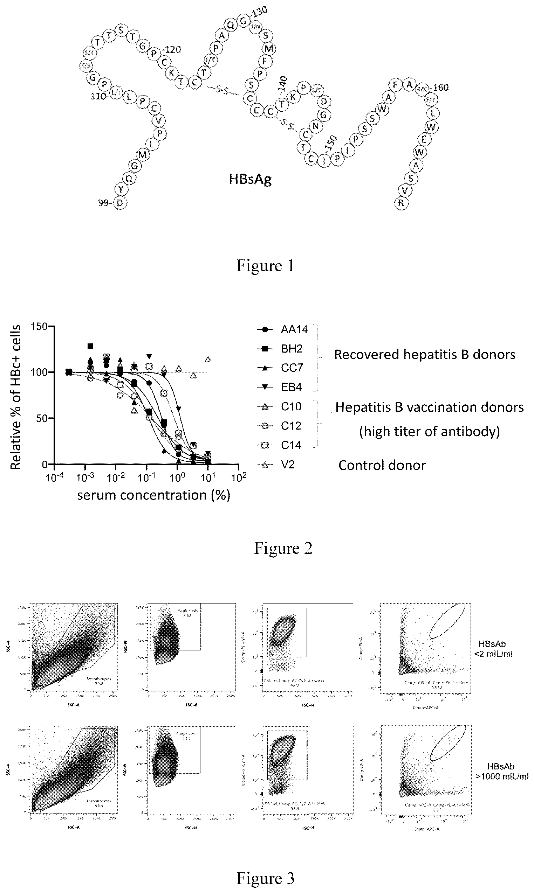

The term "hepatitis B surface antigen (HBsAg)" refers to the surface antigen contained in the outer shell of the hepatitis B virus. It is crucial for viral infection of host cells and is also an important site for recognition by the body's immune system (such as antibodies). The HBsAg protein contains four transmembrane regions, including an extracellular domain of approximately 70 amino acids in the S region, as shown in Figure 1. The full-length amino acid sequence of the HBsAg protein is shown in SEQ ID NO:497, where the extracellular domain of amino acid residues 99-169 is underlined.

MESTTSGFLGPLLVLQAGFFLLTRILTIPQSLDSWWTSLNFLGGAPTCPGQNSQSPTSNHSPT
SCPPICPGYRWMCLRRFIIFLFILLLCLIFLLVLLDYQGMLPVCPLLPGTSTTSTGPCKTCTIPA
QGTSMFPSCCCTKPSDGNCTCIPIPSSWAFARFLWEWASVRFSWLSLLVPFVQWFAGLSPT
VWLSVIWMMWYWGPSLYNILSPFLPLLPIFFCLWVYI (SEQ ID NO:497)

The terms "polynucleotide" or " nucleic acid " include both single-stranded and double-stranded nucleotide polymers. Nucleotides contained in the polynucleotides can be ribonucleotides, deoxyribonucleotides, or any type of nucleotide in modified form. The modifications include: base modifications, such as bromouridine and inosine derivatives; ribose modifications, such as 2',3'-dideoxyribose; and internucleotide linkage modifications, such as phosphorothioate, phosphorodithioate, phosphoroselenoate, phosphorodiselenoate, and phosphoramidate. The term "oligonucleotide" refers to a polynucleotide containing 200 or fewer nucleotides. In some embodiments, the oligonucleotide is 10-60 bases long. In other embodiments, the oligonucleotide is 12, 13, 14, 15, 16, 17, 18, 19, or 20-40 nucleotides long. Oligonucleotides can be single-stranded or double-stranded, such as for constructing mutant genes . Oligonucleotides can be sense or antisense oligonucleotides. Oligonucleotides may include labels for detection assays, including radioactive labels, fluorescent labels, hapten or antigen labels. Oligonucleotides can be used as, for example, PCR primers, cloning primers, or hybridization probes.

The term "vector " refers to any molecule or entity (such as nucleic acid, plasmid, bacteriophage , or virus) used to transfer protein-coding information to a host cell.

The term "expression vector" or "expression construct" refers to a vector suitable for transformation of host cells and containing a nucleic acid sequence that directs and/or regulates the expression of one or more heterologous coding regions operably linked thereto. Expression constructs may include, but are not limited to: sequences that affect or regulate transcription or translation; and, if introns are present, sequences that affect RNA splicing of coding region operably linked thereto.

As used herein, "operably linked" means that the components using this term are in a relationship that allows the components to perform their inherent functions under suitable conditions. For example, a regulatory sequence in a vector that is "operably linked" to a protein-coding sequence is linked to the protein-coding sequence to enable the expression of the protein-coding sequence under conditions compatible with the transcriptional activity of the regulatory sequence.

The term "host cell" refers to a cell that has been or can be transformed by a nucleic acid sequence to express a target gene. Regardless of whether the offspring are morphologically or genetically identical to the original parent cell, the term includes the offspring of the parent cell as long as the target gene is present in the offspring.

The term "transfection" refers to the absorption of foreign or exogenous DNA by a cell, which is "transfected" when the exogenous DNA is introduced into the cell membrane. Various transfection techniques are well known in the art and are disclosed herein. See, for example, Graham et al., 1973, Virology 52:456; Sambrook et al., 2001, Molecular Cloning: A Laboratory Manual; Davis et al., 1986, Basic Methods in Molecular Biology, Elsevier; Chu et al., 1981, Gene 13:197. These techniques can be used to introduce one or more exogenous DNA moieties into suitable host cells.

The term "transformation" refers to a change in the genetic characteristics of a cell, which is transformed when it is modified to contain new DNA or RNA. For example, a cell is transformed when its native state is genetically modified by introducing new genetic material via transfection, transduction, or other techniques. After transfection or transduction, the transformed DNA can recombine with the cell's DNA by physically integrating into the cell's chromosome, or it can be temporarily retained as an extrachromosomal element that is not replicated, or it can replicate independently as a plasmid. When the transformed DNA replicates with cell division, the cell is considered to have been "stablely transformed."

The term "amino acid" includes its general meaning in this field. The present disclosure uses conventional single-letter and three-letter amino acid codes, as shown in Table 1.

**Table 1**

| Amino acid | Three-letter code | Single-letter code |
|---|---|---|
| Alanine | ala | A |
| Arginine | arg | R |
| Asparagine | asn | N |
| Aspartic acid | asp | D |
| Cysteine | cys | C |
| Glutamic acid | glu | E |
| Glutamine | gln | Q |
| Glycine | gly | G |
| Histidine | his | H |
| Isoleucine | ile | I |
| Leucine | leu | L |
| Lysine | lys | K |
| Methionine | met | M |
| Phenylalanine | phe | F |
| Proline | pro | P |
| Serine | ser | S |
| Threonine | thr | T |
| Tryptophan | trp | W |
| Tyrosine | tyr | Y |
| Valine | val | V |

Stereoisomers of 20 conventional amino acids (e.g., D-amino acids), non-natural amino acids such as α-,α-disubstituted amino acids, N-alkyl amino acids, lactic acid, and other unconventional amino acids may also be suitable components of the antibodies of the present invention. Examples of unconventional amino acids include: 4-hydroxyproline, γ-carboxyglutamic acid, ε-N,N,N-trimethyllysine, ε-N-acetyllysine, O-phosphoserine, N-acetylserine, N-formylmethionine, 3-methylhistidine, 5-hydroxylysine, σ-N-methylarginine, and other similar amino acids and imino acids (e.g., 4-hydroxyproline). In the polypeptide notation used herein, the left-hand direction represents the amino-terminal direction, and the right-hand direction represents the carboxyl-terminal direction, consistent with standard usage and convention.

The "variant" of a protein (such as an antibody) comprises an amino acid sequence in which one or more amino acid residues are inserted, deleted, and/or substituted relative to the sequence of another protein. The variants include fusion proteins.

The term "identity" refers to the relationship between two or more polypeptide molecules or two or more nucleic acid molecules determined by aligning and comparing sequences. "Identity percentage" refers to the percentage of identical residues among amino acids or nucleotides within the compared molecules, calculated based on the smallest molecule being compared. For these calculations, gaps in the alignment (if present) are preferably resolved using a specific mathematical model or computer program ( i.e., an "algorithm"). Methods that can be used to calculate the identity of aligned nucleic acids or polypeptides include those described in Computational Molecular Biology, (edited by Lesk, A.M.), 1988, New York: Oxford University Press; Biocomputing Informatics and Genome Projects, (edited by Smith, D.W.), 1993, New York: Academic Press; Computer Analysis of Sequence Data, Part I, (edited by Griffin, A.M. and Griffin, H.G.), 1994, New Jersey: Human Press; von Heinje, G., 1987, Sequence Analysis in Molecular Biology, New York: Academic Press; Sequence Analysis Primer, (edited by Gribskov, M. and Devereux, J.), 1991, New York: M. Stockton Press; and Carillo et al., 1988, SIAM J. Applied Math. 48:1073*.*

When calculating the identity percentage, sequences to be compared are typically aligned in a way that yields the best match between them. An example of a computer program that can be used to determine the percentage of identity is the GCG package, which includes GAP (Devereux et al., 1984, Nucl. Acid Res. 12:387; Genetics Computer Group, University of Wisconsin, Madison, WI). The computer algorithm GAP is used to align two polypeptides or polynucleotides whose percentage of identity is to be determined. Sequences are aligned based on the best match of their respective amino acids or nucleotides (the "match span" is determined by the algorithm). This method is combined with a gap opening penalty (calculated as 3 x mean diagonal, where the "mean diagonal" is the average of the diagonals of the comparison matrix used; the "diagonal" is a score or numerical value assigned to each perfect amino acid match by a specific comparison matrix) and a gap extension penalty (which is typically 1/10 of the gap opening penalty) and a comparison matrix such as PAM 250 or BLOSUM 62. In some embodiments, the algorithm also uses a standard comparison matrix (for the PAM 250 comparison matrix, see Dayhoff et al., 1978, Atlas of Protein Sequence and Structure, 5:345-352; for the BLOSUM 62 comparison matrix, see Henikoff et al., 1992, Proc. Natl. Acad. Sci. USA. 89:10915-10919).

Examples of parameters that can be used in determining the percentage of identity of polypeptide or nucleotide sequences using the GAP procedure are as follows: Algorithm: Needleman et al., 1970, J. Mol. Biol. 48:443-453; Comparison matrix: from Henikoff et al., 1992, BLOSUM 62 above; Gap penalty: 12 (but no penalty for terminal gap); Gap length penalty: 4; Similarity threshold: 0.

Some alignment designs used to align two amino acid sequences can result in matching only short regions of the two sequences. Even if there is no significant relationship between the two full-length sequences, such small alignment regions can have extremely high sequence identity. Therefore, if it is necessary to generate alignments spanning at least 50 or other numbers of adjacent amino acids of the target polypeptide, the chosen alignment method (GAP procedure) can be adjusted.

The term "derivative" refers to a molecule that includes chemical modifications other than the insertion, deletion, or substitution of amino acids (or nucleic acids). In some embodiments, the derivative includes covalent modifications, including but not limited to chemical bonding with polymers, lipids, or other organic or inorganic parts. In some embodiments, the chemically modified antigen-binding protein may have a longer cycling half-life than the unmodified antigen-binding protein. In some embodiments, the chemically modified antigen-binding protein may improve the ability to target desired cells, tissues, and/or organs. In some embodiments, the derived antigen-binding protein is covalently modified to include one or more water-soluble polymeric linkers, including but not limited to polyethylene glycol, polyoxyethylene glycol, or polypropylene glycol. In some embodiments, the derived antigen-binding protein includes one or more polymers, including but not limited to monomethoxy-polyethylene glycol, dextran, cellulose or other sugar-based polymers, poly-(N-vinylpyrrolidone)-polyethylene glycol, propylene glycol homopolymers, propylene oxide/ethylene oxide copolymers, polyoxyethylated polyols (e.g., ethylene glycol), and polyvinyl alcohol, as well as mixtures of said polymers.

In some embodiments, the derivative is covalently modified with a polyethylene glycol (PEG) subunit. In some embodiments, one or more water-soluble polymers are bonded at one or more specific sites (e.g., at the amino terminus of the derivative). In some embodiments, one or more water-soluble polymers are randomly linked to one or more side chains of the derivative. In some embodiments, PEG is used to improve the therapeutic capacity of an antibody or antigen-binding fragment thereof. In some embodiments, PEG is used to improve the therapeutic capacity of a humanized antibody. Some of the methods described are discussed, for example, in U.S. Patent No. 6,133,426, which is incorporated herein by reference for any purpose.

The term "antibody" refers to an intact immunoglobulin of any isotype or a fragment thereof that can compete with an intact antibody for specific binding to a target antigen, and includes, for example, chimeric antibodies, humanized antibodies, human antibodies, and bispecific antibodies. An "antibody" is a class of antigen-binding proteins. An intact antibody will typically contain at least two full-length heavy chains and two full-length light chains, but in some cases may include fewer chains; for example, naturally occurring antibodies in camels may contain only heavy chains. Antibodies may be derived from a single source or may be "chimeric", meaning that different parts of the antibody may be derived from two different antibodies. Antigen-binding proteins, antibodies, or binding fragments can be generated in hybridomas; by recombinant DNA technology; or by enzymatic or chemical cleavage of intact antibodies. Unless otherwise stated, the term "antibody" includes not only antibodies containing two full-length heavy chains and two full-length light chains, but also their derivatives, variants, and fragments. In addition, unless explicitly excluded, antibodies include monoclonal antibodies, polyclonal antibodies, recombinant antibodies, bispecific antibodies, domain antibodies, synthetic antibodies (sometimes herein referred to as "antibody mimics"), chimeric antibodies, humanized antibodies, human antibodies, antibody fusions (sometimes herein referred to as "antibody conjugates"), and fragments thereof.

Naturally occurring antibody structural units typically comprise tetramers. Each tetramer typically consists of two identical pairs of polypeptide chains, each pair having a full-length "light chain" (approximately 25 kDa in some embodiments) and a full-length "heavy chain" (approximately 50-70 kDa in some embodiments). The amino-terminal portion of each chain typically contains a variable region of approximately 100-110 or more amino acids, which is typically responsible for antigen recognition. The carboxyl-terminal portion of each chain is typically defined as a constant region responsible for effector function. Human light chains are typically classified into κ and λ light chains. Heavy chains are typically classified into µ, δ, γ, α, or ε chains, and antibody isotypes are defined as IgM, IgD, IgG, IgA, and IgE, respectively. IgG has several subclasses, including but not limited to IgG1, IgG2, IgG3, and IgG4. IgM has subclasses, including but not limited to IgM1 and IgM2. IgA is similarly subdivided into subclasses, including but not limited to IgA1 and IgA2. Within both the full-length light and heavy chains, the variable and constant regions are typically linked by "J" regions of about 12 or more amino acids, and the heavy chain also includes "D" regions of about 10 or more amino acids. See, for example, Fundamental Immunology, Ch. 7 (edited by Paul, W., 2nd ed., Raven Press, NY (1989)) (which is incorporated herein by reference in its entirety for all purposes). The variable region of each light/heavy chain pair typically forms an antigen-binding site.

In some embodiments, even among antibodies of the same species, different antibody variable regions exhibit extensive variations in their amino acid sequences. Antibody variable regions typically determine the specificity of a particular antibody against its target.

The variable regions typically exhibit the same general structure as relatively conserved framework regions (FRs) connected by three hypervariable regions, also known as complementarity-determining regions or CDRs. CDRs from each pair of chains are typically located via the framework regions, allowing for the binding of specific epitopes. The variable regions of the two light and heavy chains typically contain domains FR1, CDR1, FR2, CDR2, FR3, CDR3, and FR4 from the N-terminus to the C-terminus. The amino acid assignment to each domain generally conforms to the following definitions: Kabat Sequences of Proteins of Immunological Interest (National Institutes of Health, Bethesda, Md. (1987 and 1991)) or Chothia & Lesk, J. Mol. Biol., 196:901-917 (1987); Chothia et al., Nature, 342:878-883 (1989). The International Immunogenetics (IMGT) Database (http://www.imgt.org) provides standardized numbers and definitions for antigen-binding sites. The correspondence between CDRs and IMGT classifications is described in Lefranc et al., Dev Comparat Immunol 27:55-77, 2003. Unless otherwise specified, CDR sequences referred to in the disclosure are based on IMGT numbers and definitions.

By convention, the CDR regions in the heavy chain are usually referred to as H1, H2, and H3, and are numbered sequentially from the amino terminus to the carboxyl terminus. The CDR regions in the light chain are usually referred to as L1, L2, and L3, and are numbered sequentially from the amino terminus to the carboxyl terminus.

The term "light chain" includes the full-length light chain and fragments thereof containing variable region sequences sufficient to confer binding specificity. The full-length light chain contains a variable region domain V_{L} and a constant region domain C_{L}. The variable region domain of the light chain is located at the amino terminus of the polypeptide. Light chains include κ chains and λ chains. The term "heavy chain" includes the full-length heavy chain and fragments thereof containing variable region sequences sufficient to confer binding specificity. The full-length heavy chain contains a variable region domain V_{H} and three constant region domains C_{H}1, C_{H}2, and C_{H}3. The V_{H} domain is located at the amino terminus of the polypeptide, while the C_{H} domains are located at the carboxyl terminus, with C_{H}3 being closest to the carboxyl terminus. The heavy chain can be any isotype, including IgG (including IgG1, IgG2, IgG3, and IgG4 subclasses), IgA (including IgA1 and IgA2 subclasses), IgM, and IgE.

The term "antigen-binding fragment" includes, but is not limited to, Fab fragments, Fab' fragments, F(ab')₂ fragments, Fv fragments, Diabodies, and single-chain antibody molecules.

The "Fc" region contains two heavy chain fragments with C_{H}1 and C_{H}2 domains. The two heavy chain fragments are held together by two or more disulfide bonds and by the hydrophobic interaction of the C_{H}3 domain.

The "Fab fragment" comprises one light chain and the C_{H}1 and the variable region of one heavy chain. The heavy chain of the Fab molecule cannot form a disulfide bond with another heavy chain molecule.

The "Fab' fragment" comprises one light chain and a portion of one heavy chain containing the VH domain, the C_{H}1 domain, and the region between the C_{H}1 and C_{H}2 domains. Therefore, interchain disulfide bonds can be formed between the two heavy chains of the two Fab' fragments to form the F(ab')₂ molecule.

The "F(ab')₂ fragment" comprises two light chains and a portion of two heavy chains containing the constant region between the C_{H}1 and C_{H}2 domains, thereby forming interchain disulfide bonds between the two heavy chains. Therefore, the F(ab')₂ fragment consists of two Fab' fragments held together by disulfide bonds between the two heavy chains.

The "Fv fragment" comprieses variable regions from both the heavy and light chains, but lacks constant regions.

The "single-chain antibody (scfv)" is an Fv molecule in which the variable regions of the heavy and light chains are linked together by a flexible linker to form a single polypeptide chain, thereby forming an antigen-binding region. Detailed descriptions of single-chain antibodies can be found in International Patent Application Publication No. WO 88/01649 and U.S. Patent Nos. 4,946,778 and 5,260,203, the contents of which are incorporated herein by reference.

The "monoclonal antibody" refers to a group of homogeneous antibodies composed of a single molecule. Monoclonal antibodies can be non-specific or multi-specific.

The "domain antibodies" are immunoglobulin fragments containing only heavy chain variable regions or light chain variable regions that have immunological functions. In some cases, two or more V_{H} regions are covalently linked to a peptide linker to form bivalent domain antibodies. The two V_{H} regions of the bivalent domain antibody can target the same or different antigens.

The "multispecific antibody" is an antibody that targets more than one antigen or epitope. A "bispecific", "dual-specific" or "bifunctional" antibody is a hybrid antibody with two distinct antigen-binding sites. The two binding sites of a bispecific antibody will bind to two different epitopes, which may be located on the same or different protein targets. Bispecific antibodies may have cross-reactivity with other related antigens, for example, cross-reactivity with the same antigen from other species (homologous) (such as humans or monkeys, such as cynomolgus (cyno) or chimpanzees), or they may bind to epitopes shared between two or more different antigens. Bispecific antibodies can be monovalent or bivalent in relation to their binding to different antigens or epitopes.

The "valence" refers to the specified number of binding sites in a molecule that are specific to the antigen. Therefore, the terms "monovalent," "divalent," "tetravalent," and "hexavalent" refer to the presence of one, two, four, and six binding sites in a molecule that are specific to the antigen, respectively. "Multivalent" refers to the presence of two or more binding sites in a molecule that are specific to the antigen.

An antibody is considered a "selective" antibody when it binds to one target more tightly than it binds to a second target.

The "recombinant antibody" is an antibody prepared using recombinant technology. The methods and techniques used to prepare recombinant antibodies are well known in the art.

Bispecific or bifunctional antibodies are typically artificial hybrid antibodies with two distinct heavy/light chain pairs and two distinct binding sites. Bispecific antibodies can be prepared using various methods, including but not limited to fusing hybridomas or linking Fab fragments. See, for example, Songsivilai et al., Clin. Exp. Immunol., 79: 315-321 (1990); Kostelny et al., J. Immunol., 148:1547-1553 (1992).

Each individual immunoglobulin chain typically consists of several "immunoglobulin domains," each composed of approximately 90-110 amino acids and exhibiting a unique folding pattern. These domains are the basic building blocks of antibody polypeptides . In humans, the IgA and IgD isotypes contain four heavy chains and four light chains; the IgG and IgE isotypes contain two heavy chains and two light chains; and the IgM isotype contains five heavy chains and five light chains. The C-region of the heavy chain typically contains one or more domains responsible for effector functions. The number of constant region domains in the heavy chain depends on the isotype. For example, the IgG heavy chain contains three C-region domains called C_{H}1, C_{H}2, and C_{H}3. The antibody provided can be of any of these isotypes and subclasses.

The term "neutralization" refers to the binding of a ligand and the prevention or reduction of its biological activity. This can be achieved, for example, by directly blocking the binding site on the ligand or by binding the ligand and altering its binding ability indirectly (e.g., by structural or energy changes in the ligand). In assessing the binding and/or specificity of an antibody or antigen-binding fragment thereof, the antibody or fragment can substantially inhibit the binding of a ligand to its binding partner when an excess of the antibody reduces the amount of the binding partner bound to the ligand by at least about 1-20%, 20-30%, 30-40%, 40-50%, 50-60%, 60-70%, 70-80%, 80-85%, 85-90%, 90-95%, 95-97%, 97-98%, 98-99% or more (as measured by an in vitro competitive binding assay). In some embodiments, the neutralizing ability is characterized and/or described via a competitive assay. In some embodiments, the neutralizing ability is described as a 50% inhibition concentration (IC₅₀) value.

The term "target" refers to a molecule or molecular portion that can be bound by an antibody or antigen-binding fragment thereof. In some embodiments, the target may have one or more epitopes. In some embodiments, the target is an antigen. The use of "antigen" in the phrase "antigen-binding fragment" simply indicates a protein sequence containing an antigen that can be bound by an antibody. In this case, the protein is not necessarily exogenous, or not necessarily capable of inducing an immune response.

When the term "compete" is used in neutralizing antibodies competing for the same epitope, it refers to competition between antibodies, which is determined by an assay in which the antibody to be detected or antigen-binding fragment thereof prevents or inhibits (e.g., reduces) the specific binding of a reference antibody to a common antigen (e.g., the S protein or its domain). Numerous types of competitive binding assays can be used to determine whether one antibody is competing with another; these assays include, for example, solid-phase direct or indirect radioimmunoassays (RIA), solid-phase direct or indirect enzyme immunoassays (EIA), and sandwich competitive assays (see, for example, Stahli et al., 1983, Methods in Enzymology. 9:242-253); solid-phase direct biotin-avidin EIA (see, for example, Kirkland et al., 1986, J. Immunol. 137:3614-3619), solid-phase direct labeling assays, solid-phase direct labeling sandwich assays (see, for example, Harlow and Lane, 1988, Antibodies, A Laboratory Manual, Cold Spring Harbor Press); solid-phase direct labeling RIAs with I-125 labels (see, for example, Morel et al., 1988, Molec. Immunol. 25:7-15); solid-phase direct biotin-avidin EIA (see, for example, Cheung et al., 1990, Virology 176:546-552); and directly labeled RIA (Moldenhauer et al., 1990, Scand. J. Immunol. 32:77-82). The assay typically involves using a purified antigen on a solid surface or cell bound to either an unlabeled detection antibody or a labeled reference antibody. Competitive inhibition is measured by measuring the amount of labels bound to the solid surface or cell in the presence of the detection antibody. Typically, an excess of the detection antibody is present. When an excess of the competing antibody is present, it will typically inhibit (e.g., reduce) at least 40-45%, 45-50%, 50-55%, 55-60%, 60-65%, 65-70%, 70-75%, or 75% or more of the specific binding of the reference antibody to the common antigen. In some cases, the binding is inhibited by at least 80-85%, 85-90%, 90-95%, 95-97%, or 97% or more.

The term "antigen" refers to a molecule or molecular portion that can be bound by a selective binding agent such as an antibody or an immunologically functional fragment thereof. In some embodiments, the antigen can be used in an animal to generate an antibody capable of binding to the antigen. An antigen may have one or more epitopes capable of interacting with different antibodies.

The term "epitope" includes any determinant capable of being bound by an antibody. An epitope is an antigenic region that is bound by an antibody targeting that antigen. When the antigen is a protein, it contains specific amino acids that come into direct contact with the antibody. In most cases, epitopes are located on proteins, but in some cases, they can be located on other types of molecules, such as nucleic acids. Epitope determinants can include chemically active surface clusters of a molecule, such as amino acids, sugar side chains, phosphoryl groups, or sulfonyl groups, and may have specific three-dimensional structural features and/or specific charge features. Epitopes can consist of continuous and/or discontinuous amino acids forming conformational spatial units. For discontinuous epitopes, amino acids from different parts of the linear sequence of the antigen are close together in three-dimensional space due to the folding of the protein molecule. Antibody "epitopes" depend on the method used to identify them. Unless otherwise specified, the present application uses a competitive ELISA in combination with alanine scanning to identify antibody epitopes.

The term "therapeutically effective amount" refers to the amount of the antibody or antigen-binding fragment of the present invention that produces a therapeutic response in mammals. The therapeutically effective amount is readily determined by those skilled in the art.

The terms "patient" and "subject" are used interchangeably and include human and non-human animal subjects, as well as subjects with a formally diagnosed disease, subjects with an undetermined disease, subjects receiving medical treatment, and subjects at risk of developing a disease.

The term "treatment" includes therapeutic treatment, preventative treatment, and applications that reduce the risk of a subject developing a disease or other risk factors. Treatment does not require a complete cure for the disease, but rather includes embodiments where symptoms or underlying risk factors are reduced.

The term "prevention" does not require the likelihood for eliminating an event is 100%. More accurately, it means that the likelihood of an event occurring is minimized in the presence of the antibody or the method.

Standard techniques can be used for recombinant DNA, oligonucleotide synthesis, and tissue culture and transformation (e.g., electroporation, lipid transfection). Enzymatic reactions and purification techniques can be performed according to the manufacturer's instructions, or according to methods generally accepted in the art, or as described herein. The aforementioned techniques and methods are generally performed according to conventional methods well known in the art, and according to the various general and more specific references cited and discussed throughout this specification. See, for example, Sambrook et al., Molecular Cloning: A Laboratory Manual (2nd edition, Cold Spring Harbor Laboratory Press, Cold Spring Harbor, NY (1989)), which is incorporated herein by reference for any purpose. Unless explicitly defined, the terminology, experimental methods, and techniques used in relation to analytical chemistry, synthetic organic chemistry, and medicine and pharmaceutical chemistry described herein are well known in the art and are commonly used in the art. Standard techniques can be used for chemical synthesis, chemical analysis, drug preparation, formulation and delivery, and patient treatment.

### II. The antibody of the present invention and the preparation method thereof

This disclosure provides antibodies or antigen-binding fragments thereof capable of effectively binding to HBV and neutralizing its activity (hereinafter collectively referred to as "antibodies"). The inventors have also discovered that antibodies cloned from recovered hepatitis B patients show better efficacy in recognizing different hepatitis B surface antigens compared to hepatitis B vaccine recipients, and are more advantageous in neutralizing hepatitis B virus mutants and reducing hepatitis B mutant antigens.

In some embodiments, the antibodies described herein neutralize HBV and its mutant strains in vitro at a 50% inhibitory concentration (IC₅₀) of less than 0.5 µg/ml. In some embodiments, the antibodies described herein neutralize HBV and its mutant strains in vitro at a 50% inhibitory concentration (IC₅₀) of less than 0.25 µg/ml. In some embodiments, the antibodies described herein neutralize HBV and its mutant strains in vitro at a 50% inhibitory concentration (IC₅₀) of less than 0.1 µg/ml. The IC₅₀ value of the antibodies described herein is measured as described in cell-based in vitro neutralization assays in the Examples.

The present invention relates to isolated antibodies against HBV and its mutant strains, or antigen-binding fragments thereof. In one embodiment, the invention relates to isolated antibodies that bind to the HBsAg protein, or antigen-binding fragments thereof. In another embodiment, the invention relates to isolated antibodies that bind to epitopes in the extracellular domain of HBsAg, or antigen-binding fragments thereof. In yet another embodiment, the invention relates to isolated antibodies that bind to epitopes in amino acid residues 99-169 of HBsAg, or antigen-binding fragments thereof. In yet another embodiment, the invention relates to isolated antibodies that bind to epitopes in amino acid residues 124-147 of HBsAg, or antigen-binding fragments thereof.

In one embodiment , the HBsAg protein comprises a single or dual-site mutation. In one embodiment, the mutation is selected from Q101R, Q101K, M103I, P105L, L109I, L109R, I110L, S113N, T114R, T115N, T116N, T118K, P120S, P120T, P120K-T123D, K122I, K122R-G145R, T123N, T123N-T143S, C124R, T125M, T126S, A128V, Q129H, G130N, G130R, M133T, F134L, F134V-D144G, C137Y, C137Y-D144V, C138Y, K141E, P142S, P142L-G145R, T143I, T143L, D144E, D144EG145R, G145E, G145R, N146S, C149Y, and Y161F, and any combination of two or more of them. In one embodiment, the mutation is G145R. In one embodiment, the mutation is K122R-G145R. In one embodiment, the mutation is F134L.

In one aspect, the present invention relates to an isolated antibody or antigen-binding fragment thereof that specifically binds to HBsAg, characterized in that said isolated antibody or antigen-binding fragment thereof comprises a heavy chain variable region VH and a light chain variable region VL, said heavy chain variable region VH comprising complementarity-determining regions CDRH1, CDRH2, and CDRH3, said light chain variable region VL comprising complementarity-determining regions CDRL1, CDRL2, and CDRL3, wherein CDRH1, CDRH2, CDRH3, CDRL1, CDRL2, and CDRL3 are selected from the following amino acid sequences:
a) CDRH1 of SEQ ID NO:2, CDRH2 of SEQ ID NO:3, CDRH3 of SEQ ID NO:4, CDRL1 of SEQ ID NO:6, CDRL2 of SEQ ID NO:7 and CDRL3 of SEQ ID NO:8;
b) CDRH1 of SEQ ID NO:10, CDRH2 of SEQ ID NO:11, CDRH3 of SEQ ID NO:12, CDRL1 of SEQ ID NO:14, CDRL2 of SEQ ID NO:15 and CDRL3 of SEQ ID NO:16;
c) CDRH1 of SEQ ID NO:18, CDRH2 of SEQ ID NO:19, CDRH3 of SEQ ID NO:20, CDRL1 of SEQ ID NO:22, CDRL2 of SEQ ID NO:23 and CDRL3 of SEQ ID NO:24;
d) CDRH1 of SEQ ID NO:26, CDRH2 of SEQ ID NO:27, CDRH3 of SEQ ID NO:28, CDRL1 of SEQ ID NO:30, CDRL2 of SEQ ID NO:31 and CDRL3 of SEQ ID NO:32;
e) CDRH1 of SEQ ID NO:34, CDRH2 of SEQ ID NO:35, CDRH3 of SEQ ID NO:36, CDRL1 of SEQ ID NO:38, CDRL2 of SEQ ID NO:39 and CDRL3 of SEQ ID NO:40;
f) CDRH1 of SEQ ID NO:42, CDRH2 of SEQ ID NO:43, CDRH3 of SEQ ID NO:44, CDRL1 of SEQ ID NO:46, CDRL2 of SEQ ID NO:47 and CDRL3 of SEQ ID NO:48;
g) CDRH1 of SEQ ID NO:50, CDRH2 of SEQ ID NO:51, CDRH3 of SEQ ID NO:52, CDRL1 of SEQ ID NO:54, CDRL2 of SEQ ID NO:55 and CDRL3 of SEQ ID NO:56;
h) CDRH1 of SEQ ID NO:58, CDRH2 of SEQ ID NO:59, CDRH3 of SEQ ID NO:60, CDRL1 of SEQ ID NO:62, CDRL2 of SEQ ID NO:63 and CDRL3 of SEQ ID NO:64;
i) CDRH1 of SEQ ID NO:66, CDRH2 of SEQ ID NO:67, CDRH3 of SEQ ID NO:68, CDRL1 of SEQ ID NO:70, CDRL2 of SEQ ID NO:71 and CDRL3 of SEQ ID NO:72;
j) CDRH1 of SEQ ID NO:74, CDRH2 of SEQ ID NO:75, CDRH3 of SEQ ID NO:76, CDRL1 of SEQ ID NO:78, CDRL2 of SEQ ID NO:79 and CDRL3 of SEQ ID NO:80;
k) CDRH1 of SEQ ID NO:82, CDRH2 of SEQ ID NO:83, CDRH3 of SEQ ID NO:84, CDRL1 of SEQ ID NO:86, CDRL2 of SEQ ID NO:87 and CDRL3 of SEQ ID NO:88;
1) CDRH1 of SEQ ID NO:90, CDRH2 of SEQ ID NO:91, CDRH3 of SEQ ID NO:92, CDRL1 of SEQ ID NO:94, CDRL2 of SEQ ID NO:95 and CDRL3 of SEQ ID NO:96;
m) CDRH1 of SEQ ID NO:98, CDRH2 of SEQ ID NO:99, CDRH3 of SEQ ID NO:100, CDRL1 of SEQ ID NO:102, CDRL2 of SEQ ID NO:103 and CDRL3 of SEQ ID NO:104;
n) CDRH1 of SEQ ID NO:106, CDRH2 of SEQ ID NO:107, CDRH3 of SEQ ID NO:108, CDRL1 of SEQ ID NO:110, CDRL2 of SEQ ID NO:111 and CDRL3 of SEQ ID NO:112;
o) CDRH1 of SEQ ID NO:114, CDRH2 of SEQ ID NO:115, CDRH3 of SEQ ID NO:116, CDRL1 of SEQ ID NO:118, CDRL2 of SEQ ID NO:119 and CDRL3 of SEQ ID NO:120;
p) CDRH1 of SEQ ID NO:122, CDRH2 of SEQ ID NO:123, CDRH3 of SEQ ID NO:124, CDRL1 of SEQ ID NO:126, CDRL2 of SEQ ID NO:127 and CDRL3 of SEQ ID NO:128;
q) CDRH1 of SEQ ID NO:130, CDRH2 of SEQ ID NO:131, CDRH3 of SEQ ID NO:132, CDRL1 of SEQ ID NO:134, CDRL2 of SEQ ID NO:135 and CDRL3 of SEQ ID NO:136;
r) CDRH1 of SEQ ID NO:138, CDRH2 of SEQ ID NO:139, CDRH3 of SEQ ID NO:140, CDRL1 of SEQ ID NO:142, CDRL2 of SEQ ID NO:143 and CDRL3 of SEQ ID NO:144;
s) CDRH1 of SEQ ID NO:146, CDRH2 of SEQ ID NO:147, CDRH3 of SEQ ID NO:148, CDRL1 of SEQ ID NO:150, CDRL2 of SEQ ID NO:151 and CDRL3 of SEQ ID NO:152;
t) CDRH1 of SEQ ID NO:154, CDRH2 of SEQ ID NO:155, CDRH3 of SEQ ID NO:156, CDRL1 of SEQ ID NO:158, CDRL2 of SEQ ID NO:159 and CDRL3 of SEQ ID NO:160;
u) CDRH1 of SEQ ID NO:162, CDRH2 of SEQ ID NO:163, CDRH3 of SEQ ID NO:164, CDRL1 of SEQ ID NO:166, CDRL2 of SEQ ID NO:167 and CDRL3 of SEQ ID NO:168;
v) CDRH1 of SEQ ID NO:170, CDRH2 of SEQ ID NO:171, CDRH3 of SEQ ID NO:172, CDRL1 of SEQ ID NO: 174, CDRL2 of SEQ ID NO:175 and CDRL3 of SEQ ID NO: 176;
w) CDRH1 of SEQ ID NO:178, CDRH2 of SEQ ID NO:179, CDRH3 of SEQ ID NO:180, CDRL1 of SEQ ID NO:182, CDRL2 of SEQ ID NO:183 and CDRL3 of SEQ ID NO:184;
x) CDRH1 of SEQ ID NO:186, CDRH2 of SEQ ID NO:187, CDRH3 of SEQ ID NO:188, CDRL1 of SEQ ID NO:190, CDRL2 of SEQ ID NO:191 and CDRL3 of SEQ ID NO:192;
y) CDRH1 of SEQ ID NO:194, CDRH2 of SEQ ID NO:195, CDRH3 of SEQ ID NO:196, CDRL1 of SEQ ID NO:198, CDRL2 of SEQ ID NO:199 and CDRL3 of SEQ ID NO:200;
z) CDRH1 of SEQ ID NO:202, CDRH2 of SEQ ID NO:203, CDRH3 of SEQ ID NO:204, CDRL1 of SEQ ID NO:206, CDRL2 of SEQ ID NO:207 and CDRL3 of SEQ ID NO:208;
aa) CDRH1 of SEQ ID NO:210, CDRH2 of SEQ ID NO:211, CDRH3 of SEQ ID NO:212, CDRL1 of SEQ ID NO:214, CDRL2 of SEQ ID NO:215 and CDRL3 of SEQ ID NO:216;
bb) CDRH1 of SEQ ID NO:218, CDRH2 of SEQ ID NO:219, CDRH3 of SEQ ID NO:220, CDRL1 of SEQ ID NO:222, CDRL2 of SEQ ID NO:223 and CDRL3 of SEQ ID NO:224;
cc) CDRH1 of SEQ ID NO:226, CDRH2 of SEQ ID NO:227, CDRH3 of SEQ ID NO:228, CDRL1 of SEQ ID NO:230, CDRL2 of SEQ ID NO:231 and CDRL3 of SEQ ID NO:232;
dd) CDRH1 of SEQ ID NO:234, CDRH2 of SEQ ID NO:235, CDRH3 of SEQ ID NO:236, CDRL1 of SEQ ID NO:238, CDRL2 of SEQ ID NO:239 and CDRL3 of SEQ ID NO:240;
ee) CDRH1 of SEQ ID NO:242, CDRH2 of SEQ ID NO:243, CDRH3 of SEQ ID NO:244, CDRL1 of SEQ ID NO:246, CDRL2 of SEQ ID NO:247 and CDRL3 of SEQ ID NO:248;
ff) CDRH1 of SEQ ID NO:250, CDRH2 of SEQ ID NO:251, CDRH3 of SEQ ID NO:252, CDRL1 of SEQ ID NO:254, CDRL2 of SEQ ID NO:255 and CDRL3 of SEQ ID NO:256;
gg) CDRH1 of SEQ ID NO:258, CDRH2 of SEQ ID NO:259, CDRH3 of SEQ ID NO:260, CDRL1 of SEQ ID NO:262, CDRL2 of SEQ ID NO:263 and CDRL3 of SEQ ID NO:264;
hh) CDRH1 of SEQ ID NO:266, CDRH2 of SEQ ID NO:267, CDRH3 of SEQ ID NO:268, CDRL1 of SEQ ID NO:270, CDRL2 of SEQ ID NO:271 and CDRL3 of SEQ ID NO:272;
ii) CDRH1 of SEQ ID NO:274, CDRH2 of SEQ ID NO:275, CDRH3 of SEQ ID NO:276, CDRL1 of SEQ ID NO:278, CDRL2 of SEQ ID NO:279 and CDRL3 of SEQ ID NO:280;
jj) CDRH1 of SEQ ID NO:282, CDRH2 of SEQ ID NO:283, CDRH3 of SEQ ID NO:284, CDRL1 of SEQ ID NO:286, CDRL2 of SEQ ID NO:287 and CDRL3 of SEQ ID NO:288;
kk) CDRH1 of SEQ ID NO:290, CDRH2 of SEQ ID NO:291, CDRH3 of SEQ ID NO:292, CDRL1 of SEQ ID NO:294, CDRL2 of SEQ ID NO:295 and CDRL3 of SEQ ID NO:296;
11) CDRH1 of SEQ ID NO:298, CDRH2 of SEQ ID NO:299, CDRH3 of SEQ ID NO:300, CDRL1 of SEQ ID NO:302, CDRL2 of SEQ ID NO:303 and CDRL3 of SEQ ID NO:304;
mm) CDRH1 of SEQ ID NO:306, CDRH2 of SEQ ID NO:307, CDRH3 of SEQ ID NO:308, CDRL1 of SEQ ID NO:310, CDRL2 of SEQ ID NO:311 and CDRL3 of SEQ ID NO:312;
nn) CDRH1 of SEQ ID NO:314, CDRH2 of SEQ ID NO:315, CDRH3 of SEQ ID NO:316, CDRL1 of SEQ ID NO:318, CDRL2 of SEQ ID NO:319 and CDRL3 of SEQ ID NO:320;
oo) CDRH1 of SEQ ID NO:322, CDRH2 of SEQ ID NO:323, CDRH3 of SEQ ID NO:324, CDRL1 of SEQ ID NO:326, CDRL2 of SEQ ID NO:327 and CDRL3 of SEQ ID NO:328;
pp) CDRH1 of SEQ ID NO:330, CDRH2 of SEQ ID NO:331, CDRH3 of SEQ ID NO:332, CDRL1 of SEQ ID NO:334, CDRL2 of SEQ ID NO:335 and CDRL3 of SEQ ID NO:336;
qq) CDRH1 of SEQ ID NO:338, CDRH2 of SEQ ID NO:339, CDRH3 of SEQ ID NO:340, CDRL1 of SEQ ID NO:342, CDRL2 of SEQ ID NO:343 and CDRL3 of SEQ ID NO:344;
rr) CDRH1 of SEQ ID NO:346, CDRH2 of SEQ ID NO:347, CDRH3 of SEQ ID NO:348, CDRL1 of SEQ ID NO:350, CDRL2 of SEQ ID NO:351 and CDRL3 of SEQ ID NO:352;
ss) CDRH1 of SEQ ID NO:354, CDRH2 of SEQ ID NO:355, CDRH3 of SEQ ID NO:356, CDRL1 of SEQ ID NO:358, CDRL2 of SEQ ID NO:359 and CDRL3 of SEQ ID NO:360;
tt) CDRH1 of SEQ ID NO:362, CDRH2 of SEQ ID NO:363, CDRH3 of SEQ ID NO:364, CDRL1 of SEQ ID NO:366, CDRL2 of SEQ ID NO:367 and CDRL3 of SEQ ID NO:368;
uu) CDRH1 of SEQ ID NO:370, CDRH2 of SEQ ID NO:371, CDRH3 of SEQ ID NO:372, CDRL1 of SEQ ID NO:374, CDRL2 of SEQ ID NO:375 and CDRL3 of SEQ ID NO:376;
vv) CDRH1 of SEQ ID NO:378, CDRH2 of SEQ ID NO:379, CDRH3 of SEQ ID NO:380, CDRL1 of SEQ ID NO:382, CDRL2 of SEQ ID NO:383 and CDRL3 of SEQ ID NO:384;
ww) CDRH1 of SEQ ID NO:386, CDRH2 of SEQ ID NO:387, CDRH3 of SEQ ID NO:388, CDRL1 of SEQ ID NO:390, CDRL2 of SEQ ID NO:391 and CDRL3 of SEQ ID NO:392;
xx) CDRH1 of SEQ ID NO:394, CDRH2 of SEQ ID NO:395, CDRH3 of SEQ ID NO:396, CDRL1 of SEQ ID NO:398, CDRL2 of SEQ ID NO:399 and CDRL3 of SEQ ID NO:400;
yy) CDRH1 of SEQ ID NO:402, CDRH2 of SEQ ID NO:403, CDRH3 of SEQ ID NO:404, CDRL1 of SEQ ID NO:406, CDRL2 of SEQ ID NO:407 and CDRL3 of SEQ ID NO:408;
zz) CDRH1 of SEQ ID NO:410, CDRH2 of SEQ ID NO:411, CDRH3 of SEQ ID NO:412, CDRL1 of SEQ ID NO:414, CDRL2 of SEQ ID NO:415 and CDRL3 of SEQ ID NO:416;
aaa) CDRH1 of SEQ ID NO:418, CDRH2 of SEQ ID NO:419, CDRH3 of SEQ ID NO:420, CDRL1 of SEQ ID NO:422, CDRL2 of SEQ ID NO:423 and CDRL3 of SEQ ID NO:424;
bbb) CDRH1 of SEQ ID NO:426, CDRH2 of SEQ ID NO:427, CDRH3 of SEQ ID NO:428, CDRL1 of SEQ ID NO:430, CDRL2 of SEQ ID NO:431 and CDRL3 of SEQ ID NO:432;
ccc) CDRH1 of SEQ ID NO:434, CDRH2 of SEQ ID NO:435, CDRH3 of SEQ ID NO:436, CDRL1 of SEQ ID NO:438, CDRL2 of SEQ ID NO:439 and CDRL3 of SEQ ID NO:440;
ddd) CDRH1 of SEQ ID NO:442, CDRH2 of SEQ ID NO:443, CDRH3 of SEQ ID NO:444, CDRL1 of SEQ ID NO:446, CDRL2 of SEQ ID NO:447 and CDRL3 of SEQ ID NO:448;
eee) CDRH1 of SEQ ID NO:450, CDRH2 of SEQ ID NO:451, CDRH3 of SEQ ID NO:452, CDRL1 of SEQ ID NO:454, CDRL2 of SEQ ID NO:455 and CDRL3 of SEQ ID NO:456;
fff) CDRH1 of SEQ ID NO:458, CDRH2 of SEQ ID NO:459, CDRH3 of SEQ ID NO:460, CDRL1 of SEQ ID NO:462, CDRL2 of SEQ ID NO:463 and CDRL3 of SEQ ID NO:464;
ggg) CDRH1 of SEQ ID NO:466, CDRH2 of SEQ ID NO:467, CDRH3 of SEQ ID NO:468, CDRL1 of SEQ ID NO:470, CDRL2 of SEQ ID NO:471 and CDRL3 of SEQ ID NO:472;
hhh) CDRH1 of SEQ ID NO:474, CDRH2 of SEQ ID NO:475, CDRH3 of SEQ ID NO:476, CDRL1 of SEQ ID NO:478, CDRL2 of SEQ ID NO:479 and CDRL3 of SEQ ID NO:480;
iii) CDRH1 of SEQ ID NO:482, CDRH2 of SEQ ID NO:483, CDRH3 of SEQ ID NO:484, CDRL1 of SEQ ID NO:486, CDRL2 of SEQ ID NO:487 and CDRL3 of SEQ ID NO:488; and
jjj) CDRH1 of SEQ ID NO:490, CDRH2 of SEQ ID NO:491, CDRH3 of SEQ ID NO:492, CDRL1 of SEQ ID NO:494, CDRL2 of SEQ ID NO:495 and CDRL3 of SEQ ID NO:496.

In some embodiments, the isolated antibody or antigen-binding fragment thereof comprises a heavy chain variable region VH and a light chain variable region VL, wherein the heavy chain variable region VH comprises complementarity-determining regions CDRH1, CDRH2, and CDRH3, and the light chain variable region VL comprises complementarity-determining regions CDRL1, CDRL2, and CDRL3, wherein CDRH1, CDRH2, CDRH3, CDRL1, CDRL2, and CDRL3 comprise the amino acid sequences as described above with a substitution, deletion, or insertion of no more than two amino acids at one or more sites.

In one aspect, the present invention relates to an isolated antibody or antigen-binding fragment thereof that specifically binds to HBsAg, characterized in that said isolated antibody or antigen-binding fragment thereof comprises a heavy chain variable region VH and a light chain variable region VL selected from the following amino acid sequence:
a) VH of SEQ ID NO:1 and VL of SEQ ID NO:5;
b) VH of SEQ ID NO:9 and VL of SEQ ID NO:13;
c) VH of SEQ ID NO:17 and VL of SEQ ID NO:21;
d) VH of SEQ ID NO:25 and VL of SEQ ID NO:29;
e) VH of SEQ ID NO:33 and VL of SEQ ID NO:37;
f) VH of SEQ ID NO:41 and VL of SEQ ID NO:45;
g) VH of SEQ ID NO:49 and VL of SEQ ID NO:53;
h) VH of SEQ ID NO:57 and VL of SEQ ID NO:61;
i) VH of SEQ ID NO:65 and VL of SEQ ID NO:69;
j) VH of SEQ ID NO:73 and VL of SEQ ID NO:77;
k) VH of SEQ ID NO:81 and VL of SEQ ID NO:85;
1) VH of SEQ ID NO:89 and VL of SEQ ID NO:93;
m) VH of SEQ ID NO:97 and VL of SEQ ID NO: 101;
n) VH of SEQ ID NO:105 and VL of SEQ ID NO:109;
o) VH of SEQ ID NO:113 and VL of SEQ ID NO:117;
p) VH of SEQ ID NO:121 and VL of SEQ ID NO:125;
q) VH of SEQ ID NO:129 and VL of SEQ ID NO:133;
r) VH of SEQ ID NO:137 and VL of SEQ ID NO:141;
s) VH of SEQ ID NO:145 and VL of SEQ ID NO:149;
t) VH of SEQ ID NO:153 and VL of SEQ ID NO:157;
u) VH of SEQ ID NO:161 and VL of SEQ ID NO:165;
v) VH of SEQ ID NO:169 and VL of SEQ ID NO:173;
w) VH of SEQ ID NO:177 and VL of SEQ ID NO:181;
x) VH of SEQ ID NO:185 and VL of SEQ ID NO:189;
y) VH of SEQ ID NO:193 and VL of SEQ ID NO:197;
z) VH of SEQ ID NO:201 and VL of SEQ ID NO:205;
aa) VH of SEQ ID NO:209 and VL of SEQ ID NO:213;
bb) VH of SEQ ID NO:217 and VL of SEQ ID NO:221;
cc) VH of SEQ ID NO:225 and VL of SEQ ID NO:229;
dd) VH of SEQ ID NO:233 and VL of SEQ ID NO:237;
ee) VH of SEQ ID NO:241 and VL of SEQ ID NO:245;
ff) VH of SEQ ID NO:249 and VL of SEQ ID NO:253;
gg) VH of SEQ ID NO:257 and VL of SEQ ID NO:261;
hh) VH of SEQ ID NO:265 and VL of SEQ ID NO:269;
ii) VH of SEQ ID NO:273 and VL of SEQ ID NO:277;
jj) VH of SEQ ID NO:281 and VL of SEQ ID NO:285;
kk) VH of SEQ ID NO:289 and VL of SEQ ID NO:293;
11) VH of SEQ ID NO:297 and VL of SEQ ID NO:301;
mm) VH of SEQ ID NO:305 and VL of SEQ ID NO:309;
nn) VH of SEQ ID NO:313 and VL of SEQ ID NO:317;
oo) VH of SEQ ID NO:321 and VL of SEQ ID NO:325;
pp) VH of SEQ ID NO:329 and VL of SEQ ID NO:333;
qq) VH of SEQ ID NO:337 and VL of SEQ ID NO:341;
rr) VH of SEQ ID NO:345 and VL of SEQ ID NO:349;
ss) VH of SEQ ID NO:353 and VL of SEQ ID NO:357;
tt) VH of SEQ ID NO:361 and VL of SEQ ID NO:365;
uu) VH of SEQ ID NO:369 and VL of SEQ ID NO:373;
vv) VH of SEQ ID NO:377 and VL of SEQ ID NO:381;
ww) VH of SEQ ID NO:385 and VL of SEQ ID NO:389;
xx) VH of SEQ ID NO:393 and VL of SEQ ID NO:397;
yy) VH of SEQ ID NO:401 and VL of SEQ ID NO:405;
zz) VH of SEQ ID NO:409 and VL of SEQ ID NO:413;
aaa) VH of SEQ ID NO:417 and VL of SEQ ID NO:421;
bbb) VH of SEQ ID NO:425 and VL of SEQ ID NO:429;
ccc) VH of SEQ ID NO:433 and VL of SEQ ID NO:437;
ddd) VH of SEQ ID NO:441 and VL of SEQ ID NO:445;
eee) VH of SEQ ID NO:449 and VL of SEQ ID NO:453;
fff) VH of SEQ ID NO:457 and VL of SEQ ID NO:461;
ggg) VH of SEQ ID NO:465 and VL of SEQ ID NO:469;
hhh) VH of SEQ ID NO:473 and VL of SEQ ID NO:477;
iii) VH of SEQ ID NO:481 and VL of SEQ ID NO:485; and
jjj) VH of SEQ ID NO:489 and VL of SEQ ID NO:493.

In some embodiments, the amino acid sequences of the heavy chain variable region (VH) and the light chain variable region (VL) each independently have at least 80%, 85%, 90%, 95%, 96%, 97%, 98%, 99%, or 100% sequence identity with any of the above sequences.

In some embodiments, the present invention relates to an isolated antibody or antigen-binding fragment thereof that specifically binds to HBsAg, characterized in that the isolated antibody or antigen-binding fragment thereof comprises a heavy chain variable region VH and a light chain variable region VL, wherein the heavy chain variable region VH comprises CDRH1 of SEQ ID NO:50, CDRH2 of SEQ ID NO:51, and CDRH3 of SEQ ID NO:52, and the light chain variable region VL comprises CDRL1 of SEQ ID NO:54, CDRL2 of SEQ ID NO:55, and CDRL3 of SEQ ID NO:56.

In some embodiments, the present invention relates to an isolated antibody or antigen-binding fragment thereof that specifically binds to HBsAg, characterized in that the isolated antibody or antigen-binding fragment thereof comprises the heavy chain variable region VH of SEQ ID NO:49 and the light chain variable region VL of SEQ ID NO:53.

In some embodiments, the present invention relates to an isolated antibody or antigen-binding fragment thereof that specifically binds to HBsAg, characterized in that the isolated antibody or antigen-binding fragment thereof comprises a heavy chain variable region VH and a light chain variable region VL, wherein the heavy chain variable region VH comprises CDRH1 of SEQ ID NO:234, CDRH2 of SEQ ID NO:235, and CDRH3 of SEQ ID NO:236, and the light chain variable region VL comprises CDRL1 of SEQ ID NO:238, CDRL2 of SEQ ID NO:239, and CDRL3 of SEQ ID NO:240.

In some embodiments, the present invention relates to an isolated antibody or antigen-binding fragment thereof that specifically binds to HBsAg, characterized in that the isolated antibody or antigen-binding fragment thereof comprises the heavy chain variable region VH of SEQ ID NO:233 and the light chain variable region VL of SEQ ID NO:237.

In some embodiments, the present invention relates to an isolated antibody or antigen-binding fragment thereof that specifically binds to HBsAg, characterized in that the isolated antibody or antigen-binding fragment thereof comprises a heavy chain variable region VH and a light chain variable region VL, wherein the heavy chain variable region VH comprises CDRH1 of SEQ ID NO:282, CDRH2 of SEQ ID NO:283, and CDRH3 of SEQ ID NO:284, and the light chain variable region VL comprises CDRL1 of SEQ ID NO:286, CDRL2 of SEQ ID NO:287, and CDRL3 of SEQ ID NO:288.

In some embodiments, the present invention relates to an isolated antibody or antigen-binding fragment thereof that specifically binds to HBsAg, characterized in that the isolated antibody or antigen-binding fragment thereof comprises the heavy chain variable region VH of SEQ ID NO:281 and the light chain variable region VL of SEQ ID NO:285.

The sequence information of the antibody of the present invention is shown in Table 2 below.

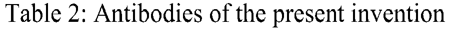

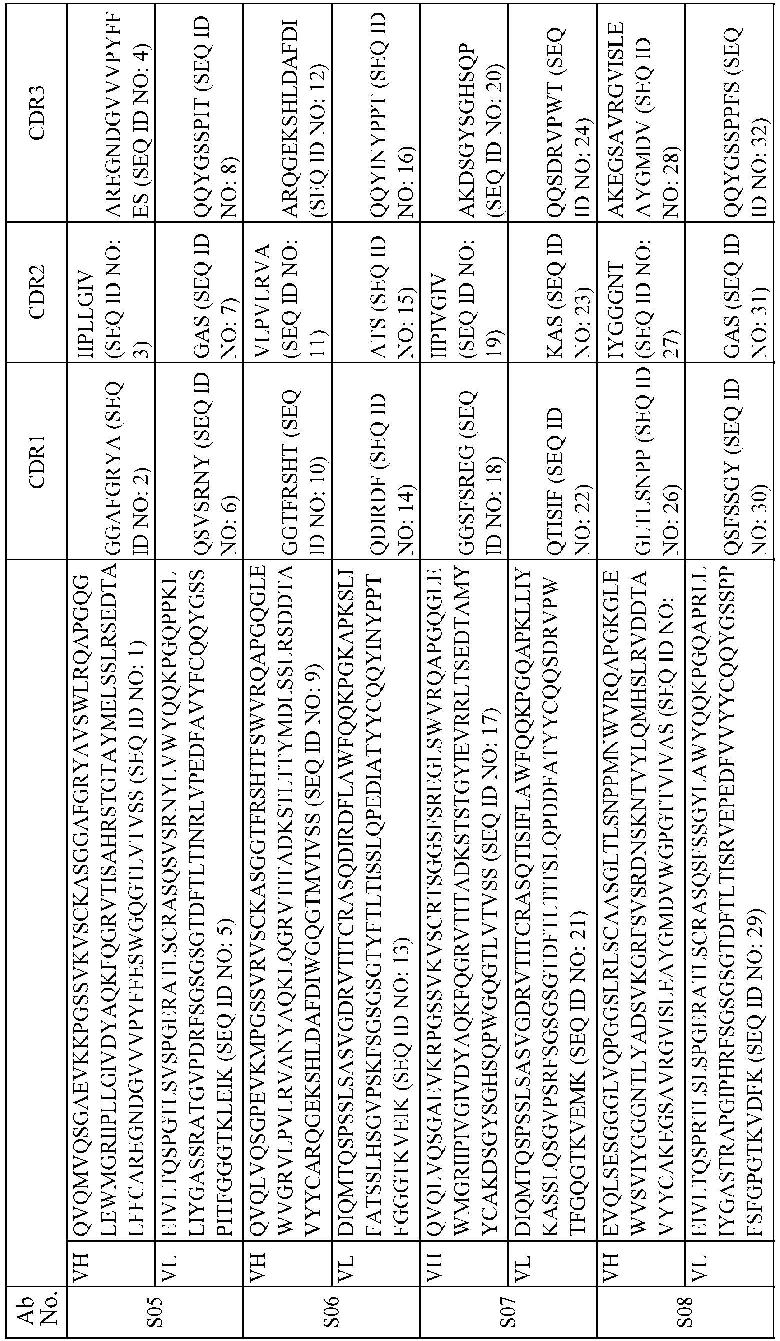

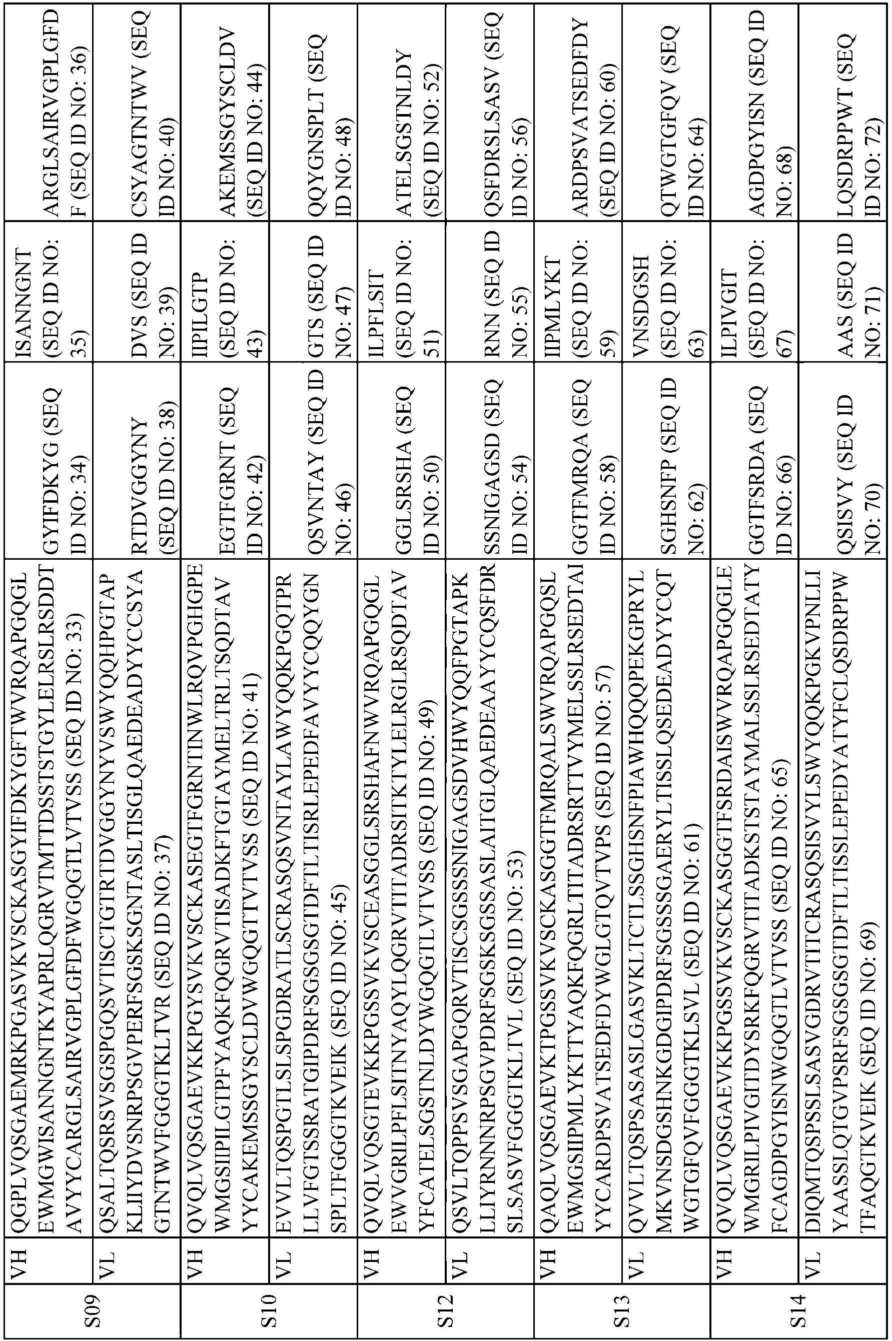

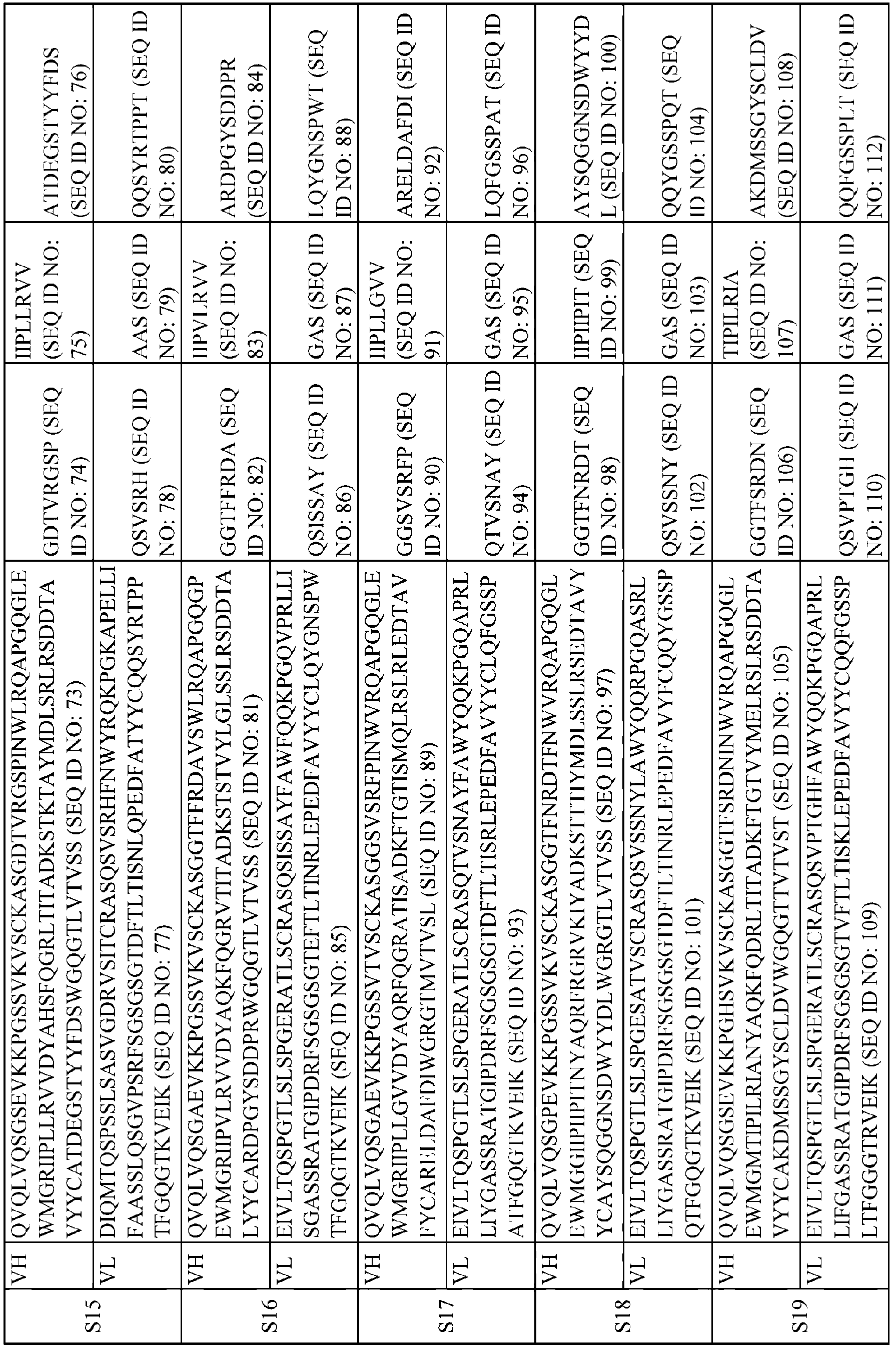

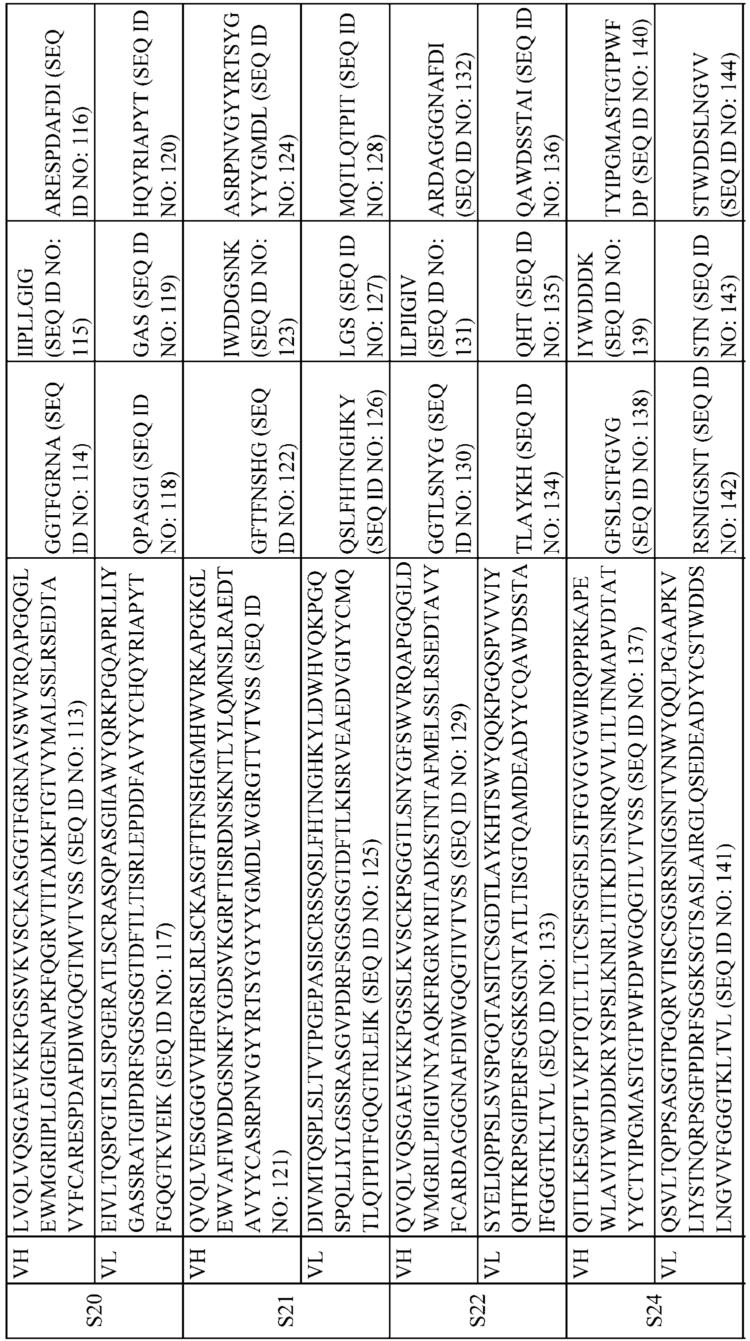

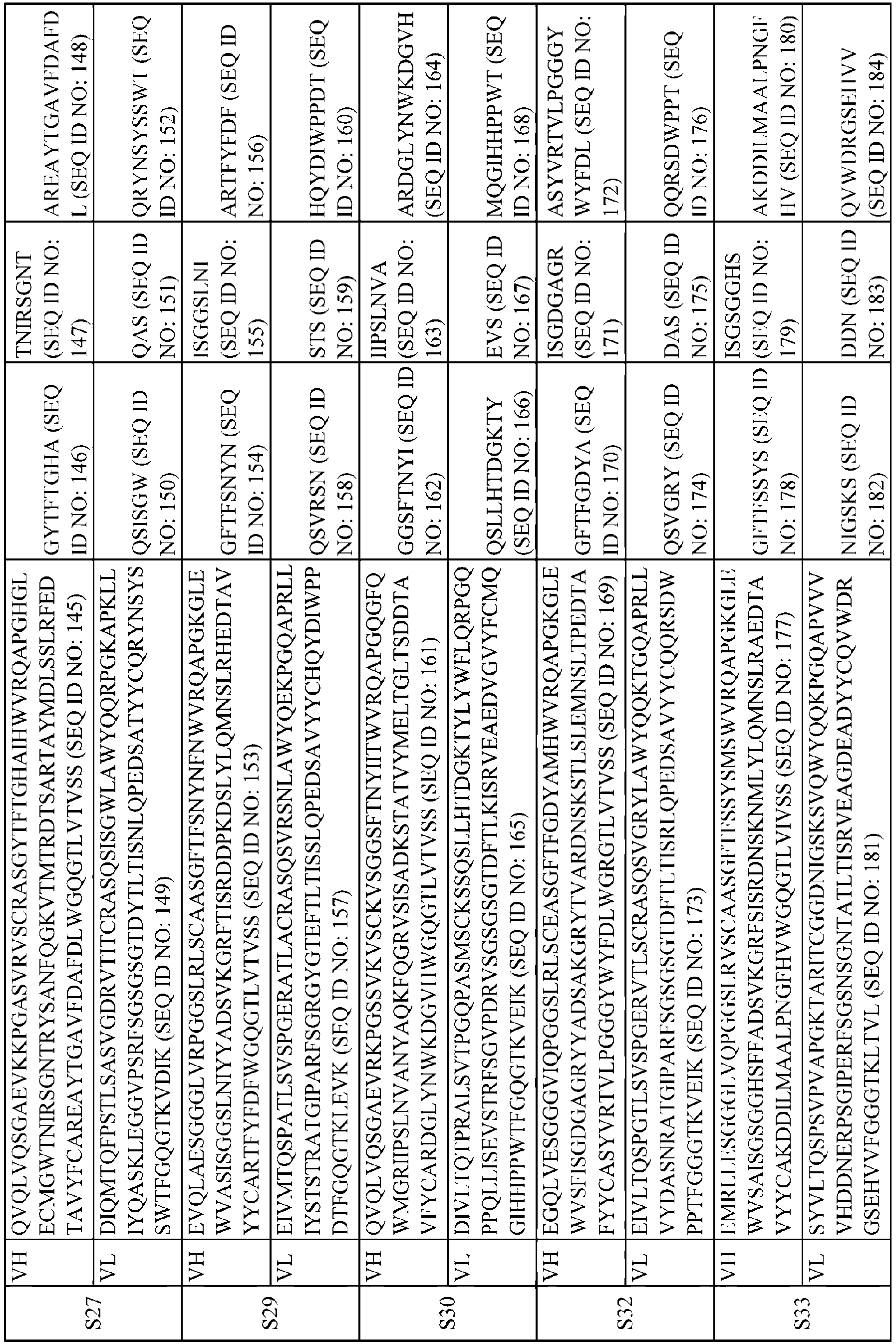

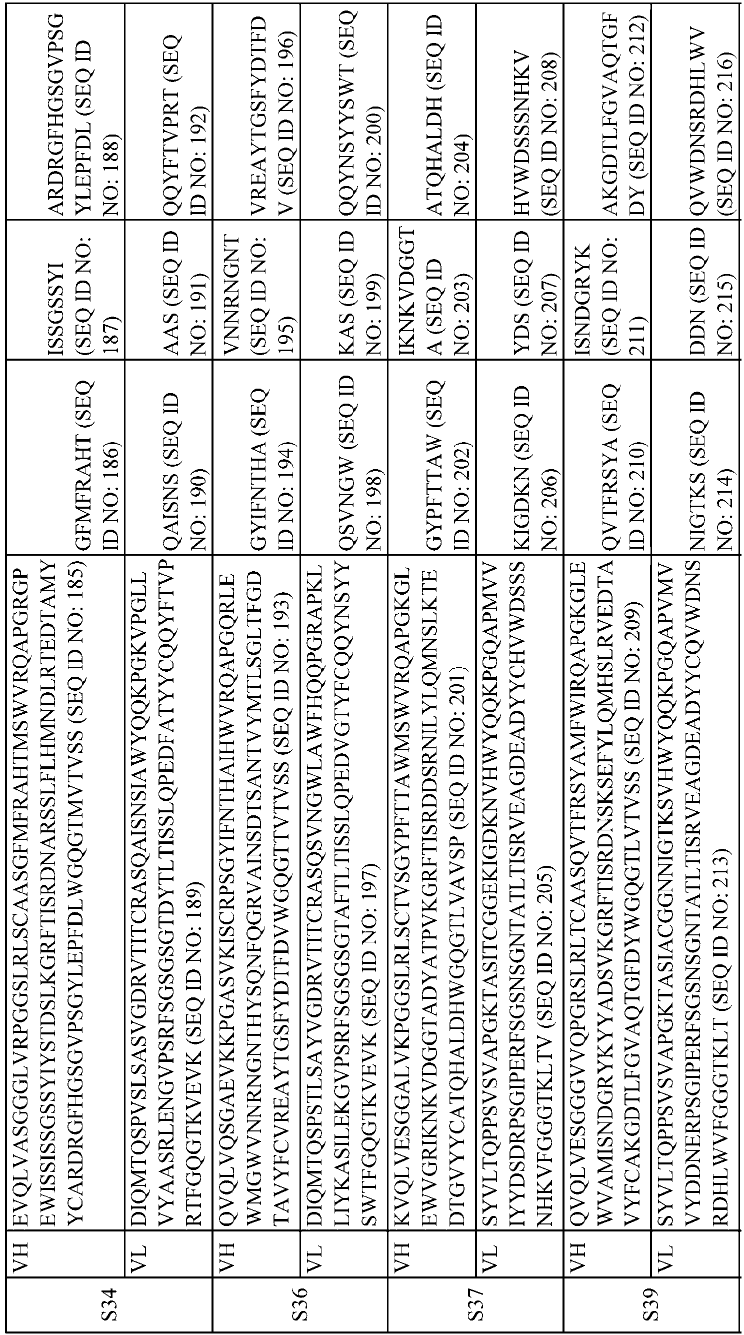

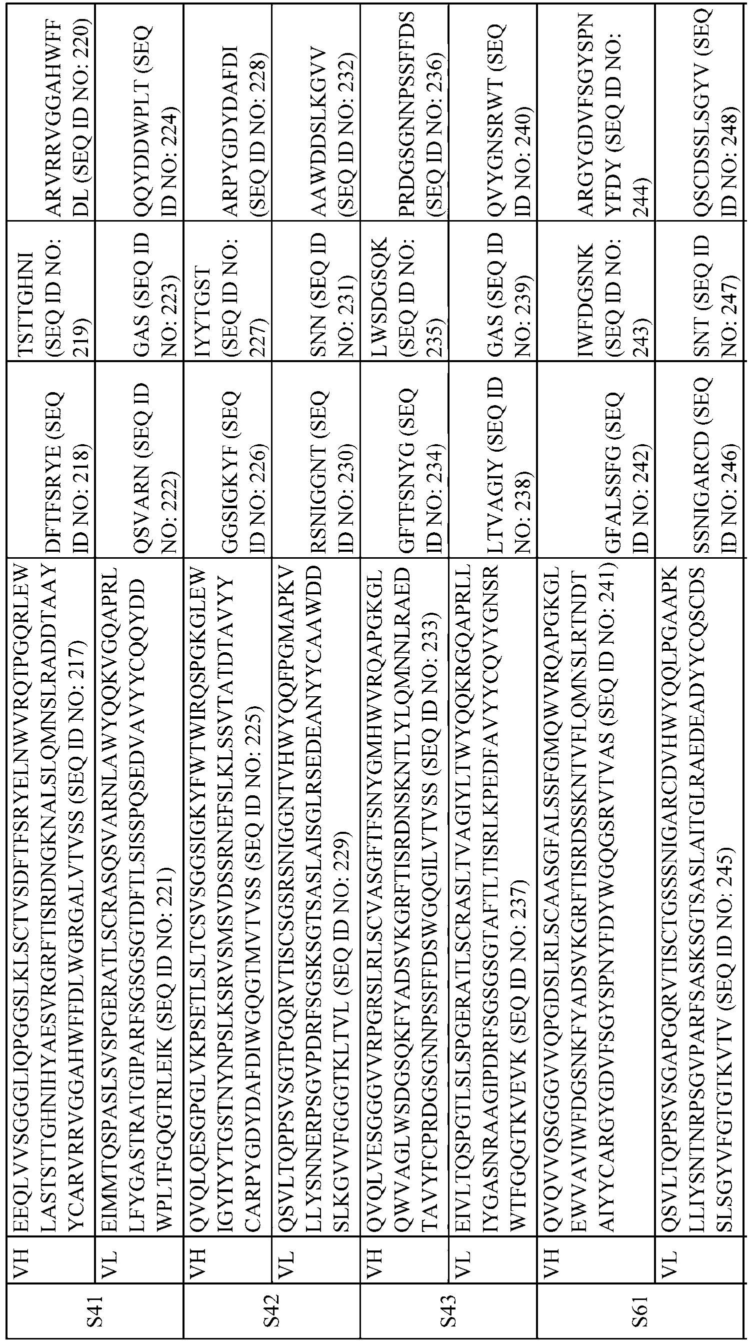

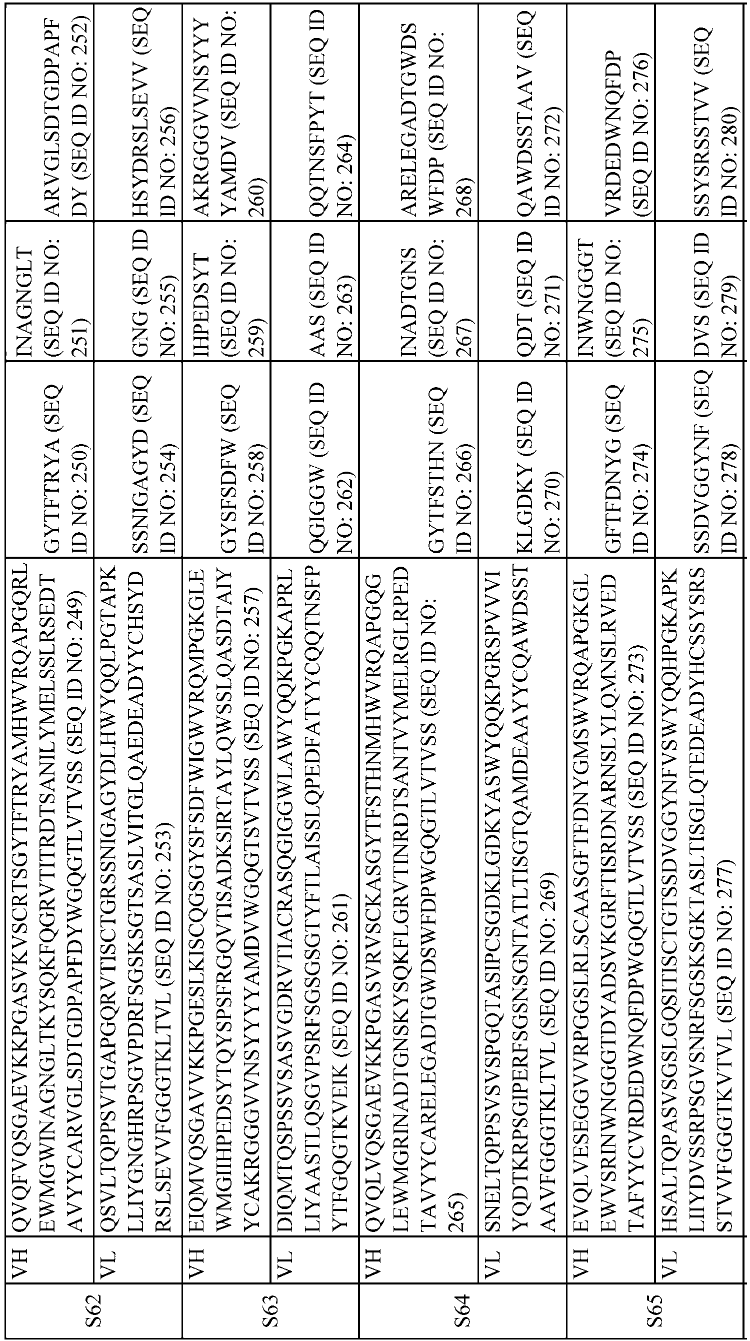

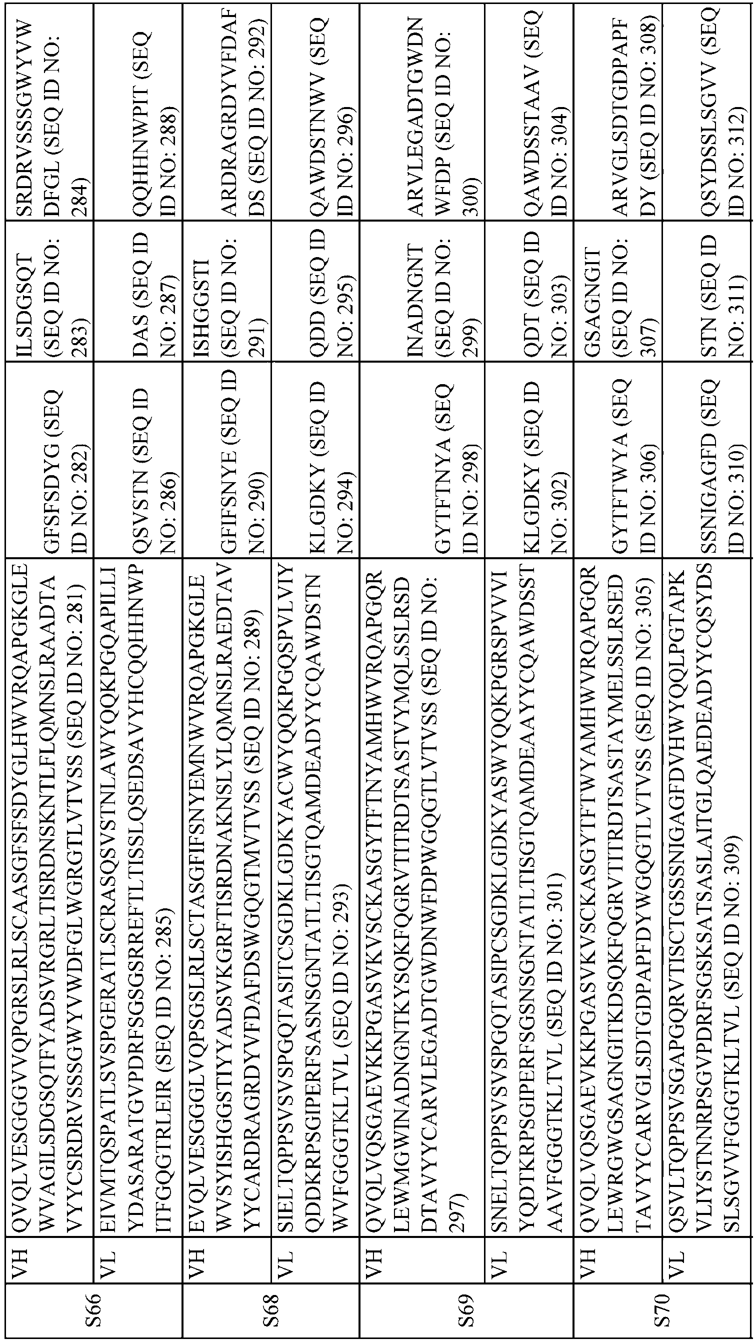

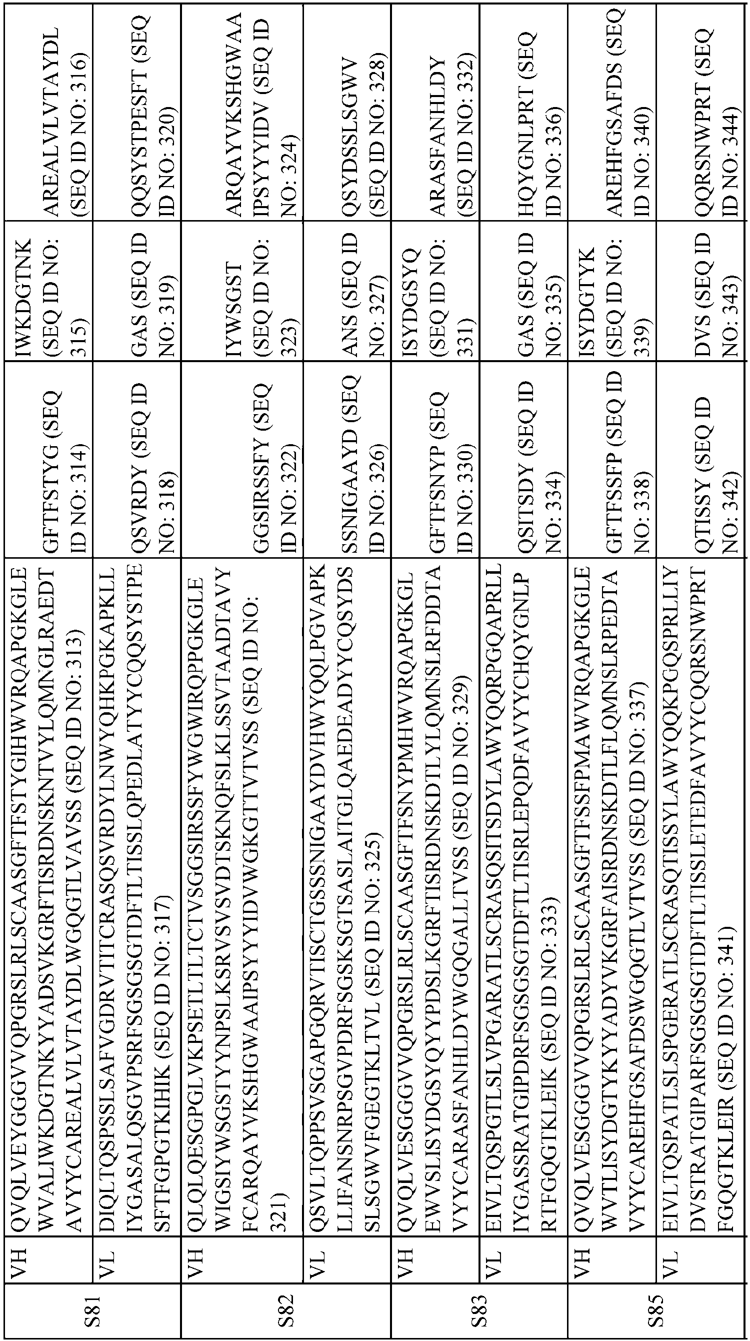

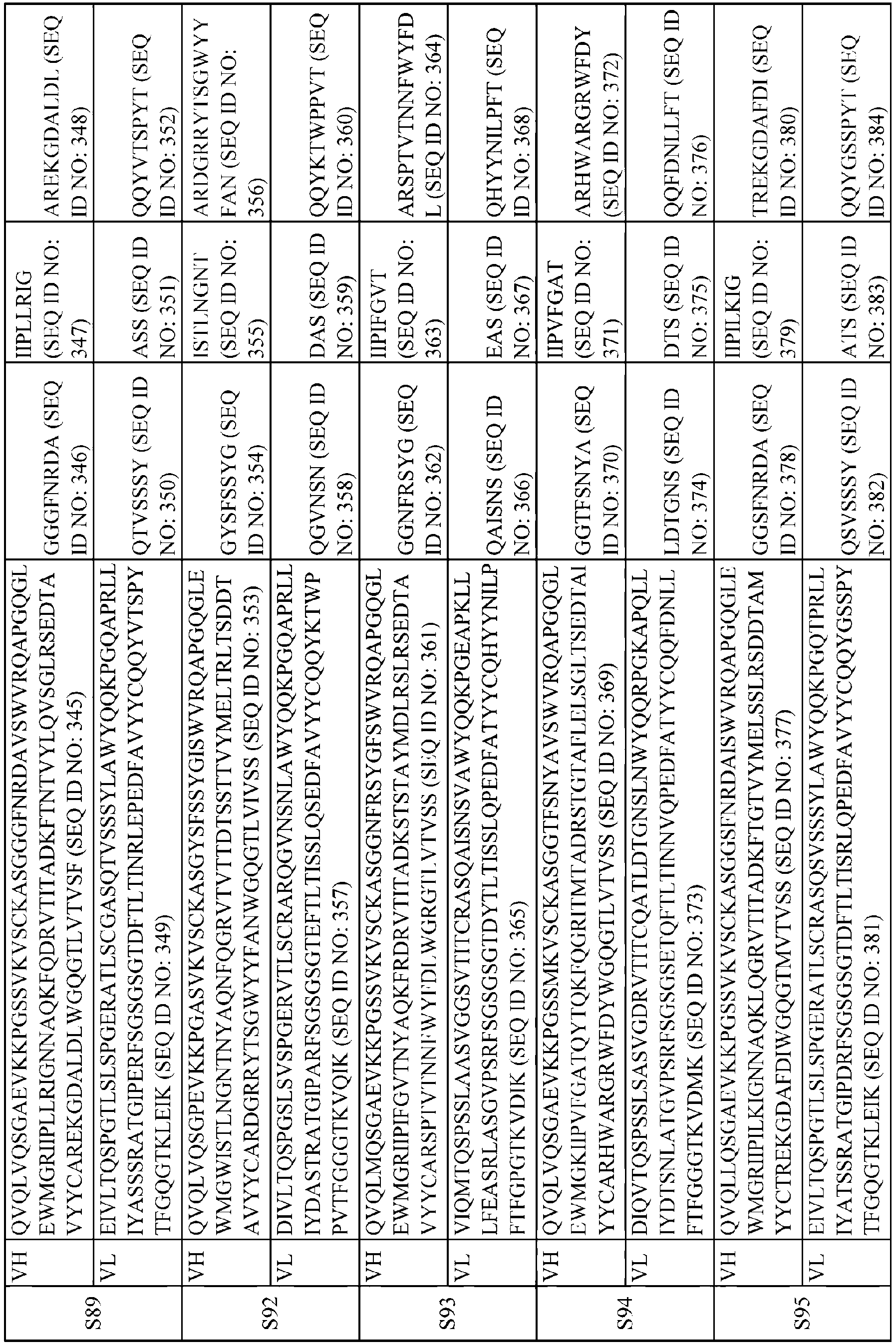

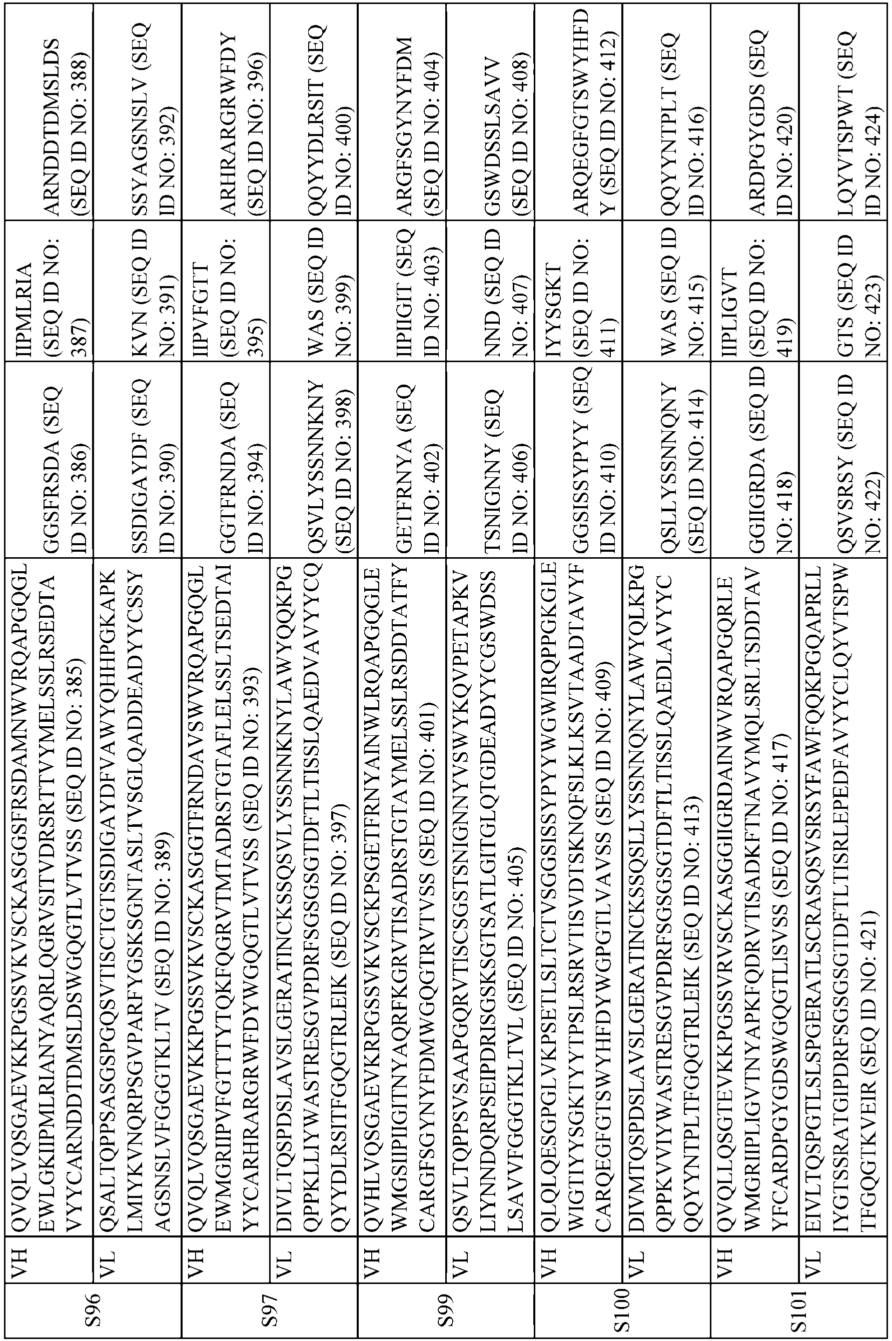

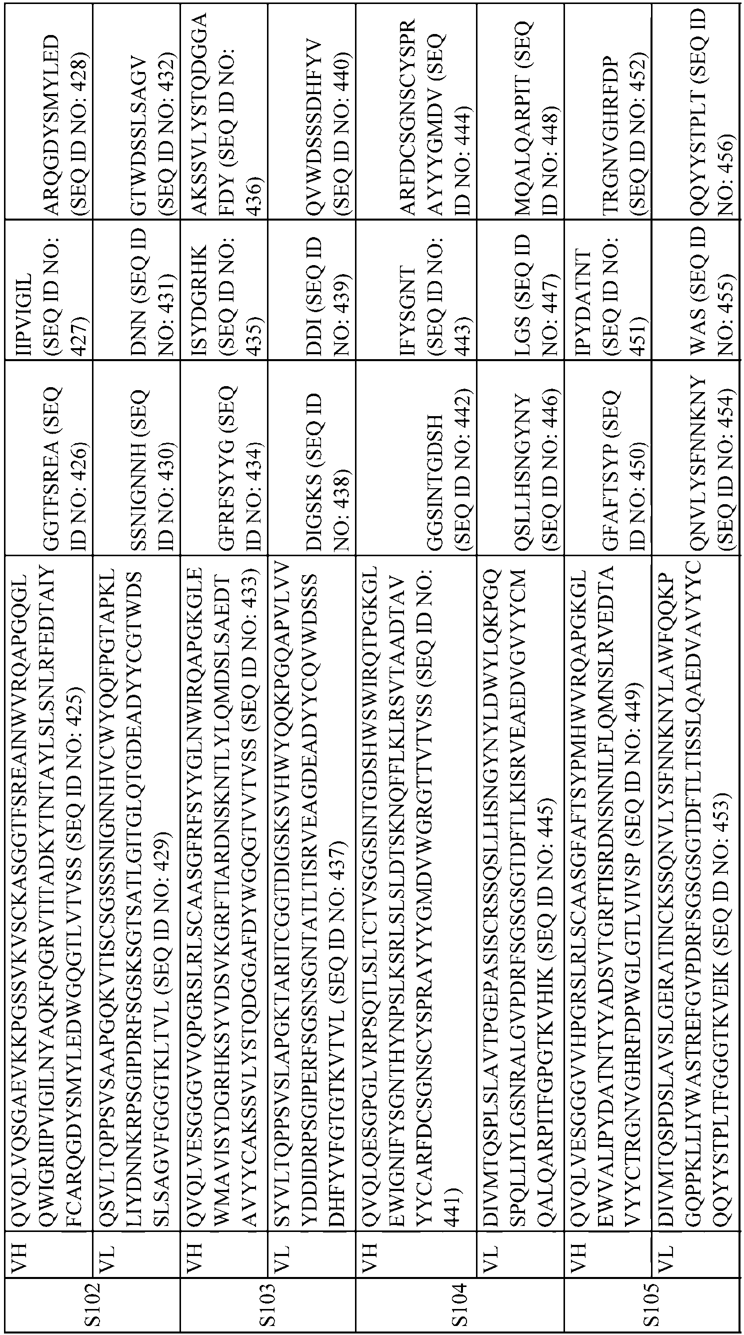

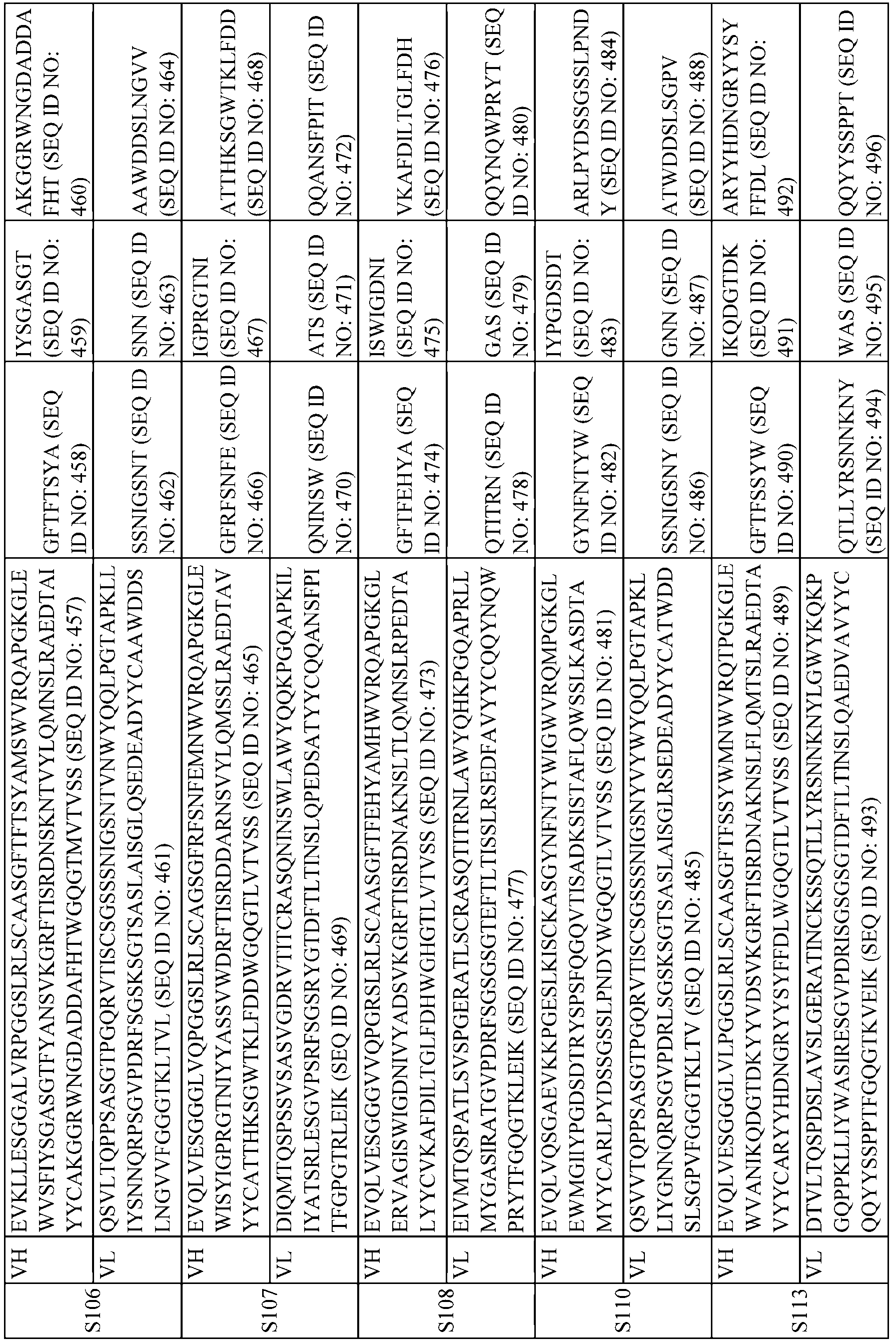

Variants of the antibodies of the present invention comprising the VH or VL amino acid sequences described above are within the scope of the present invention. For example, variants may contain 1, 2, 3, 4, 5, 6, 7, 8, 9, 10, or 11 amino acid substitutions in VH and/or VL that do not adversely affect the properties of the antibody. In some embodiments, the sequence identity relative to the VH or VL amino acid sequence of the present invention may be about 90%, 91%, 92%, 93%, 94%, 95%, 96%, 97%, 98%, or 99%. Exemplary modifications are, for example, conservative amino acid substitutions at the antigen-binding sites or within the frame regions that do not adversely alter the properties of the antibody. Conservative substitutions may also be made to improve the properties of the antibody, such as stability or affinity. Conservative substitutions are those that occur in side chains within the associated amino acid family. The amino acids encoded by genes can be divided into four categories: (1) acidic (aspartic acid, glutamic acid); (2) basic (lysine, arginine, histidine); (3) nonpolar (alanine, valine, leucine, isoleucine, proline, phenylalanine, methionine, tryptophan); and (4) uncharged and polar (glycine, asparagine, glutamine, cysteine, serine, threonine, tyrosine). Phenylalanine, tryptophan, and tyrosine are sometimes classified together as aromatic amino acids. Alternatively, all amino acid components can be grouped as: (1) acidic (aspartic acid, glutamic acid); (2) basic (lysine, arginine, histidine); (3) aliphatic (glycine, alanine, valine, leucine, isoleucine, serine, threonine), wherein serine and threonine are optionally grouped as hydroxyl-containing aliphatic; (4) aromatic (phenylalanine, tyrosine, tryptophan); (5) amide (asparagine, glutamine); and (6) sulfur-containing (cysteine and methionine) (Stryer (ed.), Biochemistry, 2nd edition, WH Freeman and Co., 1981). Furthermore, any native residue in the polypeptide can be substituted with alanine, as previously described with alanine scanning mutagenesis (MacLennan et al. (1998) Acta Physiol. Scand. Suppl. 643:55-67; Sasaki et al. (1998) Adv. Biophys. 35:1-24). The desired amino acid substitutions can be determined by those skilled in the art when such substitutions are required. The assays described herein can be used to characterize the resulting antibody variants.

Amino acid substitutions can be performed, for example, by PCR mutagenesis (US Patent No. 4,683,195). Alternatively, a variant library can be generated using known methods, such as using random (NNK) codons or non-random codons (e.g., DVK codons encoding 11 amino acids (Ala, Cys, Asp, Glu, Gly, Lys, Asn, Arg, Ser, Tyr, Trp)), and then variants with the desired properties can be screened within the library.

Although the antibodies of the present invention comprise paired variable regions, one from the heavy chain and one from the light chain, those skilled in the art will recognize that alternative embodiments may include a single heavy chain variable region or a single light chain variable region. A single variable region can be used to screen for variable domains capable of forming two-domain-specific antigen-binding fragments. This screening can be performed using phage display screening methods that employ, for example, the graded dual-combination method disclosed in International Patent Publication No. WO92/01047. In this combination method, a single colony containing either an H-chain clone or an L-chain clone is used to infect a complete library of clones encoding the other chain (L or H), and the resulting double-chain-specific antigen-binding domain is then selected according to the described phage display technique. Therefore, separate VH and VL polypeptide chains can be used to identify additional antibodies that specifically bind to the S protein domain, as described in the method disclosed in International Patent Publication No. WO92/01047.

Various techniques for antibody production can be used to prepare the antibodies of the present invention. For example, the hybridoma method proposed by Kohler and Milstein in Nature 256:495, 1975 can be used to produce monoclonal antibodies. In the hybridoma method, mice or other host animals (such as hamsters, rats, or monkeys) are immunized with the S protein or a fragment thereof, and then spleen cells from the immunized animal are fused with myeloma cells using standard methods to form hybridoma cells (Goding, Monoclonal Antibodies: Principles and Practice, pp.59-103 (Academic Press, 1986)). Colonies produced by individual immortalized hybridoma cells are screened for the preparation of antibodies with the desired properties (such as binding specificity, cross-reactivity, or lack thereof, and affinity for the antigen).

The antibodies of the present invention can be prepared using various host animals. For example, Balb/c mice can be used to prepare the antibodies. Various techniques can be used to humanize antibodies prepared in Balb/c mice and other non-human animals to produce more human-like sequences. Exemplary humanization techniques, including the selection of human receptor frameworks, are known to those skilled in the art, including CDR grafting (US Patent No. 5,225,539), SDR grafting (US Patent No. 6,818,749), surface remodeling (Padlan, Mol Immunol 28:489-499, 1991), surface remodeling of specificity-determining residues (US Patent Publication No. 20100261620), human modification (or human framework modification) (US Patent Publication No. US2009/0118127), hyperhumanization (US Patent No. 7,709,226), and directional selection (Osbourn et al. (2005) Methods 36:61-68, 2005; US Patent No. 5,565,332).

Humanized antibodies can be further optimized to improve their selectivity or affinity for desired antigens by introducing modified framework-supporting residues to maintain binding affinity (reverse mutation), for example by employing techniques disclosed in International Patent Publication Nos. WO90/007861 and WO92/22653.

Transgenic mice carrying human immunoglobulin (Ig) loci in their genome can be used to produce human antibodies against target proteins, as described in, for example, International Patent Publication No. WO90/04036, US Patent No. 6150584, International Patent Publication No. WO99/45962, International Patent Publication No. WO02/066630, International Patent Publication No. WO02/43478; Lonberg et al (1994) Nature 368:856-9; Green et al (1994) Nature Genet. 7:13-21; Green & Jakobovits (1998) Exp.Med. 188:483-95; Lonberg and Huszar (1995) Int. Rev. Immunol. 13:65-93; Bruggemann et al. (1991) Eur. J. Immunol. 21:1323-1326; Fishwild et al. (1996) Nat. Biotechnol. 14:845-851; Mendez et al. (1997) Nat. Genet. 15:146-156; Green (1999) J. Immunol. Methods 231:11-23; Yang et al. (1999) Cancer Res. 59:1236-1243; Brüggemann and Taussig (1997) Curr. Opin. Biotechnol. 8:455-458; International Patent Publication No. WO02/043478. This method can disrupt or delete endogenous immunoglobulin loci in such mice, and can use transchromosome or microgene to insert at least one complete or partial human immunoglobulin locus into the mouse genome via homologous or non-homologous recombination.

Human antibodies can be selected from phage display libraries in which phages are engineered to express human immunoglobulins or portions thereof, such as Fab, single-chain antibodies (scFv), or unpaired or paired antibody variable regions (Knappik et al (2000) J. Mol. Biol. 296:57-86; Krebs et al (2001) J. Immunol. Meth. 254:67-84; Vaughan et al (1996) Nature Biotechnology 14:309-314*;* Sheets et al (1998) PITAS (USA) 95:6157-6162; Hoogenboom and Winter, (1991) J. Mol. Biol. 227:381; Marks et al (1991) J. Mol. Biol. 222:581). The antibodies of the present invention can be isolated from, for example, a phage display library, which expresses the heavy and light chain variable regions of the antibody as a fusion protein with the phage pIX coat protein, as described in Shi et al. (2010) J. Mol. Biol. 397:385-96 and International Patent Publication No. WO09/085462. Phages binding to the S protein in the library can be screened, and the obtained positive clones can be further characterized. Fab can be isolated from the clone lysate and expressed as a full-length IgG. Such phage display methods for isolating human antibodies are described, for example, in U.S. Patent Nos. 5,223,409, 5,403,484, and 5,571,698 to Ladner et al.; U.S. Patent Nos. 5,427,908 and 5,580,717 to Dower et al.; U.S. Patent Nos. 5,969,108 and 6,172,197 to McCafferty et al.; and U.S. Patent Nos. 5,885,793, 6,521,404, 6,544,731, 6,555,313, 6,582,915, and 6,593,081 to Griffiths et al.

The preparation of immunogenic antigens and the generation of monoclonal antibodies can be performed using any suitable technique, such as recombinant protein production. Immunogenic antigens can be administered to animals in the form of purified proteins or mixtures of proteins (including whole cells, cell extracts, or tissue extracts), or the antigens can be de novo formed in animals from nucleic acids encoding the antigen or a portion thereof.

The antibodies of the present invention can be bispecific or multispecific antibodies. Exemplary antibodies that can be used to engineer bispecific and multispecific antibodies comprising a first domain that specifically binds to a first epitope on hepatitis B surface antigen (HBsAg) and a second domain that specifically binds to a second epitope on HBsAg include antibodies selected from the V_{H}/V_{L} sequences and heavy/light chain CDRs shown in Table 2, as well as engineered variants thereof. In one embodiment, the antibodies used to engineer the first and second domains, and possibly more domains, are the same, or bind to the same epitope. In this case, the bispecific and multispecific antibodies are also referred to as bivalent or multivalent antibodies.

In one embodiment, the antibodies used to engineer the first and second domains, and possibly more domains, are different. For example, the antibodies used to engineer the first and second domains are each independently selected from different groups of antibodies:
Group I: S33, S39, S64, S103 and S106;
Group II: S29, S43, S41, S66 and S81; and
Group III: S5, S12, S16, S17, S19, S20, S82, S89, S92, S95, S96 and S101.

The bispecific antibodies provided herein include antibodies with a full-length antibody structure.

A "Fab arm" or "half-molecule" refers to one heavy-light chain pair that specifically binds to an antigen.

As described herein, the full-length bispecific antibody can be generated, for example, through Fab arm exchange (or half-molecule exchange) between two monospecific bivalent antibodies, by introducing a substitution at the heavy chain CH3 junction in each half-molecule to facilitate the formation of heterodimers of two antibody half-molecules with different specificities in an in vitro cell-free environment or using co-expression. The Fab arm exchange reaction is the result of disulfide bond isomerization and CH3 domain dissociation-association. The heavy chain disulfide bond in the hinge region of the parent monospecific antibody is reduced. The resulting free cysteine from one of the parent monospecific antibodies forms an inter-heavy chain disulfide bond with the cysteine residue of the second parent monospecific antibody molecule, while the CH3 domains of the parent antibodies are released and reformed through dissociation-association. The CH3 domains of the Fab arms can be modified to promote heterodimerization rather than homodimerization. The resulting product is a bispecific antibody with two Fab arms or half-molecules, each binding to a different epitope.

"Homodimerization" refers to the interaction between two heavy chains that have the same CH3 amino acid sequence. A "homodimer" refers to an antibody containing two heavy chains with the same CH3 amino acid sequence.

"Heterodimerization" refers to the interaction between two heavy chains with different CH3 amino acid sequences. A "heterodimer" is an antibody containing two heavy chains with different CH3 amino acid sequences.

In some embodiments, bispecific antibodies include designs such as Triomab/Quadroma (Trion Pharma/Fresenius Biotech), Knob-into-Hole (Genentech), CrossMAbs (Roche), and electrostatic pairing bodies (Chugai, Amgen, NovoNordisk, Oncomed), LUZ-Y (Genentech), strand exchange engineered domain bodies (SEEDbody) (EMD Serono), Biclonic (Merus), and DuoBody^{®} technology (Genmab A/S).

The Triomab quadroma technology can be used to generate full-length bispecific antibodies incorporating the VH and VL regions of the antibodies of the invention. The Triomab technology facilitates Fab arm exchange between two parental chimeric antibodies, one parental mAb having IgG2a and the second parental mAb having the rat IgG2b constant region, thereby generating chimeric bispecific antibodies.

The "Knob-into-Hole" strategy (see, for example, international publication WO 2006/028936) can be used to generate full-length bispecific antibodies. In short, selected amino acids that form the CH3 domain boundary in human IgG can be mutated at positions affecting CH3 domain interactions, thereby promoting heterodimer formation. Amino acids with small side chains (Hole) are introduced into the heavy chain of an antibody that specifically binds to the first antigen, and amino acids with large side chains (Knob) are introduced into the heavy chain of an antibody that specifically binds to the second antigen. After co-expression of the two antibodies, heterodimers are formed due to the preferential interaction between the heavy chains with "Knob" and those with "Hole". Exemplary CH3 substitution pairs forming Knob and Hole (represented as modification positions in the first CH3 domain of the first heavy chain / modification positions in the second CH3 domain of the second heavy chain) are: T366Y/F405A, T366W/F405W, F405W/Y407A, T394W/Y407T, T394S/Y407A, T366W/T394S, F405W/T394S, and T366W/T366S_L368A_Y407V.

CrossMAb technology can be used to generate full-length bispecific antibodies. In addition to facilitating Fab arm exchange using the "Knob-into-Hole" strategy, CrossMAb has exchanged CH1 and CL domains in one of its halves to ensure proper light chain pairing of the resulting bispecific antibody (see, for example, U.S. Patent 8,242,247).

Other exchange strategies can be used to generate full-length bispecific antibodies by exchanging variable or constant domains, or both, between the heavy chain and the light chain or within the heavy chain in one or both arms of the bispecific antibody. These exchanges include, for example, VH-CH1 and VL-CL, VH and VL, CH3 and CL, and CH3 and CH1, as described in international patent publications WO2009/080254, WO2009/080251, WO2009/018386, and WO2009/080252.

Other strategies can also be used, such as promoting heterodimerization of heavy chains by electrostatic interactions through the substitutions of positively charged residues on one CH3 surface and negatively charged residues on a second CH3 surface, as described in U.S. Patent Publications US2010/0015133, US2009/0182127, US2010/028637, or US2011/0123532. In other strategies, heterodimerization can be promoted by the following substitutions (represented as the modification position in the first CH3 domain of the first heavy chain / the modification position in the second CH3 domain of the second heavy chain): L351Y_F405A_Y407V/T394W, T366I_K392M_T394W/F405A_Y407V, T366L_K392M_T394W/F405A_Y407V, L351Y_Y 407A/T366A_K409F, L351Y_Y407A/T366V_K409F, Y407A/T366A_K409F, or T350V_L351Y_F405A_Y407V/T350V_T366L _K392L_T394W, as described in U.S. Patent Publications US2012/0149876 or US2013/0195849.

The LUZ-Y technique can be used to generate bispecific antibodies. In this technique, a leucine zipper is added to the C-terminus of the CH3 domain to drive the assembly of a heterodimer from a parent mAb, which is subsequently purified and removed, as described in Wranik et al., 2012, J Biol Chem, Vol. 287, No. 52, pp. 42221-42229.

SEEDbody technology can be used to generate bispecific antibodies. SEEDbodies have selected IgG residues substituted with IgA residues in their constant domain to facilitate heterodimerization, as described in U.S. Patent US20070287170.

According to the method described in International Patent Publication WO2011/131746, the bispecific antibody described herein can be produced in vitro in a cell-free environment by introducing an asymmetric mutation into the CH3 region of two monospecific homodimers and forming a bispecific heterodimer from the two parental monospecific homodimers under reducing conditions that allow disulfide isomerization. In these methods, the first and second monospecific bivalent antibodies are engineered to have certain substitutions at the CH3 domain that promote heterodimer stability; these antibodies are incubated together under reducing conditions sufficient to cause disulfide isomerization of cysteine in the hinge region; thereby generating a bispecific antibody via Fab arm exchange. The incubation conditions are then restored to non-reducing conditions. Exemplary reducing agents that can be used are 2-mercaptoethylamine (2-MEA), dithiothreitol (DTT), dithioerythritol (DTE), glutathione, tris(2-carboxyethyl)phosphine (TCEP), L-cysteine, and β-mercaptoethanol. For example, the following conditions can be used: incubation for at least 90 minutes in the presence of at least 25 mM 2-MEA or at least 0.5 mM dithiothreitol at a pH of 5-8, such as pH 7.0 or pH 7.4, at a temperature of at least 20°C.

In some embodiments described herein, a bispecific antibody comprising a first domain that specifically binds to a first epitope on hepatitis B surface antigen (HBsAg) and a second domain that specifically binds to a second epitope on HBsAg comprises at least one substitution in the CH3 constant domain of the antibody.

In some embodiments described herein, the at least one substitution in the CH3 constant domain of the antibody is a K409R, a F405L, or F405L and R409K substitutions, wherein the residues are numbered according to the EU index.

Antibody domains and position numbering are well-known. "Asymmetry" refers to the different substitutions within the two CH3 domains of the two independent heavy chains of an antibody. The IgG1 CH3 region is typically composed of residues 341-446 on IgG1 (the residues are numbered according to the EU index).

In some embodiments described herein, the bispecific antibody is an IgG1 isotype and, when compared with wild-type IgG1, comprises an F405L substitution in the first heavy chain (HC1) and a K409R substitution in the second heavy chain (HC2).

In some embodiments described herein, the bispecific antibody is an IgG1 isotype and, when compared with wild-type IgG1, comprises a K409R substitution in the first heavy chain (HC1) and an F405L substitution in the second heavy chain (HC2).

In some embodiments described herein, the bispecific antibody is an IgG4 isotype, and when compared with wild-type IgG4, comprises an S228P substitution in HC1 and S228P, F405L, and R409K substitutions in HC2.

In some embodiments described herein, the bispecific antibody is an IgG4 isotype, and when compared with wild-type IgG4, comprises S228P, F405L and R409K substitutions in HC1 and an S228P substitution in HC2.

In some embodiments described herein, the bispecific antibody is an IgG4 isotype, and when compared with wild-type IgG4, comprises S228P, F234A, and L235A substitutions in HC1 and S228P, F234A, L235A, F405L, and R409K substitutions in HC2.

In some embodiments described herein, the bispecific antibody is an IgG4 isotype, and when compared with wild-type IgG4, comprises S228P, F234A, L235A, F405L and R409K substitutions in HC1 and S228P, F234A and L235A substitutions in HC2.

In some embodiments described herein, when the residues are numbered according to the EU index, the bispecific antibody of the present invention comprises at least one, two, three, four, five, six, seven or eight asymmetric substitutions at residue positions 350, 366, 368, 370, 399, 405, 407 or 409 in HC1 and HC2.

In some embodiments described herein, when the residues are numbered according to the EU index, the bispecific antibody of the present invention comprises at least one, two, three or four asymmetric substitutions at residue positions 350, 370, 405 or 409 in HC1 and HC2.

In some embodiments described herein, when the residues are numbered according to the EU index, the bispecific antibody of the present invention comprises at least one asymmetric substitution at residue position 405 or 409 in HC1 and HC2.

Standard methods are typically used to perform substitutions at the DNA level to the molecular level (such as constant domains of antibodies).

The antibodies of the present invention can be engineered into various known forms of antibody.

In some embodiments, the bispecific antibody of the present invention is a cross-body.

In some embodiments, the bispecific antibodies of the present invention include recombinant IgG-like dual-targeting molecules, wherein each flanking the molecule contains Fab fragments or portions thereof from at least two different antibodies; IgG fusion molecules, wherein a full-length IgG antibody is fused with an additional Fab fragment or a portion thereof; Fc fusion molecules, wherein an scFv molecule or a stable diabody is fused with a heavy chain constant domain, an Fc region, or a portion thereof; Fab fusion molecules, wherein different Fab fragments are fused together; and heavy chain antibodies (e.g., domain antibodies, nanobodies) based on scFvs and diabodies, wherein different scFv molecules or different diabodies or different heavy chain antibodies (e.g., domain antibodies, nanobodies) are fused with each other or with another protein or carrier molecule.

The domain antibodies based on scFv and bivalent antibody include bispecific T-cell engagers (BITE) (Micromet), tandem bivalent antibodies (Tandab) (Affimed), dual-affinity retargeting molecules (DART) (MacroGenics), single-chain bivalent antibodies (Academic), TCR-like antibodies (AIT, ReceptorLogics), human serum albumin-scFv fusions (Merrimack) and COMBODY (Epigen Biotech), dual-targeting nanobodies (Ablynx), and dual-targeting only heavy chain domain antibodies. Various forms of bispecific antibodies have been described, for example, in Chames and Baty, 2009, Curr Opin Drug Disc Dev, Vol. 12, p. 276, and Nunz-Prado et al., 2015, Drug Discovery Today, Vol. 20, No. 5, pp. 588-594.

In some embodiments, the first and second domains of the bispecific antibody of the present invention are fused together (i.e., covalently linked). As used herein, the term "fusion" means that the first and second domains of the bispecific antibody of the present invention are directly or indirectly linked together by covalent linkage. In some embodiments, fusion includes, but is not limited to, direct fusion via peptide bonds or non-peptide bonds, or indirect fusion via linkers such as peptide linkers. Those skilled in the art will understand that any direct or indirect linking methods known in the art or that may be discovered in the future for linking polypeptides together can be used as "fusion" in this disclosure.

The first and second domains can be directly linked by a single chemical bond (such as a peptide bond) or via a peptide linker. The first domain can be fused at the N-terminus or C-terminus of any (including each) polypeptide of the second domain, or at an internal location of any (including each) polypeptide of the second domain, such as at the N-terminus of the Fc region in the heavy chain of the second domain. The fused polypeptides can be obtained through recombination or chemical methods.

The bispecific antibody of the present invention may include one or more peptide linkers located between a first domain and a second domain. In some embodiments, the first and second domains are directly fused to each other without peptide linkers.

In some embodiments, the peptide linkers connecting different domains may be the same or different. Each peptide linker can be optimized individually. The peptide linkers can have any suitable length. In some embodiments, the peptide linkers are at least about any of 1, 2, 3, 4, 5, 6, 7, 8, 9, 10, 11, 12, 13, 14, 15, 16, 17, 18, 19, 20, 25, 30 or more amino acids in length. In some embodiments, the peptide linker is any of about 1 amino acid to about 10 amino acids, about 1 amino acid to about 20 amino acids, about 1 amino acid to about 30 amino acids, about 5 amino acids to about 15 amino acids, about 10 amino acids to about 25 amino acids, about 5 amino acids to about 30 amino acids, or about 10 amino acids to about 30 amino acids in length. The peptide linkers can have naturally occurring sequences or non-naturally occurring sequences. For example, sequences derived only from the hinge region of the antibody heavy chain can be used as linkers. See, for example, WO1996/34103. In some embodiments, the peptide linker is a flexible linker. Exemplary flexible linkers include glycine polymers (G)n (n being an integer of at least 1), glycine-serine polymers (including, for example, (GS)n, (GSGGS)n, (GGGS)n, (GGGGS)n, where n is an integer of at least 1), glycine-alanine polymers, alanine-serine polymers, and other flexible linkers known in the art. In some embodiments, the peptide linker comprises the amino acid sequence GGGGSGGGS or GGGGSGGGGSGGGGS (also known as (G4S)₃). In some embodiments, the peptide linker comprises a hinge region of IgG, such as the hinge region of human IgG1. In some embodiments, the peptide linker comprises the amino acid sequence EPKSCDKTHTCPPCP. In some embodiments, the peptide linker comprises a modification sequence derived from the hinge region of IgG, such as the hinge region of human IgG1. For example, one or more cysteine residues in the hinge region of IgG may be replaced by serine residues. In some embodiments, the peptide linker comprises the amino acid sequence EPKSSDKTHTSPPSP.

In some embodiments, the first and/or second domains comprise a single-chain antibody fragment scFv, which includes VH and VL. In some embodiments, the bispecific antibody of the present invention further comprises an Fc region.

In some embodiments, the first domain comprises a heavy chain containing a VH domain and a light chain containing a VL domain. In some embodiments, the heavy chain further comprises one or more heavy chain constant domains, such as CH1, CH2, CH3, and CH4, and/or hinge regions (HR). In some embodiments, the light chain further comprises a light chain constant domain (CL). In some embodiments, the N-terminus of the second domain is fused with the C-terminus of the heavy chain. In some embodiments, the C-terminus of the second domain is fused with the N-terminus of the heavy chain. In some embodiments, the N-terminus of the second domain is fused with the C-terminus of the light chain. In some embodiments, the C-terminus of the second domain is fused with the N-terminus of the light chain.

In some embodiments, the first domain comprises a full-length antibody consisting of two heavy chains and two light chains. In some embodiments, the full-length antibody is a full-length monoclonal antibody consisting of two identical heavy chains and two identical light chains. In some embodiments, the second domain is an scfv.

It should be understood that the first and second domains are used for illustrative purposes only and are not intended to limit the relative positions of the domains of a bispecific antibody. In some cases, characteristics of the first domain may also apply to the second domain, and vice versa.

Another embodiment of the invention is an isolated polynucleotide encoding a heavy chain variable region and/or a light chain variable region of any antibody of the invention. Multiple polynucleotides encoding the same antibody of the invention, given the genetic code degeneracy or codon priority in a given expression system, are also within the scope of the invention. The polynucleotide sequence encoding the VH or VL of the antibody of the invention, or fragments thereof, can be efficiently linked to one or more regulatory elements, such as promoters or enhancers, which allow the nucleotide sequence to be expressed in the intended host cell. The polynucleotide may be cDNA.

Another embodiment of the invention is a vector comprising the polynucleotides of the invention. Such a vector may be a plasmid vector, a viral vector, a vector for baculovirus expression, a transposon-based vector, or any other vector suitable for introducing the synthetic polynucleotides of the invention into a given organism or genetic background by any means. For example, a polynucleotide optionally linked to a constant region and encoding a light chain variable region and/or a heavy chain variable region of an antibody of the invention is inserted into an expression vector. The light chain and/or heavy chain may be cloned into the same or different expression vectors. A DNA fragment encoding an immunoglobulin chain may be efficiently ligated to a control sequence in one or more expression vectors that ensures the expression of the immunoglobulin polypeptide. Such control sequences include signal sequences, promoters (e.g., naturally associated or heterologous promoters), enhancer elements, and transcription terminator sequences, which are selected to be compatible with the host cells chosen for antibody expression. Once the vector is bound to a suitable host, the host is maintained under conditions suitable for high-level expression of the protein encoded by the bound polynucleotide.

Suitable expression vectors can typically replicate in a host organism as a free gene or as part of the host's chromosomal DNA. Typically, expression vectors contain selection markers, such as ampicillin resistance, hygromycin resistance, tetracycline resistance, kanamycin resistance, or neomycin resistance, to detect cells that have been transformed with the desired DNA sequence.

Suitable promoters and enhancer elements are known in the art. For expression in bacterial cells, exemplary promoters include lacl, lacZ, T3, T7, gpt, λP, and trc. For expression in eukaryotic cells, exemplary promoters include light chain and/or heavy chain immunoglobulin gene promoters and enhancer elements; very early promoters for cytomegaloviruses; thymidine kinase promoters for herpes simplex virus; early and late SV40 promoters; promoters present in long terminal repeat sequences of retroviruses; mouse metallothionein-I promoters; and various tissue-specific promoters known in the art. For expression in yeast cells, exemplary promoters are constitutive promoters, such as the ADH1 promoter, PGK1 promoter, ENO promoter, PYK1 promoter, etc.; or regulatory promoters, such as the GAL1 promoter, GAL10 promoter, ADH2 promoter, PH05 promoter, CUP1 promoter, GAL7 promoter, MET25 promoter, MET3 promoter, CYC1 promoter, HIS3 promoter, ADH1 promoter, PGK promoter, GAPDH promoter, ADC1 promoter, TRP1 promoter, URA3 promoter, LEU2 promoter, ENO promoter, TP1 promoter, and AOX1 (e.g., for Pichia). The selection of suitable vectors and promoters is within the capabilities of those skilled in the art.

A wide range of suitable vectors and promoters are known to those skilled in the art; many are commercially available for generating recombinant constructs of subjects. The following vectors are provided by way of example. Bacterial vectors: pBs, phagescript, PsiX174, pBluescript SK, pBs KS, pNH8a, pNH16a, pNH18a, pNH46a (Stratagene, La Jolla, Calif., USA); pTrc99A, pKK223-3 , pKK233-3, pDR540, and pRIT5 (Pharmacia, Uppsala, Sweden). Eukaryotic vectors: pWLneo, pSV2cat, pOG44, PXR1, pSG (Stratagene), pSVK3, pBPV, pMSG, and pSVL (Pharmacia).

Another embodiment of the invention is a host cell comprising one or more vectors of the invention. The term "host cell" refers to a cell in which the vector has been introduced. It should be understood that the term "host cell" is intended not only to refer to a specific test cell, but also to its progeny, and to a stable cell line derived from a specific test cell. Such progeny may differ from the parent cell due to mutations or environmental influences resulting in some modifications, but are still included within the scope of the term "host cell" as used herein. Such host cells can be eukaryotic cells, prokaryotic cells, plant cells, or archaea cells.

*Escherichia coli , bacilli* (such as Bacillus *subtilis* ) and other *enterobacteriaceae* (such as *Salmonella* and *Serratia*), and various *Pseudomonas* species are examples of prokaryotic host cells. Other microorganisms such as yeast can also be used for expression. *Saccharomyces* (e.g., *Saccharomyces cerevisiae*) and *Pichia* are examples of suitable yeast host cells. Exemplary eukaryotic cells can be derived from mammalian, insect, bird, or other animal sources. Mammalian eukaryotic cells include infinitely proliferating cell lines such as hybridoma or myeloma cell lines, such as SP2/0 (American Type Culture Collection (ATCC), Manassas, VA, CRL-1581), NSO (European Collection of Authenticated Cell Cultures (ECACC), Salisbury, Wiltshire, UK, ECACC No. 85110503), FO (ATCC CRL-1646), and Ag653 (ATCC CRL-1580) murine cell lines. An exemplary human myeloma cell line is U266 (ATTC CRL-TIB-196). Other available cell lines include those derived from Chinese hamster ovary (CHO) cells, such as CHO-K1SV (Lonza Biologics, Walkersville, MD), CHO-K1 (ATCC CRL-61), or DG44.

Another embodiment of the invention is a method for preparing the antibody of the invention, comprising culturing the host cells of the invention under conditions that allow the antibody to be expressed, and then recovering the antibody produced by the host cells. Methods for preparing and purifying antibodies are well known in the art. Once synthesized (chemically or recombinantly), the entire antibody, its dimer, each of the light and/or heavy chains, or other antibody fragments (such as VH and/or VL) can be purified according to standard procedures including ammonium sulfate precipitation, affinity chromatography, column chromatography, high-performance liquid chromatography (HPLC) purification, gel electrophoresis, etc. (see generally Scopes, Protein Purification (Springer-Verlag, NY, (1982)). The antibody can be substantially pure, for example, at least about 80% to 85% pure, at least about 85% to 90% pure, at least about 90% to 95% pure, or at least about 98% to 99% pure, or even purer, for example, free from contaminants (such as cell debris, macromolecules other than the test antibody, etc.).

Another embodiment of the present invention is a method for preparing the antibody of the present invention, the method comprising:
incorporating the first polynucleotide encoding antibody VH and the second polynucleotide encoding antibody VL into the expression vector;
transforming host cells using this expression vector;
culturing host cells in a culture medium under conditions that enabled VL and VH expression and the formation of the antibody; and
recovering the antibody from host cells or culture media.

The polynucleotide encoding the specific VH or VL sequence of the present invention is incorporated into the vector using standard molecular biology methods. Host cell transformation, culture, antibody expression, and purification are performed using well-known methods.

### III. Composition and Administration

The present invention provides pharmaceutical compositions comprising the antibodies described herein, and pharmaceutically acceptable carriers. For therapeutic use, the antibodies of the present invention can be prepared into pharmaceutical compositions comprising an effective amount of the antibody as the active ingredient in a pharmaceutically acceptable carrier. The term "carrier" refers to a diluent, adjuvant, excipient, or vehicle by which the active compound is administered. Such vehicle can be liquids, such as water and oils, including those derived from petroleum, animal, or plant, or synthetic oils such as peanut oil, soybean oil, mineral oil, and sesame oil. For example, a 0.4% saline solution and 0.3% glycine can be used. These solutions are sterile and generally free of particulate matter. They can be sterilized using well-known conventional sterilization techniques, such as filtration. The compositions may contain pharmaceutically acceptable adjuvants, such as pH adjusters and buffers, stabilizers, thickeners, lubricants, and colorants, as needed, to approximate physiological conditions. The concentration of the antibody of the present invention in such pharmaceutical formulations can vary over a wide range, i.e., from less than about 0.5% by weight, typically to at least about 1% by weight, up to 15%, 20%, 25%, 30%, 35%, 40%, 45%, or 50% by weight, and will be selected based primarily on the desired dose, fluid volume, viscosity, etc., depending on the specific method of administration chosen. Suitable vehicle and formulations (including other human proteins such as human serum albumin) are described, for example, in Remington: The Science and Practice of Pharmacy, 21st edition, Troy, D.B. editor, Lipincott Williams and Wilkins, Philadelphia, PA 2006, Part 5, Pharmaceutical Manufacturing, pp. 691-1092, particularly pp. 958-989.

The administration of the antibody of the present invention in the method described herein can be by any suitable route, such as parenteral administration, for example, intradermal, intramuscular, intraperitoneal, intravenous or subcutaneous, lung, mucosal (oral, nasal, vaginal, rectal) or other methods known to those skilled in the art, as is well known in the art.

The antibodies in the method of the present invention described herein can be administered to the patient via any suitable route, such as parenteral administration via intravenous (iv) infusion or bolus, intramuscular administration, subcutaneous administration, or intraperitoneal administration. Intravenous infusion can be given over, for example, 15, 30, 60, 90, 120, 180, or 240 minutes, or over 1, 2, 3, 4, 5, 6, 7, 8, 9, 10, 11, or 12 hours.

In one embodiment, the method of the present invention described herein administers a therapeutically effective amount of the antibody of the present invention to a subject. The "therapeutically effective amount" of the antibody can be determined using standard research techniques. For example, in vitro assays can be used to help identify the optimal dose range. Optionally, the therapeutically effective amount of the antibody of the present invention can be determined by administering the antibody to a relevant animal model well known in the art. The selection of a specific effective dose can be determined by those skilled in the art based on considerations of several factors (e.g., via clinical trials). Such factors include the disease to be treated or prevented, the symptoms involved, the patient's weight, the patient's immune status, and other factors known to those skilled in the art. The precise dose to be used in the formulation will also depend on the route of administration and the severity of the disease, and should be determined based on the physician's judgment and the individual patient's situation. The effective dose can be derived from dose-response curves from in vitro or animal model testing systems. Any of the models described herein can be used to test the efficacy and effective dose of the antibody of the present invention.

The antibody in the method of this invention can be repeatedly administered after one day, two days, three days, four days, five days, six days, one week, two weeks, three weeks, one month, five weeks, six weeks, seven weeks, two months, three months, four months, five months, six months, or longer periods. Treatment courses can also be repeated, similarly to chronic administration. Repeated administration can be at the same dose or different doses.

The antibodies in the method of the present invention can also be administered prophylactically to reduce the risk of HBV infection in subjects, delay the onset of HBV infection, and/or reduce the risk of relapse of HBV infection in remission.

The antibodies in the method of the present invention described herein can be lyophilized and stored, and then reconstituted in a suitable carrier before use. This technique has been proven effective for conventional protein formulations and can be employed using well-known lyophilization and reconstitution techniques.

In one embodiment, the pharmaceutical composition of this disclosure comprises one or more antibodies described herein. Those skilled in the art will readily understand that when the HBV infection is caused by multiple mutations, or when the body develops multiple mutations under drug or immune stress, a combination of one or more antibodies binding to different mutation sites will have greater clinical significance and value in both treatment and prevention.

In one embodiment, the pharmaceutical composition of this disclosure further comprises one or more second agents or antibodies. The combined use of multiple agents or antibodies is well known in the art and readily implemented by those skilled in the art based on conventional practice.

The present invention will now be described with reference to the following specific non-limiting examples, which are for illustrative purposes only and are not intended to limit the scope of the invention.

### Materials and Methods

The inventors isolated hepatitis B surface antibody-specific B cells from peripheral blood mononuclear cells (PBMCs) of recovered hepatitis B patients using flow cytometry. Subsequently, the antibody sequence within the cells was cloned using PCR, and the antibody sequence was obtained through sequencing and constructed into an expression vector. The plasmid was then introduced into the cells via transient transfection for protein expression. The cell supernatant was then collected to purify and concentrate the antibody.

All human subjects involved in the present invention were approved by the Ethics Committee of Fudan University (Project Ethics No.: 2020-C013), and experiments involving all donor samples were conducted at the School of Basic Medical Sciences, Fudan University. The research subjects included 325 recovered hepatitis B patient donors and 16 post-hepatitis B vaccination high-titer anti-HBs donors. Serum samples used for screening volunteers were heat-inactivated at 56°C for 60 minutes after collection and then aliquoted and stored at -80°C. 200 ml of blood was drawn from recovered hepatitis B patient donors (AA14, BH2, CC7, and EB4) and hepatitis B vaccine recipient donors (C10, C12, and C14). Human peripheral blood mononuclear cells (PBMCs) were isolated and then resuspended in a cryopreservation solution of 10% DMSO and 90% fetal bovine serum (inactivated), and then frozen in liquid nitrogen.

B cell purification, bait protein-binding B cell dual-fluorescent dye labeling, and single-cell sorting were described previously (Y. Zhou et al. (2020) STAR Protoc 1, 100129). PBMCs were rapidly thawed in a 37°C water bath and washed with RPMI medium. PBMCs were then incubated with CD19 microbeads for positive selection of B lymphocytes. Cell surface molecules were blocked with human Fc block at 4°C, followed by continuous incubation with bait protein-PE/APC (10ug/ml) and anti-CD20-PE-Cy7 (BD Bioscience). Single-cell sorting was performed using a FACSAria II (BD Bioscience) flow cytometer, and single cells were sorted into 96-well plates and stored at -80°C.

As mentioned earlier (Y. Zhou et al (2020) STAR Protoc 1, 100129), sorted single cells were subjected to reverse transcription and antibody sequencing PCR. After a first round of PCR and a second round of nested PCR, electrophoresis and purification were performed on a 2% agarose gel, followed by Sanger sequencing. The heavy and light chain sequences were analyzed using IMGT/V-QUEST (Brochet, X. et al (2008) Nucl. Acids Res. 36, W503-508) and IgBlast (J. Ye et al (2013) Nucl. Acids Res. 41, W34-40), and the V(D)J gene segment and CDR3 sequence of each antibody were determined. The selected antibodies were then used for vector construction and antibody expression.

The antibodies obtained in the present invention were used for ELISA detection. HBsAg antigen was diluted to 10 µg/ml with PBS and coating was performed overnight at 4°C. Blocking was then performed for 2 hours with 2% bovine serum albumin (BSA) dissolved in PBS. The antibody was diluted eight times at a 1:3 ratio, with 10 ug/ml as the maximum concentration, and incubated at room temperature for 1 hour after addition. Incubation was then performed with HRP-conjugated goat anti-human IgG (Thermo Fisher Scientific), followed by ABTS chromogenic reaction. Readings were taken at 10, 20, and 30 minutes using a BioRad plate reader. Analysis was performed using PRISM software, and the area under the curve (AUC) of each antibody was calculated to assess antigen-binding capability.

The detection of antibody competition using a competitive ELISA has been previously reported (Q. Wang et al (2020) Cell Host & Microbe 28, 335-349). In brief, HBsAg antigen was diluted to 3 µg/ml with PBS and coating was performed overnight. The primary blocking antibody concentration was 15 µg/ml, and incubation was performed at room temperature for 2 hours. Subsequently, a biotinylated secondary antibody (0.25 µg/ml or 0.75 µg/ml) was added directly at room temperature, and incubation was continued for 30 minutes. Detection was performed using streptavidin-HRP (BD Bioscience). PBS buffer was used as a normalization reference instead of the primary blocking antibody.

Neutralization experiments were conducted using a cell model infected with HBV in vitro (Q. Wang et al (2020) Cell Host & Microbe 28, 335-349). HBV virus was prepared in two ways: one using HBV virus derived from the HepDE19 cell line with genotype D; the other using X-tremeGENE HP (Roche) transfection reagent to transfect HBV plasmids with different genotypes HepG2 cell lines to express HBV genotypes A/B/C. Both viruses, whether from stable cell lines or obtained through transfection, were concentrated using ultrafiltration. After ultrafiltration, the obtained viruses were aliquoted, and a small amount was serially diluted 10-fold and HBV copy number was quantified by qPCR (Sansure Biotech) (F. Shen et al (2018) Hepatology 67, 1237-1252). HepG2-NTCP cells were seeded in 96-well plates and treated with DMEM medium containing 2% DMSO, 3% FBS, 1% NEAA, and 1% penicillin/streptomycin (infection medium) for 24 hours before infection. To test the neutralizing capacity of serum or antibodies, human serum (maximum concentration 10%) or monoclonal antibody (maximum concentration 10 µg/ml) was serially diluted in the infection medium at a 1:3 ratio, a total of nine dilutions. The diluted serum or monoclonal antibody was then incubated with HBV virus at 37°C for 30 minutes before being added to HepG2-NTCP cells. Sixteen hours after infection, each well was washed five times with 200 µL of PBS and then the infection medium was added. After 7 days of culture, the levels of HBsAg and HBeAg in the cell supernatant were detected using a CLIA kit (Autobio), and the level of HBcAg in the cells was detected by immunofluorescence.

In the present invention, the neutralizing effect of antibodies against different hepatitis B virus mutants was detected using HBV mutant viruses obtained by transfecting HepG2 cells with plasmids (pHBV1.3-G145R, pHBV1.3-K122R G145R, and pHBV1.3-F134L) and collecting and concentrating the supernatant. The neutralization experiment procedure was the same as that described above for neutralization experiments using the cell model infected with HBV virus in vitro.

The experimental methods for amino acid epitope screening of antibody (amino acid scanning), ELISA with antibody binding to different viral genotypes, and antibody binding to different viral mutants were as described previously (Q. Wang et al (2020) Cell Host & Microbe 28, 335-349). In brief, Huh7.5 cells were seeded in 6-well plates, and transfected with HBV1.3 plasmids with different mutation sites using Lipofectamine 3000 (Thermo Fisher Scientific). Sixteen hours after transfection, the cell culture medium was replaced with DMEM medium containing a mixture of 1% NEAA and 1% penicillin-streptomycin. After 3 days of culture, the cell culture supernatant was collected, centrifuged to remove cell debris, and then incubated in 100 µL per well of a 96-well ELISA plate at 4°C overnight. Blocking was then performed with 2% bovine serum albumin (BSA) dissolved in PBS for 2 hours. Incubation was performed with antibody at a concentration of 1 µg/ml, followed by incubation with HRP-conjugated goat anti-human IgG (Thermo Fisher Scientific), followed by ABTS chromogenic reaction. Readings were taken at 10, 20, and 30 minutes using a BioRad plate reader. The reading with antibody binding to wild-type HBV was used as a normalization reference, and analysis was performed using PRISM software.

For immunofluorescence, cells were fixed in 4% paraformaldehyde/PBS at room temperature for 20 minutes, neutralized in PBS containing 0.1M glycine for 15 minutes, and then 0.1% Triton X-100/PBS. Blocking was performed with 5% goat serum/PBS at room temperature for 1 hour. Anti-HBc (Austral BIologicals) was diluted with 5% goat serum/PBS and incubated overnight at 4°C. Cells were visualized using a goat anti-rabbit Alexa Fluor 594 (thermo Fisher Scientific), and cell nuclei were stained with DAPI. Cell fluorescence imaging was performed using a high-content cell imaging analyzer (Perkin Elmer, Operetta).

The detailed results of the statistical analysis are shown in the results and legend sections. The area under the ELISA curve (AUC) and the 50% inhibitory concentration (IC₅₀) values for antibody neutralization capability were calculated using PRISM software.

### Example 1. Antibody response in the serum of recovered hepatitis B individuals

Serum samples were collected from 325 HBV-infected but recovered donors from multiple hospitals in Shanghai. All enrolled donors were positive for hepatitis B core antibody in their hepatitis B serology panel results, with varying titers of hepatitis B surface antibody. Neutralizing activity was measured in the serum of recovered HBV patients. The ability of donor serum samples to block HBV infection of HepG2-NTCP cells was measured in HepG2 cells overexpressing the HBV receptor protein sodium ion- taurocholic acid cotransporter (NTCP), i.e., neutralizing activity. We selected donors AA14, BH2, CC7, and EB4 with high anti-HBs titers and high neutralizing activity from recovered HBV patients, and C10, C12, and C14 from HBV-vaccinated individuals. Donors with anti-HBs titers <2.0 IU/ml were used as negative controls. Demographic information of the donors is shown in Table 3. We confirmed the neutralizing activity of donors at different serum concentrations by diluting the serum samples, as shown in Figure 2.

**Table 3. Demographic information of donors**

| serial number | gender | age | nationality | Hepatitis B surface antigen | Hepatitis B surface antibody (mIU/ml) | Hepatitis B core antibody | Hepatitis B e antigen | Hepatitis B e antibody (S/CO) |
|---|---|---|---|---|---|---|---|---|
| AA14 | male | 36 years old | Chinese | Negative | 890 | Positive | Negative | 0.002 |
| BH2 | male | 36 years old | Chinese | Negative | >1000 | Positive | Negative | 0.86 |
| CC7 | male | 38 years old | Chinese | Negative | >1000 | Positive | Negative | 1.38 |
| EB4 | male | 37 years old | Chinese | Negative | >1000 | Positive | Negative | 1.6 |
| V2 | male | 27 years old | Chinese | Negative | <2.0 | Negative | Negative | Negative |
| C10 | female | 23 years old | Chinese | Negative | >1000 | Negative | Negative | Negative |
| C12 | female | 25 years old | Chinese | Negative | >1000 | Negative | Negative | Negative |
| C14 | male | 23 years old | Chinese | Negative | >1000 | Negative | Negative | Negative |

### Example 2. The production of a fully human monoclonal antibody that specifically binds to HBsAg

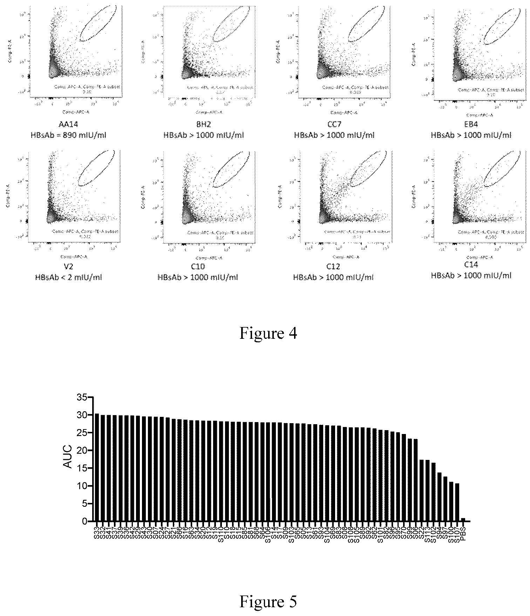

HBV surface protein is a four-transmembrane protein, which can be divided into three regions: preS1, preS2, and S. The antibodies cloned in the present invention mainly target the S region, using CHO-purified HBsAg protein as the bait protein. To select B cells expressing HBsAg-specific IgG antibodies in individuals, a dual-fluorescent dye labeling strategy was used to identify HBsAg-specifically binding B cells (Figure 3). Hepatitis B vaccine-treated donors with anti-HBs ELISA titers less than 2 mIU/ml were recruited as negative controls, with B cells stained as bait protein-specific B cells at the background level, while donors (AA14, BH2, CC7, EB4) and vaccine-immunized donors (C10, C12, C14) with high serum neutralizing activity showed bait protein-specific B cells (Figure 4).

Flow cytometry-gated double-positive cells (bait protein-PE+ and bait protein-APC+) were sorted into single cells, and then the heavy and light chains of specific B cells were amplified by nested PCR using specific primers for immunoglobulin heavy and light chain genes after cell lysis and RNA reverse transcription of individual B cells.

### Example 3. ELISA detection of antibody binding to HBsAg protein

After obtaining the antibody sequence, it was cloned into a vector, followed by protein expression and purification. The resulting antibody was then subjected to ELISA to detect its binding to HBsAg. The AUC values were calculated using PRISM based on the ELISA data. Among the antibodies numbered S001-S108, 62 antibodies were found to bind to HBsAg, and they are presented in descending order of AUC value (see Figure 5).

### Example 4. Evaluation of antibody neutralizing activity using an HBV cell infection model

To determine whether antibodies that bind to HBsAg can block HBV infection in vitro, HepG2-NTCP cells were infected with concentrated and purified HBV virus (genotype D) derived from cell sources for neutralization assay. During the experiment, antibodies were serially diluted and then mixed with the same virus to infect HepG2-NTCP cells. Finally, the levels of HBsAg and HBeAg in the cell supernatant were measured. The infection rate was compared between the antibody-treated group and no antibody-treated group, and the 50% inhibitory concentration (IC₅₀) of the antibody was calculated. The neutralizing activities of these antibodies varied significantly, ranging from effective neutralizing antibodies to almost no neutralizing activity (Figure 6). Specific IC50 values are shown in Table 4.

**Table 4. IC₅₀ values of antibodies in cell infection models**

| **Ab No.** | HBsAg | HBeAg |
|---|---|---|
| S05 | 5.22 | 39.47 |
| S06 | > 500 | > 500 |
| S07 | 51.5 | 291.8 |
| S08 | 272.1 | 237.7 |
| S09 | > 500 | > 500 |
| S10 | 141.1 | 28.47 |
| S12 | 6.24 | 8.79 |
| S13 | 113 | 602 |
| S14 | 132.4 | 37.7 |
| S15 | 29.35 | 48.73 |
| S16 | 5.84 | 55.31 |
| S17 | 4.45 | 12.54 |
| S18 | > 500 | > 500 |
| S19 | 2.76 | 5.2 |
| S20 | 1.65 | 3.32 |
| S21 | > 500 | > 500 |
| S22 | 50.27 | 381.9 |
| S24 | 81.9 | 120.4 |
| S27 | > 500 | > 500 |
| S29 | 8 | 14.57 |
| S30 | > 500 | > 500 |
| S32 | 106.5 | 274.7 |
| S33 | 6.68 | 7.84 |
| S34 | 466.5 | > 500 |
| S36 | 35.69 | 365.2 |
| S37 | 423.6 | > 500 |
| S39 | 12.76 | 19.81 |
| S41 | 5 | 5.12 |
| S42 | > 1000 | > 1000 |
| S43 | 5.78 | 13.18 |
| S61 | 27.22 | 88.01 |
| S62 | 189.9 | > 500 |
| S63 | > 500 | > 500 |
| S64 | 16.05 | 14.43 |
| S65 | 178.8 | > 500 |
| S66 | 5.1 | 5.34 |
| S68 | > 500 | > 500 |
| S69 | > 500 | > 500 |
| S70 | > 500 | > 500 |
| S81 | 4.08 | 14.27 |
| S82 | 27.62 | 136.7 |
| S83 | > 500 | > 500 |
| S85 | > 500 | > 500 |
| S89 | 2.1 | 7 |
| S92 | 9.51 | 29.31 |
| S93 | 4.24 | 5.62 |
| S94 | > 500 | > 500 |
| S95 | 2.35 | 6.7 |
| S96 | 8.89 | 19.17 |
| S97 | > 500 | > 500 |
| S99 | 291.6 | > 500 |
| S100 | > 500 | > 500 |
| S101 | 10.01 | 11.89 |
| S102 | 297.1 | 227.6 |
| S103 | 7.56 | 52.43 |
| S104 | > 500 | > 500 |
| S105 | 38.54 | 119.1 |
| S106 | 3.32 | 8.6 |
| S107 | > 500 | > 500 |
| S108 | > 500 | > 500 |
| S110 | > 500 | > 500 |
| S113 | > 500 | > 500 |

### Example 5. Antibody Epitope Analysis

For the 23 antibodies with relatively good neutralizing activity (S05, S12, S16, S17, S19, S20, S29, S33, S39, S41, S43, S64, S66, S81, S82, S89, S92, S95, S96, S101, S103, S105, and S106), their binding epitopes were characterized. First, it was determined whether the HBsAg-binding antibodies bound overlapping or non-overlapping epitopes, and a competitive ELISA was performed. Since the binding of antibody S105 was weak, its competitive ELISA results are not shown here. Three mutually exclusive antibody groups (Group I, Group II, and Group III) were identified by competitive ELISA, as shown in Figure 7. Group I included S33, S39, S64, S103, and S106; Group II included S29, S43, S41, S66, and S81, and Group III included S5, S12, S16, S17, S19, S20, S82, S89, S92, S95, S96, and S101.

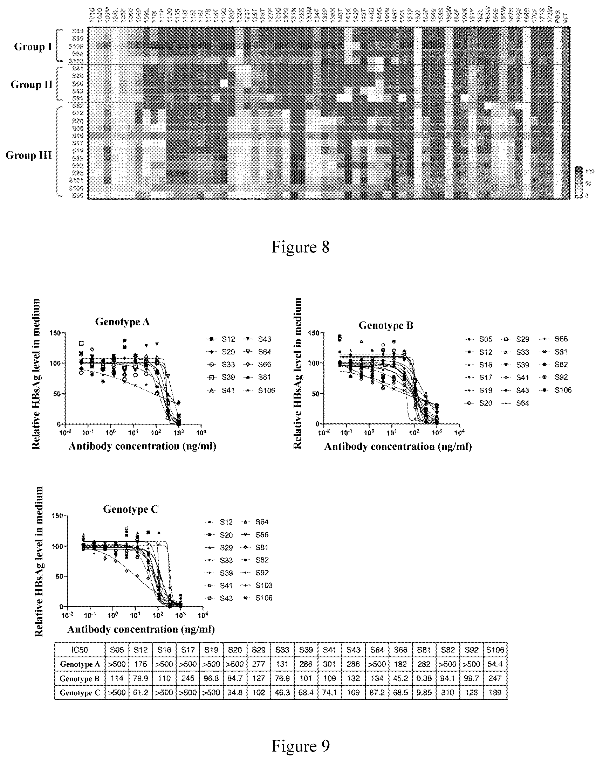

Antibody recognition of the S protein primarily targets a region of approximately 72 amino acids in the extracellular domain of HBsAg. Further alanine scanning was performed. Cells were transfected using a pre-constructed HBsAg point-mutant plasmid (original amino acid mutated to alanine) to provide HBsAg with different amino acid mutations. ELISA was then used to detect whether the antibody could bind to the HBsAg amino acid mutants, thus clarifying the specific antigenic amino acid site recognized by the obtained antibody (Figure 8). Combined with the results of competitive ELISA, it was found that different groups of antibodies exhibited significant clustering at key antigenic amino acid recognition sites. Antibodies derived from hepatitis B vaccination donors and antibodies derived from recovered hepatitis B patients showed significant differences in recognition sites. The average number of amino acids affecting antibodies derived from hepatitis B vaccination was 12.6, while the average number affecting antibodies derived from recovered hepatitis B patients was 22.1. The amino acid sites recognized by antibodies from hepatitis B vaccination donors are discontinuous, while those recognized by recovered hepatitis B donors can be continuous, consisting of 3-5 amino acids. For example, S43 recognizes 143T-145G, S20 recognizes 133M-136S, and S17 recognizes 142P-146N. Subsequently, combined with the results of competitive ELISA, the alanine scanning results of antibody were analyzed. All four groups of antibodies recognized spatial epitopes of the S protein; 101Q, 102G, 103M, 105P, and 106V all influenced the binding of each antibody. The antibodies in the four groups exhibited their own specific amino acid mutant binding patterns. Group I recognized the relevant amino acid sites 110I, 122K, 125T, 134F, 141K, and 165W; Group II recognized the relevant amino acid sites 122K, 123T, 145G, 161Y, and 165W; and Group III mainly recognized the relevant amino acid sites 109L-111P, 120P-130G, 133M-136S, 144D, 146N, 153P, 161Y, and 164E-167S. In Group I, antibodies S64, and S33, S39 were derived from two different hepatitis B vaccination donors, repectively, while in Group II, antibodies S66, and S29, S41 were derived from two different hepatitis B vaccination donors repectively, suggesting that while antibodies obtained from hepatitis B vaccination in different individuals have some similarity, the resulting antibodies exhibit diversity in epitope, recognizing at least two non-overlapping epitopes.

### Example 6. Binding capability and neutralizing activity against different HBV genotypes

Hepatitis B virus (HBV) is an ancient DNA virus with nine confirmed genotypes, plus one undetermined genotype. Numerous studies have shown that HBV genotype distribution varies across different regions. In China, approximately 60% of HBV-infected individuals have genotype B, and approximately 30% have genotype C.

The inventor's laboratory has successfully established an in vitro infection system for HepG2-NTCP cells infected with different HBV genotypes. This system was used to test whether antibodies broadly binding to different HBV genotypes could block infection by these genotypes in vitro (Figure 9). After mixing the antibody and virus in vitro, the mixture was incubated at 37°C for 1 hour, and then used to infect HepG2-NTCP cells. On day 7 post-infection, the levels of HBsAg and HBeAg in the cell supernatant were measured. The 50% inhibitory concentration (IC50) of the antibody was calculated by antibody-treated group vs. no antibody-treated group.

The results showed that S12, S29, S33, S39, S41, S43, S66, S81, and S106 exhibited neutralizing activity against all four genotypes (A, B, C, and D) (see Figure 9 and Table 4). Antibodies derived from recovered hepatitis B donors generally did not neutralize genotype A virus well, while most antibodies derived from hepatitis B vaccination donors showed neutralizing activity against all four genotypes of HBV virus.

### Example 7. Binding capability against naturally occurring HBV mutant strains

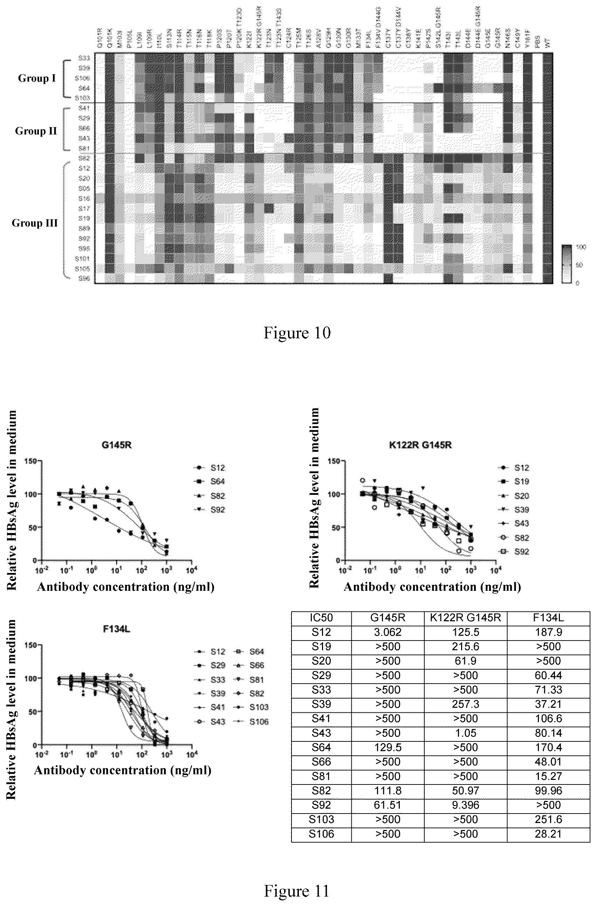

Previous studies have reported numerous immune escapes in patients with chronic hepatitis B infection. To test whether antibodies obtained from recovered hepatitis B donors or hepatitis B vaccination donors can bind to these mutant strains, amino acid mutant plasmids of common hepatitis B surface antigens were constructed. The escape mutations selected in the present invention include: Q101R, Q101K, M103I, P105L, L109I, L109R, I110L, S113N, T114R, T115N, T116N, T118K, P120S, P120T, P120K-T123D, K122I, K122R-G145R, T123N, T123N-T143S, C124R, T125M, T126S, A128V, Q129H, G130N, G130R, M133T, F134L, F134V-D144G, C137Y, C137Y-D144V, C138Y, K141E, P142S, P142L-G145R, T143I, T143L, D144E, D144E-G145R, G145E, G145R, N146S, C149Y, and Y161F. Cells were transfected with these plasmids, and the cell supernatant contained the antigens containing the HBsAg mutants. ELISA was then used to detect whether antibodies bound to the antigens (Figure 10). Antibodies from hepatitis B vaccination donors recognized an average of 31 mutation sites, while antibodies from recovered hepatitis B donors recognized an average of 37.4 mutation sites. The specific HBsAg mutant sites recognized by antibodies from these two immune backgrounds also showed significant differences. Group I antibodies derived from hepatitis B vaccination donors do not recognize Q101R, P105L, C124R, C137Y, C137Y D144V, C138Y, K141E, C149Y, while S64 in Group I can recognize K122R G145R, P142S, S142L G145R, D144E G145R, G145E, and G145R sites. Group II antibodies, primarily derived from hepatitis B vaccination donors, do not recognize Q101R, P105L, P120K T123D, K122R G145R, T123N, T123N T143S, C124R, C137Y, C137Y D144V, C138Y, S142L G145R, D144E G145R, G145E, G145R, while S43 in Group II derived from recovered hepatitis B donors cannot recognize the above mutation sites, nor T143I, T143L, and D144E. Group III antibodies from recovered hepatitis B donors cannot recognize Q101R, P105L, P120K T123D, T123N, T123N T143S, G130N, G130R, M133T, C138Y, and C149Y, but can recognize C137Y, C137Y D144Y sites, which are not recognized in Groups I and II. The most common mutation site clinically is G145R. ELISA results show that only S64 antibodies from hepatitis B vaccination donors can recognize G145R.

### Example 8. Neutralizing activity against naturally occurring HBV mutants

As shown in the ELISA results of Example 6, the present invention relates to the binding of neutralizing antibodies to different HBV mutant strains. Here, we selected the most clinically common HBsAg G145R single-site mutant strain and the HBsAg K122R-G145R double-mutant strain for neutralization experiments. Example 6 indicates that antibodies derived from hepatitis B vaccination donors can bind better to the HBsAg F134L mutant; the HBsAg F134L mutant virus was also purified here.

The experimental results indicate that antibodies S12, S64, S82, and S92 can effectively neutralize the G145R mutant HBV virus; S12, S19, S20, and S43 from recovered hepatitis B donors and S39, S82, and S92 from hepatitis B vaccination donors can effectively neutralize the K122R-G145R double mutant HBV virus; and S12, S43, S103, and S106 from recovered hepatitis B donors and S29, S33, S39, S41, S64, S66, S81, and S82 from hepatitis B vaccination donors can effectively neutralize the F134L mutant HBV virus (Figure 11).

### Example 9. Construction and analysis of bispecific antibodies

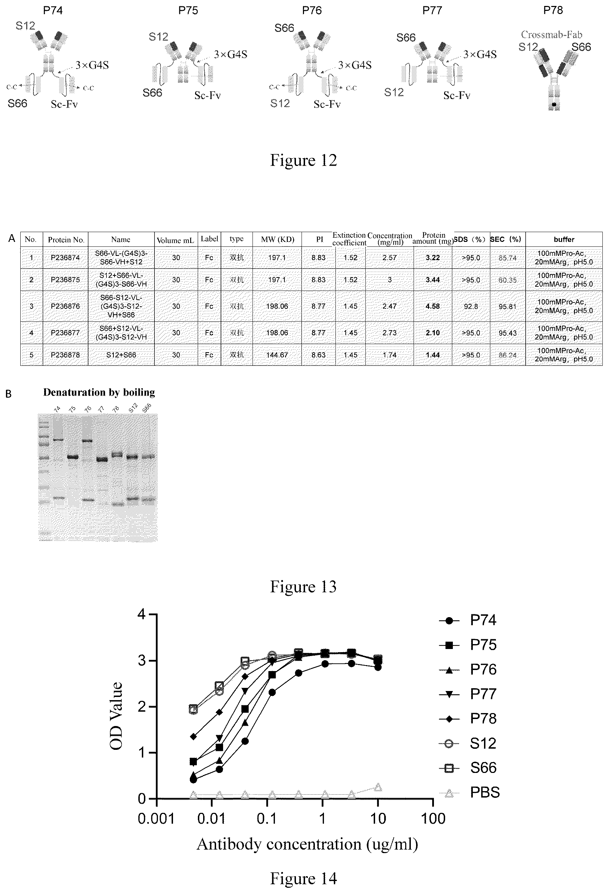

In this example, S66 and S12 antibodies were selected as parental antibody sources for the design and modification of bispecific antibodies, resulting in five constructs, as shown in Figure 12. The preparation was completed by Sanyou Biopharmaceutical (Shanghai) Co., Ltd., which also provided the physicochemical properties and SDS-PAGE results of the bispecific antibodies, as shown in Figure 13.

Following the method in Example 3, the bispecific antibodies were subjected to ELISA to detect antibody binding to HBsAg. All constructed bispecific antibodies, P74, P75, P76, P77, and P78, could bind to HBsAg, with binding affinity similar to that of the parent antibodies S66 and S12, as shown in Figure 14.

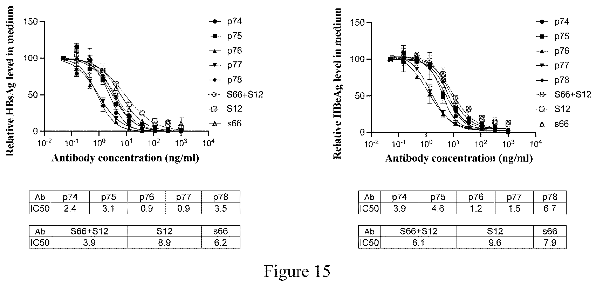

To determine whether a bispecific antibody that can bind to HBsAg can block HBV infection in vitro, neutralization was assessed by infecting HepG2-NTCP cells with concentrated and purified HBV virus (genotype D) derived from cell sources, following the method in Example 4. The antibody was serially diluted and then mixed with the same virus to infect HepG2-NTCP cells. Finally, the levels of HBsAg and HBeAg in the cell supernatant were measured. The infection rate was compared between antibody-treated group and no antibody-treated group, and the 50% inhibitory concentration (IC50) of the antibody was calculated. The results are shown in Figure 15.

The results showed that all the bispecific antibodies P74, P75, P76, P77 and P78 constructed were superior to the parental antibodies S66 and S12 in blocking HBV infection, in which p76 and p77 had IC50 values that were about 1 log lower than the two parental monoclonal antibodies.

Based on the antibody epitope analysis in Example 5, various bispecific antibodies were further constructed from monoclonal antibodies of group I: S33, S39, S64, S103 and S106, group II: S29, S43, S41, S66 and S81, and group III: S5, S12, S16, S17, S19, S20, S82, S89, S92, S95, S96 and S101. These antibodies yielded similar results to the S66/S12 bispecific antibody (data not shown).

### References

1. Hehle V, Beretta M, Bourgine M, Ait-Goughoulte M, Planchais C, Morisse S, Vesin B, et al. Potent human broadly neutralizing antibodies to hepatitis B virus from natural controllers. J Exp Med 2020;217.
2. Wang Q, Michailidis E, Yu YP, Wang ZJ, Hurley AM, Oren DA, Mayer CT, et al. A Combination of Human Broadly Neutralizing Antibodies against Hepatitis B Virus HBsAg with Distinct Epitopes Suppresses Escape Mutations. Cell Host & Microbe 2020;28:335-+.
3. Huang CH, Yuan Q, Chen PJ, Zhang YL, Chen CR, Zheng QB, Yeh SH, et al. Influence of mutations in hepatitis B virus surface protein on viral antigenicity and phenotype in occult HBV strains from blood donors. J Hepatol 2012;57:720-729.
4. Ito K, Qin Y, Guarnieri M, Garcia T, Kwei K, Mizokami M, Zhang J, et al. Impairment of hepatitis B virus virion secretion by single-amino-acid substitutions in the small envelope protein and rescue by a novel glycosylation site. J Virol 2010;84:12850-12861.
5. Carman WF, Zanetti AR, Karayiannis P, Waters J, Manzillo G, Tanzi E, Zuckerman AJ, et al. Vaccine-induced escape mutant of hepatitis B virus. Lancet 1990;336:325-329.
6. Zhang M, Ge G, Yang Y, Cai X, Fu Q, Cai J, Huang Z. Decreased antigenicity profiles of immune-escaped and drug-resistant hepatitis B surface antigen (HBsAg) double mutants. Virol J 2013;10:292.
7. Zanetti AR, Tanzi E, Manzillo G, Maio G, Sbreglia C, Caporaso N, Thomas H, et al. Hepatitis B variant in Europe. Lancet 1988;2:1132-1133.
8. Roltgen K, Nielsen SCA, Silva O, Younes SF, Zaslavsky M, Costales C, Yang F, et al. Immune imprinting, breadth of variant recognition, and germinal center response in human SARS-CoV-2 infection and vaccination. Cell 2022;185:1025-1040 e1014.

## Claims

1. An isolated antibody or antigen-binding fragment thereof, said isolated antibody or antigen-binding fragment specifically binding to hepatitis B surface antigen (HBsAg), **characterized in that** said isolated antibody or antigen-binding fragment comprises a heavy chain variable region VH and a light chain variable region VL, said heavy chain variable region VH comprising complementarity-determining regions CDRH1, CDRH2, and CDRH3, said light chain variable region VL comprising complementarity-determining regions CDRL1, CDRL2, and CDRL3, wherein CDRH1, CDRH2, CDRH3, CDRL1, CDRL2, and CDRL3 are selected from the following amino acid sequences:
a) CDRH1 of SEQ ID NO:2, CDRH2 of SEQ ID NO:3, CDRH3 of SEQ ID NO:4, CDRL1 of SEQ ID NO:6, CDRL2 of SEQ ID NO:7 and CDRL3 of SEQ ID NO:8;
b) CDRH1 of SEQ ID NO:10, CDRH2 of SEQ ID NO:11, CDRH3 of SEQ ID NO:12, CDRL1 of SEQ ID NO:14, CDRL2 of SEQ ID NO:15 and CDRL3 of SEQ ID NO:16;
c) CDRH1 of SEQ ID NO:18, CDRH2 of SEQ ID NO:19, CDRH3 of SEQ ID NO:20, CDRL1 of SEQ ID NO:22, CDRL2 of SEQ ID NO:23 and CDRL3 of SEQ ID NO:24;
d) CDRH1 of SEQ ID NO:26, CDRH2 of SEQ ID NO:27, CDRH3 of SEQ ID NO:28, CDRL1 of SEQ ID NO:30, CDRL2 of SEQ ID NO:31 and CDRL3 of SEQ ID NO:32;
e) CDRH1 of SEQ ID NO:34, CDRH2 of SEQ ID NO:35, CDRH3 of SEQ ID NO:36, CDRL1 of SEQ ID NO:38, CDRL2 of SEQ ID NO:39 and CDRL3 of SEQ ID NO:40;
f) CDRH1 of SEQ ID NO:42, CDRH2 of SEQ ID NO:43, CDRH3 of SEQ ID NO:44, CDRL1 of SEQ ID NO:46, CDRL2 of SEQ ID NO:47 and CDRL3 of SEQ ID NO:48;
g) CDRH1 of SEQ ID NO:50, CDRH2 of SEQ ID NO:51, CDRH3 of SEQ ID NO:52, CDRL1 of SEQ ID NO:54, CDRL2 of SEQ ID NO:55 and CDRL3 of SEQ ID NO:56;
h) CDRH1 of SEQ ID NO:58, CDRH2 of SEQ ID NO:59, CDRH3 of SEQ ID NO:60, CDRL1 of SEQ ID NO:62, CDRL2 of SEQ ID NO:63 and CDRL3 of SEQ ID NO:64;
i) CDRH1 of SEQ ID NO:66, CDRH2 of SEQ ID NO:67, CDRH3 of SEQ ID NO:68, CDRL1 of SEQ ID NO:70, CDRL2 of SEQ ID NO:71 and CDRL3 of SEQ ID NO:72;
j) CDRH1 of SEQ ID NO:74, CDRH2 of SEQ ID NO:75, CDRH3 of SEQ ID NO:76, CDRL1 of SEQ ID NO:78, CDRL2 of SEQ ID NO:79 and CDRL3 of SEQ ID NO:80;
k) CDRH1 of SEQ ID NO:82, CDRH2 of SEQ ID NO:83, CDRH3 of SEQ ID NO:84, CDRL1 of SEQ ID NO:86, CDRL2 of SEQ ID NO:87 and CDRL3 of SEQ ID NO:88;
l) CDRH1 of SEQ ID NO:90, CDRH2 of SEQ ID NO:91, CDRH3 of SEQ ID NO:92, CDRL1 of SEQ ID NO:94, CDRL2 of SEQ ID NO:95 and CDRL3 of SEQ ID NO:96;
m) CDRH1 of SEQ ID NO:98, CDRH2 of SEQ ID NO:99, CDRH3 of SEQ ID NO:100, CDRL1 of SEQ ID NO:102, CDRL2 of SEQ ID NO:103 and CDRL3 of SEQ ID NO:104;
n) CDRH1 of SEQ ID NO:106, CDRH2 of SEQ ID NO:107, CDRH3 of SEQ ID NO:108, CDRL1 of SEQ ID NO:110, CDRL2 of SEQ ID NO:111 and CDRL3 of SEQ ID NO:112;
o) CDRH1 of SEQ ID NO:114, CDRH2 of SEQ ID NO:115, CDRH3 of SEQ ID NO:116, CDRL1 of SEQ ID NO:118, CDRL2 of SEQ ID NO:119 and CDRL3 of SEQ ID NO:120;
p) CDRH1 of SEQ ID NO:122, CDRH2 of SEQ ID NO:123, CDRH3 of SEQ ID NO:124, CDRL1 of SEQ ID NO:126, CDRL2 of SEQ ID NO:127 and CDRL3 of SEQ ID NO:128;
q) CDRH1 of SEQ ID NO:130, CDRH2 of SEQ ID NO:131, CDRH3 of SEQ ID NO:132, CDRL1 of SEQ ID NO:134, CDRL2 of SEQ ID NO:135 and CDRL3 of SEQ ID NO:136;
r) CDRH1 of SEQ ID NO:138, CDRH2 of SEQ ID NO:139, CDRH3 of SEQ ID NO:140, CDRL1 of SEQ ID NO:142, CDRL2 of SEQ ID NO:143 and CDRL3 of SEQ ID NO:144;
s) CDRH1 of SEQ ID NO:146, CDRH2 of SEQ ID NO:147, CDRH3 of SEQ ID NO:148, CDRL1 of SEQ ID NO:150, CDRL2 of SEQ ID NO:151 and CDRL3 of SEQ ID NO:152;
t) CDRH1 of SEQ ID NO:154, CDRH2 of SEQ ID NO:155, CDRH3 of SEQ ID NO:156, CDRL1 of SEQ ID NO:158, CDRL2 of SEQ ID NO:159 and CDRL3 of SEQ ID NO:160;
u) CDRH1 of SEQ ID NO:162, CDRH2 of SEQ ID NO:163, CDRH3 of SEQ ID NO:164, CDRL1 of SEQ ID NO:166, CDRL2 of SEQ ID NO:167 and CDRL3 of SEQ ID NO:168;
v) CDRH1 of SEQ ID NO:170, CDRH2 of SEQ ID NO:171, CDRH3 of SEQ ID NO:172, CDRL1 of SEQ ID NO:174, CDRL2 of SEQ ID NO: 175 and CDRL3 of SEQ ID NO:176;
w) CDRH1 of SEQ ID NO:178, CDRH2 of SEQ ID NO:179, CDRH3 of SEQ ID NO:180, CDRL1 of SEQ ID NO:182, CDRL2 of SEQ ID NO:183 and CDRL3 of SEQ ID NO:184;
x) CDRH1 of SEQ ID NO:186, CDRH2 of SEQ ID NO:187, CDRH3 of SEQ ID NO:188, CDRL1 of SEQ ID NO:190, CDRL2 of SEQ ID NO:191 and CDRL3 of SEQ ID NO:192;
y) CDRH1 of SEQ ID NO:194, CDRH2 of SEQ ID NO:195, CDRH3 of SEQ ID NO:196, CDRL1 of SEQ ID NO:198, CDRL2 of SEQ ID NO:199 and CDRL3 of SEQ ID NO:200;
z) CDRH1 of SEQ ID NO:202, CDRH2 of SEQ ID NO:203, CDRH3 of SEQ ID NO:204, CDRL1 of SEQ ID NO:206, CDRL2 of SEQ ID NO:207 and CDRL3 of SEQ ID NO:208;
aa) CDRH1 of SEQ ID NO:210, CDRH2 of SEQ ID NO:211, CDRH3 of SEQ ID NO:212, CDRL1 of SEQ ID NO:214, CDRL2 of SEQ ID NO:215 and CDRL3 of SEQ ID NO:216;
bb) CDRH1 of SEQ ID NO:218, CDRH2 of SEQ ID NO:219, CDRH3 of SEQ ID NO:220, CDRL1 of SEQ ID NO:222, CDRL2 of SEQ ID NO:223 and CDRL3 of SEQ ID NO:224;
cc) CDRH1 of SEQ ID NO:226, CDRH2 of SEQ ID NO:227, CDRH3 of SEQ ID NO:228, CDRL1 of SEQ ID NO:230, CDRL2 of SEQ ID NO:231 and CDRL3 of SEQ ID NO:232;
dd) CDRH1 of SEQ ID NO:234, CDRH2 of SEQ ID NO:235, CDRH3 of SEQ ID NO:236, CDRL1 of SEQ ID NO:238, CDRL2 of SEQ ID NO:239 and CDRL3 of SEQ ID NO:240;
ee) CDRH1 of SEQ ID NO:242, CDRH2 of SEQ ID NO:243, CDRH3 of SEQ ID NO:244, CDRL1 of SEQ ID NO:246, CDRL2 of SEQ ID NO:247 and CDRL3 of SEQ ID NO:248;
ff) CDRH1 of SEQ ID NO:250, CDRH2 of SEQ ID NO:251, CDRH3 of SEQ ID NO:252, CDRL1 of SEQ ID NO:254, CDRL2 of SEQ ID NO:255 and CDRL3 of SEQ ID NO:256;
gg) CDRH1 of SEQ ID NO:258, CDRH2 of SEQ ID NO:259, CDRH3 of SEQ ID NO:260, CDRL1 of SEQ ID NO:262, CDRL2 of SEQ ID NO:263 and CDRL3 of SEQ ID NO:264;
hh) CDRH1 of SEQ ID NO:266, CDRH2 of SEQ ID NO:267, CDRH3 of SEQ ID NO:268, CDRL1 of SEQ ID NO:270, CDRL2 of SEQ ID NO:271 and CDRL3 of SEQ ID NO:272;
ii) CDRH1 of SEQ ID NO:274, CDRH2 of SEQ ID NO:275, CDRH3 of SEQ ID NO:276, CDRL1 of SEQ ID NO:278, CDRL2 of SEQ ID NO:279 and CDRL3 of SEQ ID NO:280;
jj) CDRH1 of SEQ ID NO:282, CDRH2 of SEQ ID NO:283, CDRH3 of SEQ ID NO:284, CDRL1 of SEQ ID NO:286, CDRL2 of SEQ ID NO:287 and CDRL3 of SEQ ID NO:288;
kk) CDRH1 of SEQ ID NO:290, CDRH2 of SEQ ID NO:291, CDRH3 of SEQ ID NO:292, CDRL1 of SEQ ID NO:294, CDRL2 of SEQ ID NO:295 and CDRL3 of SEQ ID NO:296;
ll) CDRH1 of SEQ ID NO:298, CDRH2 of SEQ ID NO:299, CDRH3 of SEQ ID NO:300, CDRL1 of SEQ ID NO:302, CDRL2 of SEQ ID NO:303 and CDRL3 of SEQ ID NO:304;
mm) CDRH1 of SEQ ID NO:306, CDRH2 of SEQ ID NO:307, CDRH3 of SEQ ID NO:308, CDRL1 of SEQ ID NO:310, CDRL2 of SEQ ID NO:311 and CDRL3 of SEQ ID NO:312;
nn) CDRH1 of SEQ ID NO:314, CDRH2 of SEQ ID NO:315, CDRH3 of SEQ ID NO:316, CDRL1 of SEQ ID NO:318, CDRL2 of SEQ ID NO:319 and CDRL3 of SEQ ID NO:320;
oo) CDRH1 of SEQ ID NO:322, CDRH2 of SEQ ID NO:323, CDRH3 of SEQ ID NO:324, CDRL1 of SEQ ID NO:326, CDRL2 of SEQ ID NO:327 and CDRL3 of SEQ ID NO:328;
pp) CDRH1 of SEQ ID NO:330, CDRH2 of SEQ ID NO:331, CDRH3 of SEQ ID NO:332, CDRL1 of SEQ ID NO:334, CDRL2 of SEQ ID NO:335 and CDRL3 of SEQ ID NO:336;
qq) CDRH1 of SEQ ID NO:338, CDRH2 of SEQ ID NO:339, CDRH3 of SEQ ID NO:340, CDRL1 of SEQ ID NO:342, CDRL2 of SEQ ID NO:343 and CDRL3 of SEQ ID NO:344;
rr) CDRH1 of SEQ ID NO:346, CDRH2 of SEQ ID NO:347, CDRH3 of SEQ ID NO:348, CDRL1 of SEQ ID NO:350, CDRL2 of SEQ ID NO:351 and CDRL3 of SEQ ID NO:352;
ss) CDRH1 of SEQ ID NO:354, CDRH2 of SEQ ID NO:355, CDRH3 of SEQ ID NO:356, CDRL1 of SEQ ID NO:358, CDRL2 of SEQ ID NO:359 and CDRL3 of SEQ ID NO:360;
tt) CDRH1 of SEQ ID NO:362, CDRH2 of SEQ ID NO:363, CDRH3 of SEQ ID NO:364, CDRL1 of SEQ ID NO:366, CDRL2 of SEQ ID NO:367 and CDRL3 of SEQ ID NO:368;
uu) CDRH1 of SEQ ID NO:370, CDRH2 of SEQ ID NO:371, CDRH3 of SEQ ID NO:372, CDRL1 of SEQ ID NO:374, CDRL2 of SEQ ID NO:375 and CDRL3 of SEQ ID NO:376;
vv) CDRH1 of SEQ ID NO:378, CDRH2 of SEQ ID NO:379, CDRH3 of SEQ ID NO:380, CDRL1 of SEQ ID NO:382, CDRL2 of SEQ ID NO:383 and CDRL3 of SEQ ID NO:384;
ww) CDRH1 of SEQ ID NO:386, CDRH2 of SEQ ID NO:387, CDRH3 of SEQ ID NO:388, CDRL1 of SEQ ID NO:390, CDRL2 of SEQ ID NO:391 and CDRL3 of SEQ ID NO:392;
xx) CDRH1 of SEQ ID NO:394, CDRH2 of SEQ ID NO:395, CDRH3 of SEQ ID NO:396, CDRL1 of SEQ ID NO:398, CDRL2 of SEQ ID NO:399 and CDRL3 of SEQ ID NO:400;
yy) CDRH1 of SEQ ID NO:402, CDRH2 of SEQ ID NO:403, CDRH3 of SEQ ID NO:404, CDRL1 of SEQ ID NO:406, CDRL2 of SEQ ID NO:407 and CDRL3 of SEQ ID NO:408;
zz) CDRH1 of SEQ ID NO:410, CDRH2 of SEQ ID NO:411, CDRH3 of SEQ ID NO:412, CDRL1 of SEQ ID NO:414, CDRL2 of SEQ ID NO:415 and CDRL3 of SEQ ID NO:416;
aaa) CDRH1 of SEQ ID NO:418, CDRH2 of SEQ ID NO:419, CDRH3 of SEQ ID NO:420, CDRL1 of SEQ ID NO:422, CDRL2 of SEQ ID NO:423 and CDRL3 of SEQ ID NO:424;
bbb) CDRH1 of SEQ ID NO:426, CDRH2 of SEQ ID NO:427, CDRH3 of SEQ ID NO:428, CDRL1 of SEQ ID NO:430, CDRL2 of SEQ ID NO:431 and CDRL3 of SEQ ID NO:432;
ccc) CDRH1 of SEQ ID NO:434, CDRH2 of SEQ ID NO:435, CDRH3 of SEQ ID NO:436, CDRL1 of SEQ ID NO:438, CDRL2 of SEQ ID NO:439 and CDRL3 of SEQ ID NO:440;
ddd) CDRH1 of SEQ ID NO:442, CDRH2 of SEQ ID NO:443, CDRH3 of SEQ ID NO:444, CDRL1 of SEQ ID NO:446, CDRL2 of SEQ ID NO:447 and CDRL3 of SEQ ID NO:448;
eee) CDRH1 of SEQ ID NO:450, CDRH2 of SEQ ID NO:451, CDRH3 of SEQ ID NO:452, CDRL1 of SEQ ID NO:454, CDRL2 of SEQ ID NO:455 and CDRL3 of SEQ ID NO:456;
fff) CDRH1 of SEQ ID NO:458, CDRH2 of SEQ ID NO:459, CDRH3 of SEQ ID NO:460, CDRL1 of SEQ ID NO:462, CDRL2 of SEQ ID NO:463 and CDRL3 of SEQ ID NO:464;
ggg) CDRH1 of SEQ ID NO:466, CDRH2 of SEQ ID NO:467, CDRH3 of SEQ ID NO:468, CDRL1 of SEQ ID NO:470, CDRL2 of SEQ ID NO:471 and CDRL3 of SEQ ID NO:472;
hhh) CDRH1 of SEQ ID NO:474, CDRH2 of SEQ ID NO:475, CDRH3 of SEQ ID NO:476, CDRL1 of SEQ ID NO:478, CDRL2 of SEQ ID NO:479 and CDRL3 of SEQ ID NO:480;
iii) CDRH1 of SEQ ID NO:482, CDRH2 of SEQ ID NO:483, CDRH3 of SEQ ID NO:484, CDRL1 of SEQ ID NO:486, CDRL2 of SEQ ID NO:487 and CDRL3 of SEQ ID NO:488; and
jjj) CDRH1 of SEQ ID NO:490, CDRH2 of SEQ ID NO:491, CDRH3 of SEQ ID NO:492, CDRL1 of SEQ ID NO:494, CDRL2 of SEQ ID NO:495 and CDRL3 of SEQ ID NO:496.

2. The isolated antibody or antigen-binding fragment thereof according to claim 1, **characterized in that** the heavy chain variable region VH and the light chain variable region VL are selected from the following amino acid sequences:
a) VH of SEQ ID NO:1 and VL of SEQ ID NO:5;
b) VH of SEQ ID NO:9 and VL of SEQ ID NO:13;
c) VH of SEQ ID NO:17 and VL of SEQ ID NO:21;
d) VH of SEQ ID NO:25 and VL of SEQ ID NO:29;
e) VH of SEQ ID NO:33 and VL of SEQ ID NO:37;
f) VH of SEQ ID NO:41 and VL of SEQ ID NO:45;
g) VH of SEQ ID NO:49 and VL of SEQ ID NO:53;
h) VH of SEQ ID NO:57 and VL of SEQ ID NO:61;
i) VH of SEQ ID NO:65 and VL of SEQ ID NO:69;
j) VH of SEQ ID NO:73 and VL of SEQ ID NO:77;
k) VH of SEQ ID NO:81 and VL of SEQ ID NO:85;
l) VH of SEQ ID NO:89 and VL of SEQ ID NO:93;
m) VH of SEQ ID NO:97 and VL of SEQ ID NO: 101;
n) VH of SEQ ID NO:105 and VL of SEQ ID NO:109;
o) VH of SEQ ID NO:113 and VL of SEQ ID NO:117;
p) VH of SEQ ID NO:121 and VL of SEQ ID NO:125;
q) VH of SEQ ID NO:129 and VL of SEQ ID NO:133;
r) VH of SEQ ID NO:137 and VL of SEQ ID NO:141;
s) VH of SEQ ID NO:145 and VL of SEQ ID NO:149;
t) VH of SEQ ID NO:153 and VL of SEQ ID NO:157;
u) VH of SEQ ID NO:161 and VL of SEQ ID NO:165;
v) VH of SEQ ID NO:169 and VL of SEQ ID NO:173;
w) VH of SEQ ID NO:177 and VL of SEQ ID NO:181;
x) VH of SEQ ID NO:185 and VL of SEQ ID NO:189;
y) VH of SEQ ID NO:193 and VL of SEQ ID NO:197;
z) VH of SEQ ID NO:201 and VL of SEQ ID NO:205;
aa) VH of SEQ ID NO:209 and VL of SEQ ID NO:213;
bb) VH of SEQ ID NO:217 and VL of SEQ ID NO:221;
cc) VH of SEQ ID NO:225 and VL of SEQ ID NO:229;
dd) VH of SEQ ID NO:233 and VL of SEQ ID NO:237;
ee) VH of SEQ ID NO:241 and VL of SEQ ID NO:245;
ff) VH of SEQ ID NO:249 and VL of SEQ ID NO:253;
gg) VH of SEQ ID NO:257 and VL of SEQ ID NO:261;
hh) VH of SEQ ID NO:265 and VL of SEQ ID NO:269;
ii) VH of SEQ ID NO:273 and VL of SEQ ID NO:277;
jj) VH of SEQ ID NO:281 and VL of SEQ ID NO:285;
kk) VH of SEQ ID NO:289 and VL of SEQ ID NO:293;
ll) VH of SEQ ID NO:297 and VL of SEQ ID NO:301;
mm) VH of SEQ ID NO:305 and VL of SEQ ID NO:309;
nn) VH of SEQ ID NO:313 and VL of SEQ ID NO:317;
oo) VH of SEQ ID NO:321 and VL of SEQ ID NO:325;
pp) VH of SEQ ID NO:329 and VL of SEQ ID NO:333;
qq) VH of SEQ ID NO:337 and VL of SEQ ID NO:341;
rr) VH of SEQ ID NO:345 and VL of SEQ ID NO:349;
ss) VH of SEQ ID NO:353 and VL of SEQ ID NO:357;
tt) VH of SEQ ID NO:361 and VL of SEQ ID NO:365;
uu) VH of SEQ ID NO:369 and VL of SEQ ID NO:373;
vv) VH of SEQ ID NO:377 and VL of SEQ ID NO:381;
ww) VH of SEQ ID NO:385 and VL of SEQ ID NO:389;
xx) VH of SEQ ID NO:393 and VL of SEQ ID NO:397;
yy) VH of SEQ ID NO:401 and VL of SEQ ID NO:405;
zz) VH of SEQ ID NO:409 and VL of SEQ ID NO:413;
aaa) VH of SEQ ID NO:417 and VL of SEQ ID NO:421;
bbb) VH of SEQ ID NO:425 and VL of SEQ ID NO:429;
ccc) VH of SEQ ID NO:433 and VL of SEQ ID NO:437;
ddd) VH of SEQ ID NO:441 and VL of SEQ ID NO:445;
eee) VH of SEQ ID NO:449 and VL of SEQ ID NO:453;
fff) VH of SEQ ID NO:457 and VL of SEQ ID NO:461;
ggg) VH of SEQ ID NO:465 and VL of SEQ ID NO:469;
hhh) VH of SEQ ID NO:473 and VL of SEQ ID NO:477;
iii) VH of SEQ ID NO:481 and VL of SEQ ID NO:485; and
jjj) VH of SEQ ID NO:489 and VL of SEQ ID NO:493,
or amino acid sequences that have at least 80%, 85%, 90%, 95%, 96%, 97%, 98%, 99%, or 100% sequence identity with any of the above sets of sequences.

3. The isolated antibody or antigen-binding fragment thereof according to claim 1 or 2, **characterized in that** the isolated antibody or antigen-binding fragment thereof specifically binds to an epitope in the S region of the extracellular domain of HBsAg.

4. The isolated antibody or antigen-binding fragment thereof according to claim 1 or 2, **characterized in that** the isolated antibody or antigen-binding fragment thereof specifically binds to an epitope in amino acid residues 99-169 of the HBsAg protein shown in SEQ ID NO:497.

5. The isolated antibody or antigen-binding fragment thereof according to claim 1 or 2, **characterized in that** the isolated antibody or antigen-binding fragment thereof specifically binds to HBsAg proteins of wild-type HBV and its mutant strains.

6. The isolated antibody or antigen-binding fragment thereof according to claim 5, **characterized in that** the mutant strain comprises a mutation selected from G145R, K122R-G145R and F134L.

7. The isolated antibody or antigen-binding fragment thereof according to claim 1 or 2, **characterized in that** the isolated antibody or antigen-binding fragment thereof is a monoclonal antibody, a polyclonal antibody, a recombinant antibody, a human antibody, a humanized antibody, a chimeric antibody, a multispecific antibody, or an antibody fragment thereof.

8. The isolated antibody or antigen-binding fragment thereof according to claim 1 or 2, **characterized in that** the antigen-binding fragment is a Fab fragment, a Fab' fragment, an F(ab')₂ fragment, an Fv fragment, a Diabody, or a single-chain antibody molecule.

9. The isolated antibody or antigen-binding fragment thereof according to claim 1 or 2, **characterized in that** the isolated antibody or antigen-binding fragment thereof is IgG1, IgG2, IgG3 or IgG4 isotype.

10. A bispecific antibody, **characterized in that** the bispecific antibody comprises a first domain that specifically binds to a first epitope on hepatitis B surface antigen (HBsAg) and a second domain that specifically binds to a second epitope on HBsAg, wherein the first epitope and the second epitope are each independently selected from:
a) an epitope comprising amino acid residues 110I, 122K, 125T, 134F, 141K, and 165W;
b) an epitope comprising amino acid residues 122K, 123T, 145G, 161Y, and 165W; and
c) an epitope comprising amino acid residues 109L-111P, 120P-130G, 133M-136S, 144D, 146N, 153P, 161Y, and 164E-167S,
wherein the numbering of amino acid residues is based on the amino acid positions shown in SEQ ID NO:497,
wherein the first epitope is different from the second epitope.

11. The bispecific antibody according to claim 10, **characterized in that**:
the first domain comprises a heavy chain variable region VH and a light chain variable region VL, said heavy chain variable region VH comprising complementarity-determining regions CDRH1, CDRH2, and CDRH3, said light chain variable region VL comprising complementarity-determining regions CDRL1, CDRL2, and CDRL3, wherein CDRH1, CDRH2, CDRH3, CDRL1, CDRL2, and CDRL3 are selected from the following amino acid sequences:
a) CDRH1 of SEQ ID NO:178, CDRH2 of SEQ ID NO:179, CDRH3 of SEQ ID NO:180, CDRL1 of SEQ ID NO:182, CDRL2 of SEQ ID NO:183 and CDRL3 of SEQ ID NO:184;
b) CDRH1 of SEQ ID NO:210, CDRH2 of SEQ ID NO:211, CDRH3 of SEQ ID NO:212, CDRL1 of SEQ ID NO:214, CDRL2 of SEQ ID NO:215 and CDRL3 of SEQ ID NO:216;
c) CDRH1 of SEQ ID NO:266, CDRH2 of SEQ ID NO:267, CDRH3 of SEQ ID NO:268, CDRL1 of SEQ ID NO:270, CDRL2 of SEQ ID NO:271 and CDRL3 of SEQ ID NO:272;
d) CDRH1 of SEQ ID NO:434, CDRH2 of SEQ ID NO:435, CDRH3 of SEQ ID NO:436, CDRL1 of SEQ ID NO:438, CDRL2 of SEQ ID NO:439 and CDRL3 of SEQ ID NO:440; and
e) CDRH1 of SEQ ID NO:458, CDRH2 of SEQ ID NO:459, CDRH3 of SEQ ID NO:460, CDRL1 of SEQ ID NO:462, CDRL2 of SEQ ID NO:463 and CDRL3 of SEQ ID NO:464;
the second domain comprises a heavy chain variable region VH and a light chain variable region VL, said heavy chain variable region VH comprising complementarity-determining regions CDRH1, CDRH2, and CDRH3, said light chain variable region VL comprising complementarity-determining regions CDRL1, CDRL2, and CDRL3, wherein CDRH1, CDRH2, CDRH3, CDRL1, CDRL2, and CDRL3 are selected from the following amino acid sequences:
a) CDRH1 of SEQ ID NO:154, CDRH2 of SEQ ID NO:155, CDRH3 of SEQ ID NO:156, CDRL1 of SEQ ID NO:158, CDRL2 of SEQ ID NO:159 and CDRL3 of SEQ ID NO:160;
b) CDRH1 of SEQ ID NO:218, CDRH2 of SEQ ID NO:219, CDRH3 of SEQ ID NO:220, CDRL1 of SEQ ID NO:222, CDRL2 of SEQ ID NO:223 and CDRL3 of SEQ ID NO:224;
c) CDRH1 of SEQ ID NO:234, CDRH2 of SEQ ID NO:235, CDRH3 of SEQ ID NO:236, CDRL1 of SEQ ID NO:238, CDRL2 of SEQ ID NO:239 and CDRL3 of SEQ ID NO:240;
d) CDRH1 of SEQ ID NO:282, CDRH2 of SEQ ID NO:283, CDRH3 of SEQ ID NO:284, CDRL1 of SEQ ID NO:286, CDRL2 of SEQ ID NO:287 and CDRL3 of SEQ ID NO:288; and
e) CDRH1 of SEQ ID NO:314, CDRH2 of SEQ ID NO:315, CDRH3 of SEQ ID NO:316, CDRL1 of SEQ ID NO:318, CDRL2 of SEQ ID NO:319 and CDRL3 of SEQ ID NO:320.

12. The bispecific antibody according to claim 11, **characterized in that**:
the first domain comprises a heavy chain variable region VH and a light chain variable region VL selected from the following amino acid sequences:
a) VH of SEQ ID NO:177 and VL of SEQ ID NO:181;
b) VH of SEQ ID NO:209 and VL of SEQ ID NO:213;
c) VH of SEQ ID NO:265 and VL of SEQ ID NO:269;
d) VH of SEQ ID NO:433 and VL of SEQ ID NO:437; and
e) VH of SEQ ID NO:457 and VL of SEQ ID NO:461;
or amino acid sequences that have at least 80%, 85%, 90%, 95%, 96%, 97%, 98%, 99%, or 100% sequence identity with any of the above sets of sequences,
the second domain comprises a heavy chain variable region VH and a light chain variable region VL selected from the following amino acid sequences:
a) VH of SEQ ID NO:153 and VL of SEQ ID NO:157;
b) VH of SEQ ID NO:217 and VL of SEQ ID NO:221;
c) VH of SEQ ID NO:233 and VL of SEQ ID NO:237;
d) VH of SEQ ID NO:281 and VL of SEQ ID NO:285; and
e) VH of SEQ ID NO:313 and VL of SEQ ID NO:317;
or amino acid sequences that have at least 80%, 85%, 90%, 95%, 96%, 97%, 98%, 99%, or 100% sequence identity with any of the above sets of sequences.

13. The bispecific antibody according to claim 10, **characterized in that**:
the first domain comprises a heavy chain variable region VH and a light chain variable region VL, said heavy chain variable region VH comprising complementarity-determining regions CDRH1, CDRH2, and CDRH3, said light chain variable region VL comprising complementarity-determining regions CDRL1, CDRL2, and CDRL3, wherein CDRH1, CDRH2, CDRH3, CDRL1, CDRL2, and CDRL3 are selected from the following amino acid sequences:
a) CDRH1 of SEQ ID NO:178, CDRH2 of SEQ ID NO:179, CDRH3 of SEQ ID NO:180, CDRL1 of SEQ ID NO:182, CDRL2 of SEQ ID NO:183 and CDRL3 of SEQ ID NO:184;
b) CDRH1 of SEQ ID NO:210, CDRH2 of SEQ ID NO:211, CDRH3 of SEQ ID NO:212, CDRL1 of SEQ ID NO:214, CDRL2 of SEQ ID NO:215 and CDRL3 of SEQ ID NO:216;
c) CDRH1 of SEQ ID NO:266, CDRH2 of SEQ ID NO:267, CDRH3 of SEQ ID NO:268, CDRL1 of SEQ ID NO:270, CDRL2 of SEQ ID NO:271 and CDRL3 of SEQ ID NO:272;
d) CDRH1 of SEQ ID NO:434, CDRH2 of SEQ ID NO:435, CDRH3 of SEQ ID NO:436, CDRL1 of SEQ ID NO:438, CDRL2 of SEQ ID NO:439 and CDRL3 of SEQ ID NO:440; and
e) CDRH1 of SEQ ID NO:458, CDRH2 of SEQ ID NO:459, CDRH3 of SEQ ID NO:460, CDRL1 of SEQ ID NO:462, CDRL2 of SEQ ID NO:463 and CDRL3 of SEQ ID NO:464;
the second domain comprises a heavy chain variable region VH and a light chain variable region VL, said heavy chain variable region VH comprising complementarity-determining regions CDRH1, CDRH2, and CDRH3, said light chain variable region VL comprising complementarity-determining regions CDRL1, CDRL2, and CDRL3, wherein CDRH1, CDRH2, CDRH3, CDRL1, CDRL2, and CDRL3 are selected from the following amino acid sequences:
a) CDRH1 of SEQ ID NO:2, CDRH2 of SEQ ID NO:3, CDRH3 of SEQ ID NO:4, CDRL1 of SEQ ID NO:6, CDRL2 of SEQ ID NO:7 and CDRL3 of SEQ ID NO:8;
b) CDRH1 of SEQ ID NO:50, CDRH2 of SEQ ID NO:51, CDRH3 of SEQ ID NO:52, CDRL1 of SEQ ID NO:54, CDRL2 of SEQ ID NO:55 and CDRL3 of SEQ ID NO:56;
c) CDRH1 of SEQ ID NO:82, CDRH2 of SEQ ID NO:83, CDRH3 of SEQ ID NO:84, CDRL1 of SEQ ID NO:86, CDRL2 of SEQ ID NO:87 and CDRL3 of SEQ ID NO:88;
d) CDRH1 of SEQ ID NO:90, CDRH2 of SEQ ID NO:91, CDRH3 of SEQ ID NO:92, CDRL1 of SEQ ID NO:94, CDRL2 of SEQ ID NO:95 and CDRL3 of SEQ ID NO:96;
e) CDRH1 of SEQ ID NO:106, CDRH2 of SEQ ID NO:107, CDRH3 of SEQ ID NO:108, CDRL1 of SEQ ID NO:110, CDRL2 of SEQ ID NO:111 and CDRL3 of SEQ ID NO:112;
f) CDRH1 of SEQ ID NO:114, CDRH2 of SEQ ID NO:115, CDRH3 of SEQ ID NO:116, CDRL1 of SEQ ID NO:118, CDRL2 of SEQ ID NO:119 and CDRL3 of SEQ ID NO:120;
g) CDRH1 of SEQ ID NO:322, CDRH2 of SEQ ID NO:323, CDRH3 of SEQ ID NO:324, CDRL1 of SEQ ID NO:326, CDRL2 of SEQ ID NO:327 and CDRL3 of SEQ ID NO:328;
j) CDRH1 of SEQ ID NO:346, CDRH2 of SEQ ID NO:347, CDRH3 of SEQ ID NO:348, CDRL1 of SEQ ID NO:350, CDRL2 of SEQ ID NO:351 and CDRL3 of SEQ ID NO:352;
i) CDRH1 of SEQ ID NO:354, CDRH2 of SEQ ID NO:355, CDRH3 of SEQ ID NO:356, CDRL1 of SEQ ID NO:358, CDRL2 of SEQ ID NO:359 and CDRL3 of SEQ ID NO:360;
j) CDRH1 of SEQ ID NO:378, CDRH2 of SEQ ID NO:379, CDRH3 of SEQ ID NO:380, CDRL1 of SEQ ID NO:382, CDRL2 of SEQ ID NO:383 and CDRL3 of SEQ ID NO:384;
k) CDRH1 of SEQ ID NO:386, CDRH2 of SEQ ID NO:387, CDRH3 of SEQ ID NO:388, CDRL1 of SEQ ID NO:390, CDRL2 of SEQ ID NO:391 and CDRL3 of SEQ ID NO:392; and
l) CDRH1 of SEQ ID NO:418, CDRH2 of SEQ ID NO:419, CDRH3 of SEQ ID NO:420, CDRL1 of SEQ ID NO:422, CDRL2 of SEQ ID NO:423 and CDRL3 of SEQ ID NO:424.

14. The bispecific antibody according to claim 13, **characterized in that**:
the first domain comprises a heavy chain variable region VH and a light chain variable region VL selected from the following amino acid sequences:
a) VH of SEQ ID NO:177 and VL of SEQ ID NO:181;
b) VH of SEQ ID NO:209 and VL of SEQ ID NO:213;
c) VH of SEQ ID NO:265 and VL of SEQ ID NO:269;
d) VH of SEQ ID NO:433 and VL of SEQ ID NO:437; and
e) VH of SEQ ID NO:457 and VL of SEQ ID NO:461;
or amino acid sequences that have at least 80%, 85%, 90%, 95%, 96%, 97%, 98%, 99%, or 100% sequence identity with any of the above sets of sequences,
the second domain comprises a heavy chain variable region VH and a light chain variable region VL selected from the following amino acid sequences:
a) VH of SEQ ID NO:1 and VL of SEQ ID NO:5;
b) VH of SEQ ID NO:49 and VL of SEQ ID NO:53;
c) VH of SEQ ID NO:81 and VL of SEQ ID NO:85;
d) VH of SEQ ID NO:89 and VL of SEQ ID NO:93;
e) VH of SEQ ID NO:105 and VL of SEQ ID NO:109;
f) VH of SEQ ID NO:113 and VL of SEQ ID NO:117;
g) VH of SEQ ID NO:321 and VL of SEQ ID NO:325;
h) VH of SEQ ID NO:345 and VL of SEQ ID NO:349;
i) VH of SEQ ID NO:353 and VL of SEQ ID NO:357;
j) VH of SEQ ID NO:377 and VL of SEQ ID NO:381;
k) VH of SEQ ID NO:385 and VL of SEQ ID NO:389; and
l) VH of SEQ ID NO:417 and VL of SEQ ID NO:421;
or amino acid sequences that have at least 80%, 85%, 90%, 95%, 96%, 97%, 98%, 99%, or 100% sequence identity with any of the above sets of sequences.

15. The bispecific antibody according to claim 10, **characterized in that**:
the first domain comprises a heavy chain variable region VH and a light chain variable region VL, said heavy chain variable region VH comprising complementarity-determining regions CDRH1, CDRH2, and CDRH3, said light chain variable region VL comprising complementarity-determining regions CDRL1, CDRL2, and CDRL3, wherein CDRH1, CDRH2, CDRH3, CDRL1, CDRL2, and CDRL3 are selected from the following amino acid sequences:
a) CDRH1 of SEQ ID NO:154, CDRH2 of SEQ ID NO:155, CDRH3 of SEQ ID NO:156, CDRL1 of SEQ ID NO:158, CDRL2 of SEQ ID NO:159 and CDRL3 of SEQ ID NO:160;
b) CDRH1 of SEQ ID NO:218, CDRH2 of SEQ ID NO:219, CDRH3 of SEQ ID NO:220, CDRL1 of SEQ ID NO:222, CDRL2 of SEQ ID NO:223 and CDRL3 of SEQ ID NO:224;
c) CDRH1 of SEQ ID NO:234, CDRH2 of SEQ ID NO:235, CDRH3 of SEQ ID NO:236, CDRL1 of SEQ ID NO:238, CDRL2 of SEQ ID NO:239 and CDRL3 of SEQ ID NO:240;
d) CDRH1 of SEQ ID NO:282, CDRH2 of SEQ ID NO:283, CDRH3 of SEQ ID NO:284, CDRL1 of SEQ ID NO:286, CDRL2 of SEQ ID NO:287 and CDRL3 of SEQ ID NO:288; and
e) CDRH1 of SEQ ID NO:314, CDRH2 of SEQ ID NO:315, CDRH3 of SEQ ID NO:316, CDRL1 of SEQ ID NO:318, CDRL2 of SEQ ID NO:319 and CDRL3 of SEQ ID NO:320;
the second domain comprises a heavy chain variable region VH and a light chain variable region VL, said heavy chain variable region VH comprising complementarity-determining regions CDRH1, CDRH2, and CDRH3, said light chain variable region VL comprising complementarity-determining regions CDRL1, CDRL2, and CDRL3, wherein CDRH1, CDRH2, CDRH3, CDRL1, CDRL2, and CDRL3 are selected from the following amino acid sequences:
a) CDRH1 of SEQ ID NO:2, CDRH2 of SEQ ID NO:3, CDRH3 of SEQ ID NO:4, CDRL1 of SEQ ID NO:6, CDRL2 of SEQ ID NO:7 and CDRL3 of SEQ ID NO:8;
b) CDRH1 of SEQ ID NO:50, CDRH2 of SEQ ID NO:51, CDRH3 of SEQ ID NO:52, CDRL1 of SEQ ID NO:54, CDRL2 of SEQ ID NO:55 and CDRL3 of SEQ ID NO:56;
c) CDRH1 of SEQ ID NO:82, CDRH2 of SEQ ID NO:83, CDRH3 of SEQ ID NO:84, CDRL1 of SEQ ID NO:86, CDRL2 of SEQ ID NO:87 and CDRL3 of SEQ ID NO:88;
d) CDRH1 of SEQ ID NO:90, CDRH2 of SEQ ID NO:91, CDRH3 of SEQ ID NO:92, CDRL1 of SEQ ID NO:94, CDRL2 of SEQ ID NO:95 and CDRL3 of SEQ ID NO:96;
e) CDRH1 of SEQ ID NO:106, CDRH2 of SEQ ID NO:107, CDRH3 of SEQ ID NO:108, CDRL1 of SEQ ID NO:110, CDRL2 of SEQ ID NO:111 and CDRL3 of SEQ ID NO:112;
f) CDRH1 of SEQ ID NO:114, CDRH2 of SEQ ID NO:115, CDRH3 of SEQ ID NO:116, CDRL1 of SEQ ID NO:118, CDRL2 of SEQ ID NO:119 and CDRL3 of SEQ ID NO:120;
g) CDRH1 of SEQ ID NO:322, CDRH2 of SEQ ID NO:323, CDRH3 of SEQ ID NO:324, CDRL1 of SEQ ID NO:326, CDRL2 of SEQ ID NO:327 and CDRL3 of SEQ ID NO:328;
h) CDRH1 of SEQ ID NO:346, CDRH2 of SEQ ID NO:347, CDRH3 of SEQ ID NO:348, CDRL1 of SEQ ID NO:350, CDRL2 of SEQ ID NO:351 and CDRL3 of SEQ ID NO:352;
i) CDRH1 of SEQ ID NO:354, CDRH2 of SEQ ID NO:355, CDRH3 of SEQ ID NO:356, CDRL1 of SEQ ID NO:358, CDRL2 of SEQ ID NO:359 and CDRL3 of SEQ ID NO:360;
j) CDRH1 of SEQ ID NO:378, CDRH2 of SEQ ID NO:379, CDRH3 of SEQ ID NO:380, CDRL1 of SEQ ID NO:382, CDRL2 of SEQ ID NO:383 and CDRL3 of SEQ ID NO:384;
k) CDRH1 of SEQ ID NO:386, CDRH2 of SEQ ID NO:387, CDRH3 of SEQ ID NO:388, CDRL1 of SEQ ID NO:390, CDRL2 of SEQ ID NO:391 and CDRL3 of SEQ ID NO:392; and
l) CDRH1 of SEQ ID NO:418, CDRH2 of SEQ ID NO:419, CDRH3 of SEQ ID NO:420, CDRL1 of SEQ ID NO:422, CDRL2 of SEQ ID NO:423 and CDRL3 of SEQ ID NO:424.

16. The bispecific antibody according to claim 15, **characterized in that**:
the first domain comprises a heavy chain variable region VH and a light chain variable region VL selected from the following amino acid sequences:
a) VH of SEQ ID NO:153 and VL of SEQ ID NO:157;
b) VH of SEQ ID NO:217 and VL of SEQ ID NO:221;
c) VH of SEQ ID NO:233 and VL of SEQ ID NO:237;
d) VH of SEQ ID NO:281 and VL of SEQ ID NO:285; and
e) VH of SEQ ID NO:313 and VL of SEQ ID NO:317;
or amino acid sequences that have at least 80%, 85%, 90%, 95%, 96%, 97%, 98%, 99%, or 100% sequence identity with any of the above sets of sequences,
the second domain comprises a heavy chain variable region VH and a light chain variable region VL selected from the following amino acid sequences:
a) VH of SEQ ID NO:1 and VL of SEQ ID NO:5;
b) VH of SEQ ID NO:49 and VL of SEQ ID NO:53;
c) VH of SEQ ID NO:81 and VL of SEQ ID NO:85;
d) VH of SEQ ID NO:89 and VL of SEQ ID NO:93;
e) VH of SEQ ID NO:105 and VL of SEQ ID NO:109;
f) VH of SEQ ID NO:113 and VL of SEQ ID NO:117;
g) VH of SEQ ID NO:321 and VL of SEQ ID NO:325;
h) VH of SEQ ID NO:345 and VL of SEQ ID NO:349;
i) VH of SEQ ID NO:353 and VL of SEQ ID NO:357;
j) VH of SEQ ID NO:377 and VL of SEQ ID NO:381;
k) VH of SEQ ID NO:385 and VL of SEQ ID NO:389; and
l) VH of SEQ ID NO:417 and VL of SEQ ID NO:421;
or amino acid sequences that have at least 80%, 85%, 90%, 95%, 96%, 97%, 98%, 99%, or 100% sequence identity with any of the above sets of sequences.

17. The bispecific antibody according to claim 10, **characterized in that**:
a) the first domain comprises a heavy chain variable region VH and a light chain variable region VL, said heavy chain variable region VH comprising CDRH1 of SEQ ID NO:282, CDRH2 of SEQ ID NO:283, CDRH3 of SEQ ID NO:284, CDRL1 of SEQ ID NO:286, CDRL2 of SEQ ID NO:287, and CDRL3 of SEQ ID NO:288; and the second domain comprises a heavy chain variable region VH and a light chain variable region VL, said heavy chain variable region VH comprising CDRH1 of SEQ ID NO:50, CDRH2 of SEQ ID NO:51, CDRH3 of SEQ ID NO:52, CDRL1 of SEQ ID NO:54, CDRL2 of SEQ ID NO:55, and CDRL3 of SEQ ID NO:56; or
b) the first domain comprises VH of SEQ ID NO:281 and VL of SEQ ID NO:285, and the second domain comprises VH of SEQ ID NO:49 and VL of SEQ ID NO:53.

18. The bispecific antibody according to any one of claims 10-17, **characterized in that** the first domain and/or the second domain are IgG1, IgG2, IgG3 or IgG4 isotype.

19. The bispecific antibody according to any one of claims 10-18, **characterized in that** the first domain is a full-length IgG antibody, the second domain is an scfv; or the first domain is an scfv and the second domain is a full-length IgG antibody.

20. The bispecific antibody according to claim 19, **characterized in that** the N-terminus of the scfv is connected to the C-terminus of at least one heavy chain of the full-length IgG antibody; or the N-terminus of the scfv is connected to the C-terminus of at least one light chain of the full-length IgG antibody.

21. The bispecific antibody according to any one of claims 10-20, **characterized in that** the first domain is connected to the second domain by a flexible peptide linker, preferably the flexible peptide linker has a length of no more than about 30 amino acids, and more preferably the flexible peptide linker is (G4S)₃.

22. The bispecific antibody according to any one of claims 10-18, **characterized in that** the bispecific antibody is CrossMAb-Fab.

23. An isolated polynucleotide encoding the isolated antibody or antigen-binding fragment thereof according to any one of claims 1-9.

24. An expression vector comprising the polynucleotide according to claim 23.

25. A host cell comprising the expression vector according to claim 24.

26. A method for producing the isolated antibody or antigen-binding fragment thereof according to any one of claims 1-9, **characterized in that** the method comprises culturing the host cell according to claim 25 under conditions allowing expression of the antibody or antigen-binding fragment thereof, and recovering the antibody or antigen-binding fragment thereof produced by the host cell.

27. A pharmaceutical composition comprising the isolated antibody or antigen-binding fragment thereof according to any one of claims 1-9, or the bispecific antibody according to any one of claims 10-22, and a pharmaceutically acceptable carrier.

28. The isolated antibody or antigen-binding fragment thereof according to any one of claims 1-9, or the bispecific antibody according to any one of claims 10-22, for use in the treatment or prevention of HBV infection in a subject in need thereof.

29. The isolated antibody or antigen-binding fragment thereof according to any one of claims 1-9, or the bispecific antibody according to any one of claims 10-22, for use in the treatment or prevention of HBV and its mutant strain infection in a subject in need thereof.

30. The isolated antibody or antigen-binding fragment thereof or bispecific antibody for use according to claim 28 or 29, **characterized in that** the HBV includes genotypes A, B, C and D.

31. The isolated antibody or antigen-binding fragment thereof or bispecific antibody for use according to claim 29, **characterized in that** the mutant strain comprises a single- or double-site mutation in amino acid residues 99-169 of the HBsAg protein shown in SEQ ID NO:497.

32. The isolated antibody or antigen-binding fragment thereof or bispecific antibody for use according to claim 29, **characterized in that** the mutant strain comprises a mutation selected from G145R, K122R-G145R and F134L.

33. Use of the isolated antibody or antigen-binding fragment thereof according to any one of claims 1-9, or the bispecific antibody according to any one of claims 10-22 in the preparation of a medicament for treating or preventing HBV infection in a subject in need thereof.

34. Use of the isolated antibody or antigen-binding fragment thereof according to any one of claims 1-9, or the bispecific antibody according to any one of claims 10-22 in the preparation of a medicament for treating or preventing HBV and its mutant strain infection in a subject in need thereof.

35. The use according to claim 33 or 34, **characterized in that** the HBV includes genotypes A, B, C and D.

36. The use according to claim 34, **characterized in that** the mutant strain comprises a single- or double-site mutation in amino acid residues 99-169 of the HBsAg protein shown in SEQ ID NO:497.

37. The use according to claim 34, **characterized in that** the mutant strain comprises a mutation selected from G145R, K122R-G145R and F134L.

38. A method for treating or preventing HBV infection in a subject in need thereof, comprising administering to the subject a therapeutically effective amount of the isolated antibody or antigen-binding fragment thereof according to any one of claims 1-9, or the bispecific antibody according to any one of claims 10-22.

39. A method for treating or preventing HBV and its mutant strain infection in a subject in need thereof, comprising administering to the subject a therapeutically effective amount of the isolated antibody or antigen-binding fragment thereof according to any one of claims 1-9, or the bispecific antibody according to any one of claims 10-22.

40. The method according to claim 38 or 39, **characterized in that** the HBV includes genotypes A, B, C, and D.

41. The method according to claim 39, **characterized in that** the mutant strain comprises a single- or double-site mutation in amino acid residues 99-169 of the HBsAg protein shown in SEQ ID NO:497.

42. The method according to claim 39, **characterized in that** the mutant strain comprises a mutation selected from G145R, K122R-G145R and F134L.
